# EUROPEAN PATENT SPECIFICATION

(11) **EP 1 554 264 B1**
(45) Date of publication and mention of the grant of the patent: **08.08.2007**
(21) Application number: 03795793.3
(22) Date of filing: 20.10.2003
(51) Int. Cl.: C07D 319/20, C07D 405/12, C07D 413/12, A61K 31/357, A61P 11/08

(54) **MEDICINAL ARYLETHANOLAMINE COMPOUNDS**
MEDIZINISCH VERWENDBARE ARYLETHANOLAMIN VERBINDUNGEN
DERIVES D'ARYLETHANOLAMINE A USAGE MEDICINAL

(30) Priority: 22.10.2002 GB 0224494; 22.10.2002 GB 0224498
(43) Date of publication of application: 20.07.2005
(73) Proprietor: GLAXO GROUP LIMITED, Greenford, Middlesex UB6 0NN (GB)
(72) Inventor: COE, Diane Mary, Stevenage, Hertfordshire SG1 2NY (GB); GUNTRIP, Stephen Barry, Stevenage, Hertfordshire SG1 2NY (GB)
(74) Representative: Florence, Julia Anne
(86) International application number: PCT/EP2003/011648
(87) International publication number: WO 2004/037807

(56) References cited:
- WO-A-98/29405

## Description

The present invention is concerned with phenethanolamine derivatives, processes for their preparation, compositions containing them and their use in medicine, particularly in the prophylaxis and treatment of respiratory diseases.

Certain phenethanolamine compounds are known in the art as having selective stimulant action at β₂-adrenoreceptors and therefore having utility in the treatment of bronchial asthma and related disorders. Thus GB 2 140 800 describes phenethanolamine compounds including 4-hydroxy-α¹-[[[6-(4-phenylbutoxy)hexyl]amino]methyl]-1,3-benzenedimethanol 1-hydroxy-2-naphthalenecarboxylate (salmeterol xinafoate) which is now used clinically in the treatment of such medical conditions.

Although salmeterol and the other commercially available β₂-adrenoreceptor agonists are effective bronchodilators, the duration of action is approximately 12 hours, hence twice daily dosing is often required. There is therefore a clinical need for compounds having potent and selective stimulant action at β₂-adrenoreceptors and having an advantageous profile of action.

According to the present invention, there is provided a compound of formula (I) 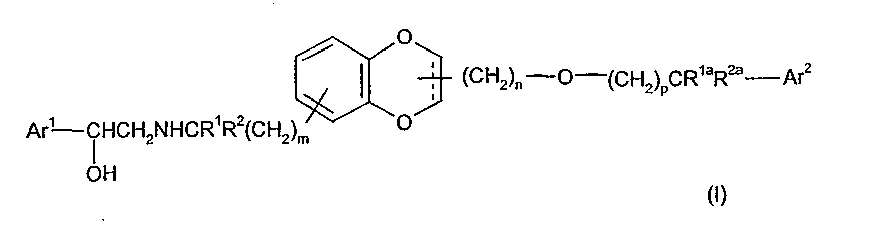 or a salt, solvate, or physiologically functional derivative thereof, wherein:
Ar¹ is a group selected from 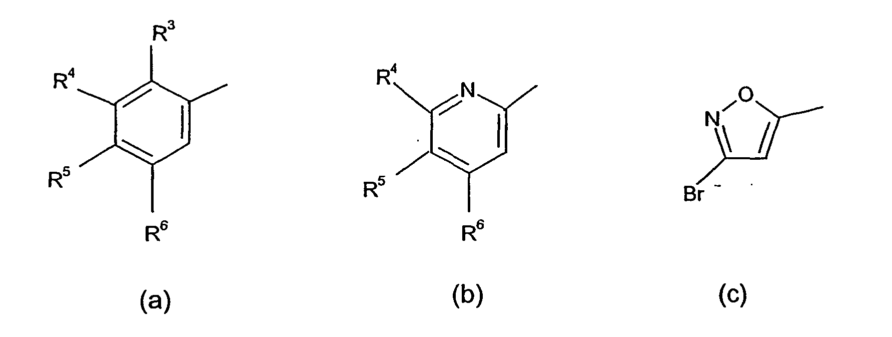 and 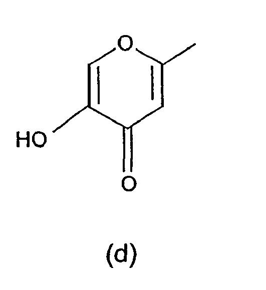 wherein R⁴ represents hydrogen, halogen, -(CH₂)_{q}OR⁷, -NR⁷C(O)R⁸, -NR⁷SO₂R⁸, -SO₂NR⁷R⁸, -NR⁷R⁸, -OC(O)R⁹ or OC(O)NR⁷R⁸, and R³ represents hydrogen, halogen or C₁₋₄ alkyl;
or R⁴ represents -NHR'° and R³ and -NHR¹⁰ together form a 5- or 6- membered heterocyclic ring;
R⁵ represents hydrogen, halogen, -OR⁷ or -NR⁷R⁸;
R⁶ represents hydrogen, halogen, haloC₁₋₄alkyl, -OR⁷, -NR⁷R⁸, -OC(O)R⁹ or OC(O)NR⁷R⁸;
R⁷ and R⁸ each independently represents hydrogen or C₁₋₄ alkyl, or in the groups -NR⁷R⁸, -SO₂NR⁷R⁸ and -OC(O)NR⁷R⁸, R⁷ and R⁸ independently represent hydrogen or C₁₋₄ alkyl or together with the nitrogen atom to which they are attached form a 5-, 6- or 7-membered nitrogen-containing ring,
R⁹ represents an aryl (eg phenyl or naphthyl) group which may be unsubstituted or substituted by one or more substituents selected from halogen, C₁₋₄ alkyl, hydroxy, C₁₋₄ alkoxy or halo C₁₋₄ alkyl; and
q is zero or an integer from 1 to 4;
Ar² is a group: 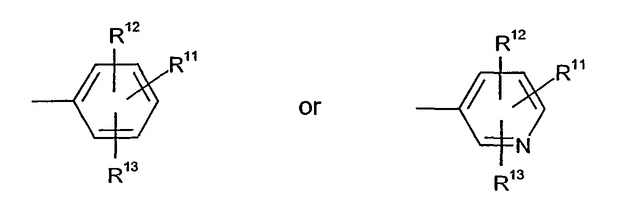 wherein
R¹¹ is selected from hydrogen, C₁₋₆alkyl, hydroxy, C₁₋₆ alkoxy, cyano, nitro, halo, C₁₋₆haloalkyl, XCO₂R¹⁶, -XC(O)NR¹⁵R¹⁶, -XNR¹⁴C(O)R¹⁵, -XNR¹⁴C(O)NR¹⁵R¹⁶, -XNR¹⁴C(O)NC(O)NR¹⁵R¹⁶, -XNR¹⁴SO₂R¹⁵, -XSO₂NR¹⁷R¹⁸, XSR¹⁴, XSOR¹⁴, XSO₂R¹⁴, -XNR¹⁵R¹⁶ -XNR¹⁴C(O)OR¹⁵, or XNR¹⁴SO₂NR¹⁵R¹⁶, or R¹¹ is selected from -X-aryl, -X-hetaryl, or -X-(aryloxy), each optionally substituted by 1 or 2 groups independently selected from hydroxy, C₁₋₆alkoxy, halo, C₁₋₆alkyl, C₁₋₆haloalkyl, cyano, nitro, CONR¹⁵R¹⁶ -NR¹⁴C(O)R¹⁵ SR¹⁴, SOR¹⁴, -SO₂R¹⁴, -SO₂NR¹⁷R¹⁸ -CO₂R¹⁶, -NR¹⁵R¹⁶ or hetaryl optionally substituted by 1 or 2 groups independently selected from hydroxy, C₁₋₆alkoxy, halo, C₁₋₆alkyl, or C₁₋₆haloalkyl;
X is -(CH₂)ᵣ - or C₂₋₆ alkenylene;
r is an integer from 0 to 6, preferably 0 to 4;
R¹⁴ and R¹⁵ are independently selected from hydrogen, C₁₋₆alkyl, C₃₋₇cycloalkyl, aryl, hetaryl, hetaryl(C₁₋₆alkyl)- and aryl(C₁₋₆alkyl)- and R¹⁴ and R¹⁵ are each independently optionally substituted by 1 or 2 groups independently selected from halo, C₁₋₆alkyl, C₃₋₇ cycloalkyl, C₁₋₆ alkoxy, C₁₋₆haloalkyl, -NHC(O)(C₁₋₆alkyl), -SO₂(C₁₋₆alkyl), -SO₂(aryl), -CO₂H, and -CO₂(C₁₋₄alkyl), -NH₂, -NH(C₁₋₆alkyl), aryl(C₁₋₆alkyl)-, aryl(C₂₋₆alkenyl)-, aryl(C₂₋₆alkynyl)-, hetaryl(C₁₋₆alkyl)-, -NHSO₂aryl, -NH(hetarylC₁₋₆alkyl), -NHSO₂hetaryl, -NHSO₂(C₁₋₆alkyl), -NHC(O)aryl, or -NHC(O)hetaryl:
or R¹⁴ and R¹⁵, together with the nitrogen atom to which they are bonded, form a 5-, 6- or 7- membered nitrogen - containing ring;
or where R¹¹ is -XNR¹⁴C(O)NR¹⁵R¹⁶ , R¹⁴ and R¹⁵ may, together with the -NC(O)N- portion of the group R¹ to which they are bonded, form a saturated or unsaturated ring, preferably a 5-, 6-, or 7- membered ring, for example an imidazolidine ring, such as imidazolidine-2,4-dione;
or where R¹¹ is -XNR¹⁴C(O)OR¹⁵, R¹⁴ and R¹⁵may, together with the -NC(O)O- portion of the group R¹¹ to which they are bonded, form a saturated or unsaturated ring, preferably a 5-, 6-, or 7- membered ring, for example an oxazolidine ring, such as oxazolidine-2,4-dione;
R¹⁶ is selected from hydrogen, C₁₋₆alkyl and C₃₋₇ cycloalkyl;
or where R¹¹ is -XC(O)NR¹⁵R¹⁶ or -XNR¹⁴C(O)NR¹⁵R¹⁶ , R¹⁵ and R¹⁶ may, together with the nitrogen to which they are bonded, form a 5-, 6-, or 7- membered nitrogen containing ring;
R¹⁷ and R¹⁸ are independently selected from hydrogen, C₁₋₆alkyl, C₃₋₇cycloalkyl, aryl, hetaryl, hetaryl(C₁₋₆alkyl)- and aryl(C₁₋₆alkyl)-, or R¹⁷ and R¹⁸, together with the nitrogen to which they are bonded, form a 5-, 6-, or 7- membered nitrogen containing ring; and R¹⁷ and R¹⁸ are each optionally substituted by one or two groups independently selected from halo, C₁₋₆alkyl, and C₃₋₇cydoalkyl, C₁₋₆haloalkyl ;
R¹² is selected from hydrogen, hydroxy, C₁₋₆alkyl, C₁₋₆alkoxy), halo, aryl, aryl(C₁₋₆alkyl)-, C₁₋₆haloalkoxy, and C₁₋₆haloalkyl ;
R¹³ is selected from hydrogen, hydroxy, C₁₋₆alkyl, C₁₋₆alkoxy, halo, aryl, aryl(C₁₋₆alkyl)-, C₁₋₆haloalkoxy, and C₁₋₆haloalkyl ;
R¹ and R² are independently selected from hydrogen and C₁₋₄ alkyl with the proviso that the total number of carbon atoms in R¹ and R² is not more than 4;
one of R^{1a} and R^{2a} is selected from hydrogen and C₁₋₄alkyl, and the other of R^{1a} and R^{2a} represents hydrogen or C₁₋₄alkyl with the proviso that the total number of carbon atoms in R^{1a} and R^{2a} is not more than 4;
m is an integer of from 1 to 3;
n is an integer of from 1 to 4; and
p is zero or an integer of from 1 to 3;
and ---- represents a single or double bond.

In a particular embodiment, the invention provides a compound of formula (I) or a salt, solvate, or physiologically functional derivative thereof, as defined herein, wherein R^{1a} and R^{2a} each represent hydrogen;
and in the group Ar¹, either:
R⁴ represents halogen, -(CH₂)_{q}OR⁷, -NR⁷C(O)R⁸, -NR⁷SO₂R⁸, -SO₂NR⁷R⁸, -NR⁷R⁸, -OC(O)R⁹ or OC(O)NR⁷R⁸, and R³ represents hydrogen or C₁₋₄ alkyl;
or:
R⁴ represents -NHR¹⁰ and R³ and -NHR¹⁰ together form a 5- or 6- membered heterocyclic ring;

In the compounds of formula (I) the group Ar¹ is preferably selected from groups (a) and (b) above. In said groups (a) and (b), when R⁴ represents halogen this is preferably chlorine or fluorine. R⁷ and R⁸ preferably each independently represent hydrogen or methyl. R⁹ preferably represents substituted phenyl. The integer q preferably represents zero or 1. Thus for example -(CH₂)_{q}OR⁷ preferably represents OH or -CH₂OH; NR⁷C(O)R⁸ preferably represents -NHC(O)H; -SO₂NR⁷R⁸ preferably represents -SO₂NH₂ or SO₂NHCH₃; NR⁷R⁸ preferably represents -NH₂; -OC(O)R⁹ preferably represents substituted benzoyloxy eg. OC(O)-C₆H₄-(p-CH₃); and -OC(O)N R⁷ R⁸ preferably represents OC(O)N(CH₃)₂.

When R⁴ represents NHR¹⁰ and together with R³ forms a 5- or 6- membered heterocyclic ring -NHR¹⁰-R³- preferably represents a group:
- NH-CO-R¹⁹- where R¹⁹ is an alkyl, alkenyl or alkyloxy moiety;
- NH-SO₂R²⁰- where R²⁰ is an alkyloxy moiety;
- NH-R²¹- where R²¹ is an alkyl or alkenyl moiety optionally substituted by COOR²² where R²² is C₁₋₄ alkyl; or
   NH-CO-S-;
wherein said alkyl and alkenyl groups and moieties contain 1 or 2 carbon atoms.

Particularly preferred groups (a) and (b) may be selected from the following groups (i) to (xxi): 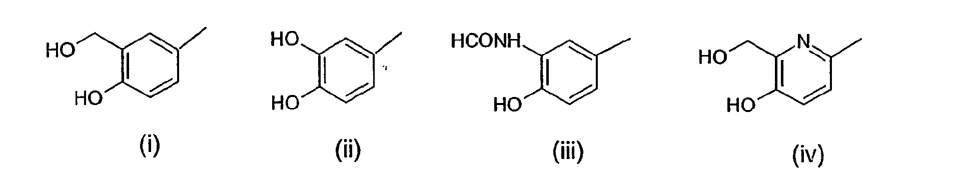 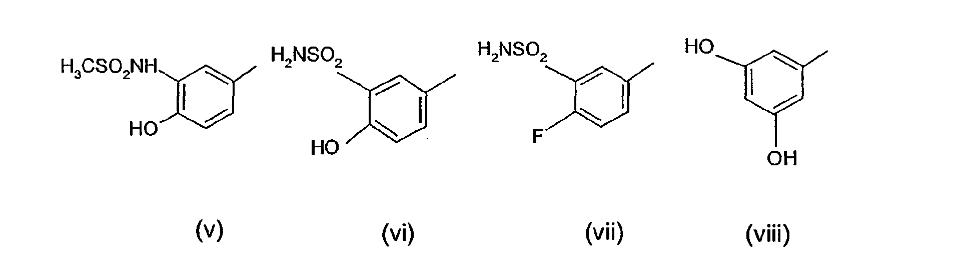 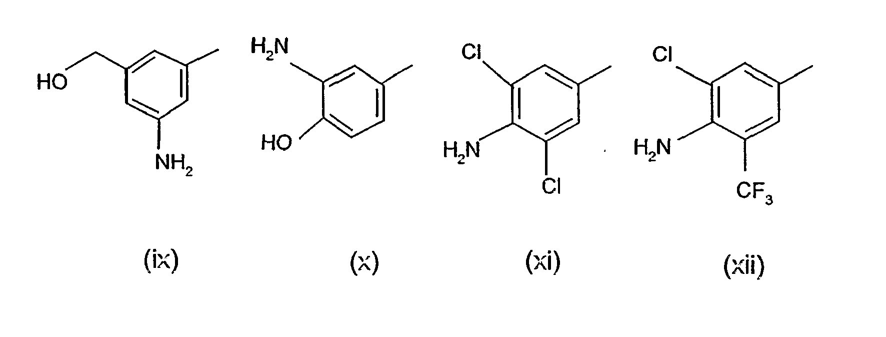 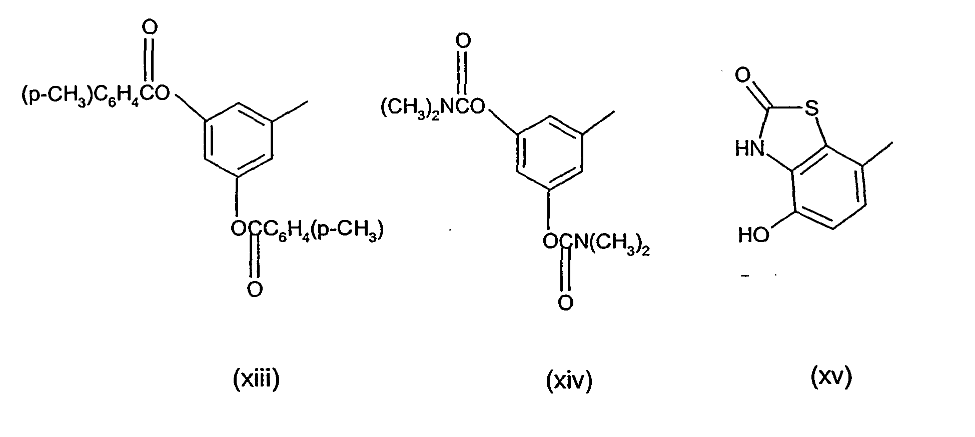 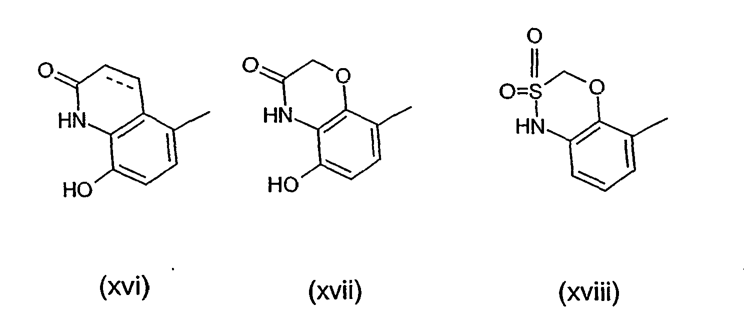 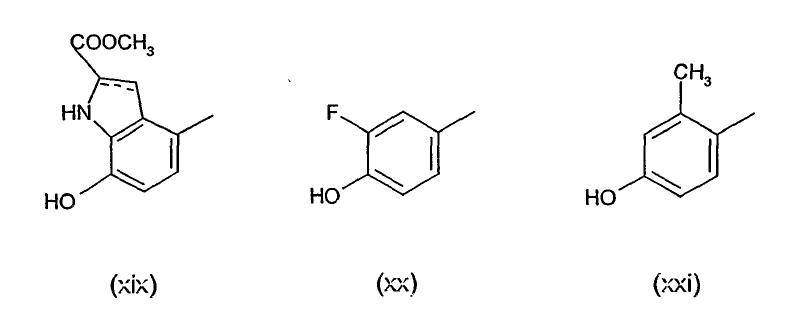 wherein the dotted line in (xv) represents a single or double bond.

Most preferably Ar¹ represents a group (i).

In the compounds of formula (I), in the group Ar² the group R¹¹ is suitably selected from hydrogen, C₁₋₄alkyl, hydroxy, halo, -NR¹⁴C(O)NR¹⁵R¹⁶, -NR¹⁴SO₂R¹⁵ and XSO₂NR¹⁷R⁸
wherein R¹⁴ to R¹⁸ are as defined above or more suitably wherein R¹⁴ is hydrogen and R¹⁵ is selected from hydrogen, C₁₋₆alkyl, C₃₋₆cycloalkyl and aryl and is optionally substituted as described above.

R¹¹ may also suitably be selected from cyano, -CONR¹⁵R¹⁶, SR¹⁴, SOR¹⁴ and SO₂R¹⁴,
wherein R¹⁴, R¹⁵ and R¹⁶ are as defined above, or more suitably wherein R¹⁴ is selected from C₁₋₆alkyl, or C₃₋₇cycloalkyl, and R¹⁵ and R¹⁶ are independently selected from hydrogen and C₁₋₆alkyl.

In the compounds of formula (I) R¹² and R¹³ preferably each represent hydrogen.

When R¹¹ represents hydrogen, R¹² and R¹³ may suitably each represent halogen, eg. chlorine or C₁₋₆alkyl eg. methyl.

In the compounds of formula (I), R¹ and R² are preferably independently selected from hydrogen and methyl, more preferably R¹ and R² are both hydrogen.

R^{1a} and R^{2a} are preferably independently selected from hydrogen and methyl, more preferably R^{1a} and R^{2a} are both hydrogen.

In the compounds of formula (I), each of m and n independently is suitably 1 or 2; p is suitably zero or 1.

In the compounds of formula (I), the group R¹¹ is preferably attached to the meta-position relative to the O(CH₂)ₚGR^{1a}R^{2a}-, link.

In the definition of X, the term alkenylene includes both *cis* and trans structures. Examples of suitable alkenylene groups include -CH=CH-.

X preferably represents (CH₂)ᵣ wherein R is 0, 1 or 2, or C₂-alkenylene.

In the compounds of formula (I) an aryl group or moiety may be for example phenyl or naphthyl.

In the compounds of formula (I) hetaryl group may be for example pyrrolyl, furyl, thienyl, pyridinyl, pyrazinyl, pyridazinyl, imidazolyl, tetrazolyl, tetrahydrofuranyl, oxazolyl, thiazolyl or thiadiazolyl.

It is to be understood that the present invention covers all combinations of particular and preferred groups described hereinabove.

Preferred compounds of the invention include:
4-((1*R*)-2-{[2-((3R)-3-{[(2,6-Dichlorobenzyl)oxy]methyl}-2,3-dihydro-1,4-benzodioxin-6-yl)ethyl]amino}-1-hydroxyethyl)-2-(hydroxymethyl)phenol;
4-{(1*R*)-2-[(2-{(3*R*)-3-[(Benzyloxy)methyl]-2,3-dihydro-1,4-benzodioxin-6-yl}ethyl)amino]-1-hydroxyethyl}-2-(hydroxymethyl)phenol;
4-{(1 1*R*)-2-[{2-{(3*S*)-3-[(Benzyloxy)methyl]-2,3-dihydro-1,4-benzodioxin-6-yl}ethyl)amino]-1-hydroxyethyl}-2-(hydroxymethyl)phenol;
2-(Hydroxymethyl)-4-{(1*R*)-1-hydroxy-2-[(2-{(3*R*)-3-[(pyridin-3-ylmethoxy)methyl]-2,3-dihydro-1,4-benzodioxin-6-yl}ethyl)amino]ethyl}phenol;
4-((1*R*)-2-{[2-((3*R*)-3-{[(6-Chloropyridin-3-yl)methoxy]methyl}-2,3-dihydro-1,4-benzodioxin-6-yl)ethyl]amino}-1-hydroxyethyl)-2-(hydroxymethyl)phenol;
4-((1R)-2-{[2-((3R)-3-{[(2,6-Dichloropyridin-3-yl)methoxy]methyl}-2,3-dihydro-1,4-benzodioxin-6-yl)ethyl]amino}-1-hydroxyethyl)-2-(hydroxymethyl)phenol;
4-{(1*R*)-2-[(2-(2-[(Benzyloxy)methyl]-2,3-dihydro-1,4-benzodioxin-6-yl}ethyl)amino]-1-hydroxyethyl}-2-(hydroxymethyl)phenol;
4-((1*R*)-2-{[2-((3*R*)-3-{[(5-Bromopyridin-3-yl)methoxy]methyl}-2,3-dihydro-1,4-benzodioxin-6-yl)ethyl]amino}-1-hydroxyethyl)-2-(hydroxymethyl)phenol;
3-[({(2*R*)-7-[2-({(2*R*)-2-Hydroxy-2-[4-hydroxy-3-(hydroxymethyl)phenyl]ethyl}amino)ethyl]-2,3-dihydro-1,4-benzodioxin-2-yl}methoxy)methyl]benzonitrile;
3-[({(2*R*)-7-[2-({(2*R*)-2-Hydroxy-2-[4-hydroxy-3-(hydroxymethyl)phenyl]ethyl}amino)ethyl]-2,3-dihydro-1,4-benzodioxin-2-yl}methoxy)methyl]benzamide;
4-[(1*R*)-2-({2-[(3*R*)-3-({[3-(Cyclopentylthio)benzyl]oxy}methyl)-2,3-dihydro-1,4-benzodiaxin-6-yl]ethyl}amino)-1-hydroxyethyl]-2-(hydroxymethyl)phenol;
4-[(1*R*)-2-({2-[(3*R*)-3-({[3-(Cyclopentylsulfonyl)benzyl]oxy}methyl)-2,3-dihydro-1,4-benzodioxin-6-yl]ethyl}amino)-1-hydroxyethyl)-2-(hydroxymethyl)phenol;
2-(Hydroxymethyl)-4-{(1*R*)-1-hydroxy-2-[(2-{(3*R*)-3-[({5-[4-(methylsulfinyl)phenyl]pyridin-3-yl}methoxy)methyl]-2,3-dihydro-1,4-benzodioxin-6-yl}ethyl)amino]ethyl}phenol;
N-{3-[({(2*R*)-7-[2-({(2*R*)-2-Hydroxy-2-[4-hydroxy-3-(hydroxymethyl)phenyl]ethyl}amino)ethyl]-2,3-dihydro-1,4-benzodioxin-2-yl}methoxy)methyl]phenyl}urea;
4-((1*R*)-2-{[2-((3*R*)-3-{[(4-Chlorobenzyl)oxy]methyl}-2,3-dihydro-1,4-benzodioxin-6-yl)ethyl]amino}-1-hydroxyethyl)-2-(hydroxymethyl)phenol;
4-((1*R*)-2-{[2-((3*R*)-3-{[(4-Fluorobenzyl)oxy]methyl}-2,3-dihydro-1,4-benzodioxin-6-yl)ethyl]amino}-1-hydroxyethyl)-2-(hydroxymethyl)phenol;
4-((1*R*)-2-{[2-((3*R*)-3-{[(3,5-Dimethylbenzyl)oxy]methyl}-2,3-dihydro-1,4-benzodioxin-6-yl)ethyl]amino}-1-hydroxyethyl)-2-(hydroxymethyl)phenol;
2-(Hydroxymethyl)-4-{(1*R*)-1-hydroxy-2-[(2-{(3*R*)-3-[(1-phenylethoxy)methyl]-2,3-dihydro-1,4-benzodioxin-6-yl}ethyl)amino]ethyl}phenol;
2-(Hydroxymethyl)-4-[(1*R*)-1-hydroxy-2-({2-[(3*R*)-3-({[3-(methylsulfonyl)benzyl]oxy}methyl)-2,3-dihydro-1,4-benzodioxin-6-yl]ethyl}amino)ethyl]phenol;
4-((1*R*)-2-{[2-((3*R*)-3-{[3-(2,6-Dichlorophenyl)propoxy]methyl}-2,3-dihydro-1,4-benzodioxin-6-yl)ethyl]amino}-1-hydroxyethyl)-2-(hydroxymethyl)phenol;
3-[({(2*R*)-7-[2-({(2*R*)-2-Hydroxy-2-[4-hydroxy-3-(hydroxymethyl)phenyl]ethyl}amino)ethyl]-2,3-dihydro-1,4-benzodioxin-2-yl}methoxy)methyl]benzenesulfonamide;
6-{2-[(2-{(3*R*)-3-[(Benzyloxy)methy)]-2,3-dihydro-1,4-benzodioxin-6-yl}ethyl)amino]-1 - hydroxyethyl}-2-(hydroxymethyl)pyridin-3-ol;
N-(5-{(1*R*)-2-[(2-{(3*R*)-3-[(Benzyloxy)methyl]-2,3-dihydro-1,4-benzodioxin-6-yl}ethyl)amino]-1-hydroxyethyl}-2-hydroxyphenyl)methanesulfonamide;
4-{(1*R*)-2-[(2-{(3*R*)-3-[(Benzy)oxy)methyl]-2,3-dihydro-1,4-benzodioxin-6-yl}ethyl)amino]-1 - hydroxyethyl}-2-fluorophenol;
4-{(1*R*)-2-[(2-((3*R*)-3-[(Benzyloxy)methyl]-2,3-dihydro-1 ,4-benzodioxin-6-yl)ethyl)amino]-1-hydroxyethyl}-3-methylphenol;
(1 *R*)-1-(4-Amino-3,5-dichlorophenyl)-2-[(2-{(3R)-3-[(benzyloxy)methyl]-2,3-dihydro-1,4-benzodioxin-6-yl}ethyl)amino]ethanol;
5-{(1*R*)-2-[(2-((3*R*)-3-[(Benzyloxy)methyl]-2,3-dihydro-1,4-benzodioxin-6-yl)ethyl)amino]-1-hydroxyethyl}-2-hydroxyphenylformamide;
and salts, solvates.

The compounds of formula (I) include an asymmetric centre, namely the carbon atom of the 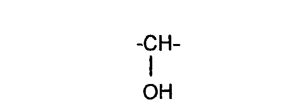 group. The present invention includes both (S) and (R) enantiomers either in substantially pure form or admixed in any proportions. Preferably, the compounds of the invention are in the form of the (R) enantiomers.

Similarly, where R¹ and R² are different groups, or where R^{1a} and R^{2a} are different groups the carbon atom to which they are attached is an asymmetric centre and the present invention includes both (S) and (R) enantiomers at this centre either in substantially pure form or admixed in any proportions.

Furthermore, when ---- represents a single bond, the carbon atom in the benzodioxan ring to which the moiety -(CH₂)ₙ- is attached will represent a further asymmetric centre and the present invention includes both (S) and (R) enantiomers at this centre either in substantially pure form or admixed in any proportions.

Thus the compounds of formula (I) include all enantiomers and diastereoisomers as well as mixtures thereof in any proportions.

Salts and solvates of compounds of formula (I) which are suitable for use in medicine are those wherein the counterion or associated solvent is pharmaceutically acceptable. However, salts and solvates having non-pharmaceutically acceptable counterions or associated solvents are within the scope of the present invention, for example, for use as intermediates in the preparation of other compounds of formula (I) and their pharmaceutically acceptable salts, solvates.

Suitable salts according to the invention include those formed with both organic and inorganic acids or bases. Pharmaceutically acceptable acid addition salts include those formed from hydrochloric, hydrobromic, sulphuric, citric, tartaric, phosphoric, lactic, pyruvic, acetic, trifluoroacetic, triphenylacetic, sulphamic, sulphanilic, succinic, oxalic, fumaric, maleic, malic, glutamic, aspartic, oxaloacetic, methanesulphonic, ethanesulphonic, arylsulphonic (for example p-toluenesulphonic, benzenesulphonic, naphthalenesulphonic or naphthalenedisulphonic), salicylic, glutaric, gluconic, tricarballylic, cinnamic, substituted cinnamic (for example, phenyl, methyl , methoxy or halo substituted cinnamic, including 4-methyl and 4-methoxycinnamic acid), ascorbic, oleic, naphthoic, hydroxynaphthoic (for example 1- or 3-hydroxy-2-naphthoic), naphthaleneacrylic (for example naphthalene-2-acrylic), benzoic, 4-methoxybenzoic, 2- or 4-hydroxybenzoic, 4-chlorobenzoic, 4-phenylbenzoic, benzeneacrylic (for example 1,4-benzenediacrylic) and isethionic acids. Pharmaceutically acceptable base salts include ammonium salts, alkali metal salts such as those of sodium and potassium, alkaline earth metal salts such as those of calcium and magnesium and salts with organic bases such as dicyclohexyl amine and N-methyl-D-glucamine.

Pharmaceutically acceptable esters of the compounds of formula (I) may have a hydroxyl group converted to a C₁₋₆alkyl, aryl, aryl C₁₋₆ alkyl, or amino acid ester.

As mentioned above, the compounds of formula (I) are selective β₂-adrenoreceptor agonists as demonstrated using functional or reporter gene readout from cell lines transfected with human beta-adrenoreceptors, or membranes derived from these cells, as described below. Compounds according to the present invention also have the potential to combine long duration of effect with rapid onset of action. Furthermore, certain compounds have shown an improved therapeutic index in animal models relative to existing long-acting β₂-agonist bronchodilators. As such, compounds of the invention may be suitable for once-daily administration.

Therefore, compounds of formula (I) and their pharmaceutically acceptable salts and solvates have use in the prophylaxis and treatment of clinical conditions for which a selective β₂-adrenoreceptor agonist is indicated. Such conditions include diseases associated with reversible airways obstruction such as asthma, chronic obstructive pulmonary diseases (COPD) (e.g. chronic and wheezy bronchitis, emphysema), respiratory tract infection and upper respiratory tract disease.

Other conditions which may be treated include premature labour, depression, congestive heart failure, skin diseases (e.g. inflammatory, allergic, psoriatic, and proliferative skin diseases), conditions where lowering peptic acidity is desirable (e.g. peptic and gastric ulceration) and muscle wasting disease.

Accordingly, the present invention provides compounds of formula (I) or a pharmaceutical acceptable salt, solvate for use in a method for the prophylaxis or treatment of a clinical condition in a mammal, such as a human, for which a selective β₂-adrenoreceptor agonist is indicated, which comprises administration of a therapeutically effective amount of a compound of formula (I) or a pharmaceutically acceptable salt, solvate. In particular, they are involved in such a method for the prophylaxis or treatment of a disease associated with reversible airways obstruction such as asthma, chronic obstructive pulmonary disease (COPD), respiratory tract infection or upper respiratory tract disease. In a further aspect the present invention provides such a method for the prophylaxis or treatment of a clinical condition selected from premature labour, depression, congestive heart failure, skin diseases (e.g. inflammatory, allergic, psoriatic, and proliferative skin diseases), conditions where lowering peptic acidity is desirable (e.g. peptic and gastric ulceration) or muscle wasting disease.

In the alternative, there is also provided a compound of formula (I) or a pharmaceutically acceptable salt, solvate, for use in medical therapy, particularly, for use in the prophylaxis or treatment of a clinical condition in a mammal, such as a human, for which a selective β₂-adrenoreceptor agonist is indicated. In particular, there is provided a compound of formula (I) or a pharmaceutically acceptable salt, solvate, for the prophylaxis or treatment of a disease associated with reversible airways obstruction such as asthma, chronic obstructive pulmonary disease (COPD), respiratory tract infection or upper respiratory tract disease. In a further aspect, there is provided a compound of formula (I) or a pharmaceutically acceptable salt, solvate for the prophylaxis or treatment of a clinical condition selected from premature labour, depression, congestive heart failure, skin diseases (e.g. inflammatory, allergic, psoriatic, and proliferative skin diseases), conditions where lowering peptic acidity is desirable (e.g. peptic and gastric ulceration) or muscle wasting disease.

The present invention also provides the use of a compound of formula (I) or a pharmaceutically acceptable salt, solvate in the manufacture of a medicament for the prophylaxis or treatment of a clinical condition for which a selective β₂-adrenoreceptor agonist is indicated, for example a disease associated with reversible airways obstruction such as asthma, chronic obstructive pulmonary disease (COPD), respiratory tract infection or upper respiratory tract disease. In a further aspect, there is provided a compound of formula (I) or a pharmaceutically acceptable salt, solvate, or physiologically functional derivative thereof in the manufacture of a medicament for the prophylaxis or treatment of a clinical condition selected from premature labour, depression, congestive heart failure, skin diseases (e.g. inflammatory, allergic, psoriatic, and proliferative skin diseases), conditions where lowering peptic acidity is desirable (e.g. peptic and gastric ulceration) and muscle wasting disease.

The amount of a compound of formula (I) or a pharmaceutically acceptable salt, solvate which is required to achieve a therapeutic effect will, of course, vary with the particular compound, the route of administration, the subject under treatment, and the particular disorder or disease being treated. The compounds of the invention may be administered by inhalation at a dose of from 0.0005mg to 10 mg, preferably 0.005mg to 0.5mg, eg. 0.05mg to 0.5mg. The dose range for adult humans is generally from 0.0005 mg to 10mg per day and preferably 0.01 mg to 1 mg per day, most preferably 0.05mg to 0.5mg per day.

While it is possible for a compound of formula (I) or a pharmaceutically acceptable salt, solvate to be administered alone, it is preferable to present it as a pharmaceutical formulation.

Accordingly, the present invention further provides a pharmaceutical formulation comprising a compound of formula (I) or a pharmaceutically acceptable salt, solvate and a pharmaceutically acceptable carrier or excipient, and optionally one or more other therapeutic ingredients.

Hereinafter, the term "active ingredient" means a compound of formula (I) or a pharmaceutically acceptable salt, solvate.

The formulations include those suitable for oral, parenteral (including subcutaneous, intradermal, intramuscular, intravenous and intraarticular), inhalation (including fine particle dusts or mists which may be generated by means of various types of metered dose pressurised aerosols, nebulisers or insufflators), rectal and topical (including dermal, buccal, sublingual and intraocular) administration although the most suitable route may depend upon for example the condition and disorder of the recipient. The formulations may conveniently be presented in unit dosage form and may be prepared by any of the methods well known in the art of pharmacy. All methods include the step of bringing the active ingredient into association with the carrier which constitutes one or more accessory ingredients. In general the formulations are prepared by uniformly and intimately bringing into association the active ingredient with liquid carriers or finely divided solid carriers or both and then, if necessary, shaping the product into the desired formulation.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein.

Formulations for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilised) condition requiring only the addition of the sterile liquid carrier, for example saline or water-for-injection, immediately prior to use. Extemporaneous injection solutions and suspensions may be prepared from sterile powders, granules and tablets of the kind previously described.

Dry powder compositions for topical delivery to the lung by inhalation may, for example, be presented in capsules and cartridges of for example gelatine, or blisters of for example laminated aluminium foil, for use in an inhaler or insufflator. Powder blend formulations generally contain a powder mix for inhalation of the compound of the invention and a suitable powder base (carrier/diluent/excipient substance) such as mono-, di or polysaccharides (eg. lactose or starch). Use of lactose is preferred.

Each capsule or cartridge may generally contain between 20µg-10mg of the compound of formula (I) optionally in combination with another therapeutically -active ingredient. Alternatively, the compound of the invention may be presented without excipients. Packaging of the formulation may be suitable for unit dose or multi-dose delivery. In the case of multi-dose delivery, the formulation can be pre-metered (eg as in Diskus, see GB 2242134, US Patent Nos. 6,632,666, 5,860,419, 5,873,360 and 5,590,645 or Diskhaler, see GB 2178965, 2129691 and 2169265, US Patent No.s 4,778,054, 4,811,731, 5,035,237, the disclosures of which are hereby incorporated by reference) or metered in use (eg as in Turbuhaler, see EP 69715 or in the devices described in US Patents No. 6,321,747 the disclosures of which are hereby incorporated by reference). An example of a unit-dose device is Rotahaler (see GB 2064336 and US Patent No. 4,353,656, the disclosures of which are hereby incorporated by reference). The Diskus inhalation device comprises an elongate strip formed from a base sheet having a plurality of recesses spaced along its length and a lid sheet hermetically but peelably sealed thereto to define a plurality of containers, each container having therein an inhalable formulation containing a compound of formula (I) preferably combined with lactose. Preferably, the strip is sufficiently flexible to be wound into a roll. The lid sheet and base sheet will preferably have leading end portions which are not sealed to one another and at least one of the said leading end portions is constructed to be attached to a winding means. Also, preferably the hermetic seal between the base and lid sheets extends over their whole width. The lid sheet may preferably be peeled from the base sheet in a longitudinal direction from a first end of the said base sheet.

Spray compositions for topical delivery to the lung by inhalation may for example be formulated as aqueous solutions or suspensions or as aerosols delivered from pressurised packs, such as a metered dose inhaler, with the use of a suitable liquefied propellant. Aerosol compositions suitable for inhalation can be either a suspension or a solution and generally contain the compound of formula (I) optionally in combination with another therapeutically active ingredient and a suitable propellant such as a fluorocarbon or hydrogen-containing chlorofluorocarbon or mixtures thereof, particularly hydrofluoroalkanes, e.g. dichlorodifluoromethane, trichlorofluoromethane, dichlorotetrafluoroethane, especially 1,1,1,2-tetrafluoroethane, 1,1,1,2,3,3,3-heptafluoro-n-propane or a mixture thereof. Carbon dioxide or other suitable gas may also be used as propellant. The aerosol composition may be excipient free or may optionally contain additional formulation excipients well known in the art such as surfactants eg oleic acid or lecithin and cosolvents eg ethanol. Pressurised formulations will generally be retained in a canister (eg an aluminium canister) closed with a valve (eg a metering valve) and fitted into an actuator provided with a mouthpiece.

Medicaments for administration by inhalation desirably have a controlled particle size. The optimum particle size for inhalation into the bronchial system is usually 1-10µm, preferably 2-5µm. Particles having a size above 20µm are generally too large when inhaled to reach the small airways. To achieve these particle sizes the particles of the active ingredient as produced may be size reduced by conventional means eg by micronisation. The desired fraction may be separated out by air classification or sieving. Preferably, the particles will be crystalline. When an excipient such as lactose is employed, generally, the particle size of the excipient will be much greater than the inhaled medicament within the present invention. When the excipient is lactose it will typically be present as milled lactose,
wherein not more than 85% of lactose particles will have a MMD of 60-90µm and not less than 15% will have a MMD of less than 15µm.

Intranasal sprays may be formulated with aqueous or non-aqueous vehicles with the addition of agents such as thickening agents, buffer salts or acid or alkali to adjust the pH, isotonicity adjusting agents or anti-oxidants.

Solutions for inhalation by nebulation may be formulated with an aqueous vehicle with the addition of agents such as acid or alkali, buffer salts, isotonicity adjusting agents or antimicrobials. They may be sterilised by filtration or heating in an autoclave, or presented as a non-sterile product.

Formulations for rectal administration may be presented as a suppository with the usual carriers such as cocoa butter or polyethylene glycol.

Formulations for topical administration in the mouth, for example buccally or sublingually, include lozenges comprising the active ingredient in a flavoured basis such as sucrose and acacia or tragacanth, and pastilles comprising the active ingredient in a basis such as gelatin and glycerin or sucrose and acacia.

Preferred unit dosage formulations are those containing an effective dose, as hereinbefore recited, or an appropriate fraction thereof, of the active ingredient.

It should be understood that in addition to the ingredients particularly mentioned above, the formulations of this invention may include other agents conventional in the art having regard to the type of formulation in question, for example those suitable for oral administration may include flavouring agents.

The compounds and pharmaceutical formulations according to the invention may be used in combination with or include one or more other therapeutic agents, for example selected from anti-inflammatory agents, anticholinergic agents (particularly an M₁, M₂, M₁/M₂ or M₃ receptor antagonist), other β₂-adrenoreceptor agonists, antiinfective agents (e.g. antibiotics, antivirals), or antihistamines. The invention thus provides, in a further aspect, a combination comprising a compound of formula (I) or a pharmaceutically acceptable salt, solvate together with one or more other therapeutically active agents, for example selected from an anti-inflammatory agent (for example a corticosteroid or an NSAID), an anticholinergic agent, another β₂-adrenoreceptor agonist, an antiinfective agent (e.g. an antibiotic or an antiviral), or an antihistamine. Preferred are combinations comprising a compound of formula (I) or a pharmaceutically acceptable salt, solvate together with a corticosteroid, and/or an anticholinergic, and/or a PDE-4 inhibitor. Preferred combinations are those comprising one or two other therapeutic agents.

It will be clear to a person skilled in the art that, where appropriate, the other therapeutic ingredient(s) may be used in the form of salts, (e.g. as alkali metal or amine salts or as acid addition salts), or prodrugs, or as esters (e.g. lower alkyl esters), or as solvates (e.g. hydrates) to optimise the activity and/or stability and/or physical characteristics (e.g. solubility) of the therapeutic ingredient. It will be clear also that where appropriate, the therapeutic ingredients may be used in optically pure form.

Suitable anti-inflammatory agents include corticosteroids and NSAIDs. Suitable corticosteroids which may be used in combination with the compounds of the invention are those oral and inhaled corticosteroids and their pro-drugs which have anti-inflammatory activity. Examples include methyl prednisolone, prednisolone, dexamethasone, fluticasone propionate, 6α,9α-difluoro-17α-[(2-furanylcarbonyl)oxy]-11β-hydroxy-16α-methyl-3-oxo-androsta-1,4-diene-17β-carbothlolc acid S-fluoromethyl ester, 6α,9α-difluoro-11β-hydroxy-16α-methyl-3-oxo-17α-propionyloxy- androsta-1,4-diene-17β-carbothioic acid S-(2-oxo-tetrahydro-furan-3S-yl)ester, beclomethasone esters (e.g. the 17-propionate ester or the 17,21-dipropionate ester), budesonide, flunisolide, mometasone esters (e.g. the furoate ester), triamcinolone acetonide, rofleponide, ciclesonide, butixocort propionate, RPR-106541, and ST-126. Preferred corticosteroids include fluticasone propionate, 6α,9α-difluoro-11β-hydroxy-16α-methyl-17α-[(4-methyl-1,3-thiazole-5-carbonyl)oxy]-3-oxo-androsta-1,4-diene-17β-carbothioic acid S-fluoromethyl ester and 6α,9α-difluoro-17β-[(2-furanylcarbonyl)oxy]-11β-hydroxy-16α-methyl-3-oxo-androsta-1,4-diene-17β-carbothioic acid S-fluoromethyl ester, more preferably 6α,9α-difluoro-17α-[(2-furanylcarbonyl)oxy]-1 1β-hydroxy-16α-methyl-3-oxo-androsta-1,4-diene-17β-carbothioic acid S-fluoromethyl ester.

Suitable NSAIDs include sodium cromoglycate, nedocromil sodium, phosphodiesterase (PDE) inhibitors (e.g. theophylline, PDE4 inhibitors or mixed PDE3/PDE4 inhibitors), leukotriene antagonists, inhibitors of leukotriene synthesis, iNOS inhibitors, tryptase and elastase inhibitors, beta-2 integrin antagonists and adenosine receptor agonists or antagonists (e.g. adenosine 2a agonists), cytokine antagonists (e.g. chemokine antagonists) or inhibitors of cytokine synthesis. Suitable other β₂-adrenoreceptor agonists include salmeterol (e.g. as the xinafoate), salbutamol (e.g. as the sulphate or the free base), formoterol (e.g. as the fumarate), fenoterol or terbutaline and salts thereof.

Of particular interest is use of the compound of formula (I) in combination with a phosphodiesterase 4 (PDE4) inhibitor or a mixed PDE3/PDE4 inhibitor. The PDE4-specific inhibitor useful in this aspect of the invention may be any compound that is known to inhibit the PDE4 enzyme or which is discovered to act as a PDE4 inhibitor, and which are only PDE4 inhibitors, not compounds which inhibit other members of the PDE family as well as PDE4. Generally it is preferred to use a PDE4. inhibitor which has an IC₅₀ ratio of about 0.1 or greater as regards the IC₅₀ for the PDE4 catalytic form which binds rolipram with a high affinity divided by the IC₅₀ for the form which binds rolipram with a low affinity. For the purposes of this disclosure, the cAMP catalytic site which binds R and S rolipram with a low affinity is denominated the "low affinity" binding site (LPDE 4) and the other form of this catalytic site which binds rolipram with a high affinity is denominated the "high affinity" binding site (HPDE 4). This term "HPDE4" should not be confused with the term "hPDE4" which is used to denote human PDE4.

A method for determining IC₅₀s ratios is set out in US patent 5,998,428 which is incorporated herein in full by reference as though set out herein. See also PCT application WO 00/51599 for an another description of said assay.

The preferred PDE4 inhibitors of use in this invention will be those compounds which have a salutary therapeutic ratio, i.e., compounds which preferentially inhibit cAMP catalytic activity where the enzyme is in the form that binds rolipram with a low affinity, thereby reducing the side effects which apparently are linked to inhibiting the form which binds rolipram with a high affinity. Another way to state this is that the preferred compounds will have an IC₅₀ ratio of about 0.1 or greater as regards the IC₅₀ for the PDE4 catalytic form which binds rolipram with a high affinity divided by the IC₅₀ for the form which binds rolipram with a low affinity.

A further refinement of this standard is that of one wherein the PDE4 inhibitor has an IC₅₀ ratio of about 0.1 or greater; said ratio is the ratio of the IC₅₀ value for competing with the binding of 1nM of [³H]R-rolipram to a form of PDE4 which binds rolipram with a high affinity over the IC₅₀ value for inhibiting the PDE4 catalytic activity of a form which binds rolipram with a low affinity using 1 µM[³H]-cAMP as the substrate.

Most preferred are those PDE4 inhibitors which have an IC₅₀ ratio of greater than 0.5, and particularly those compounds having a ratio of greater than 1.0. Preferred compounds are *cis* 4-cyano-4-(3-cyclopentyloxy-4-methoxyphenyl)cyclohexan-1-carboxylic acid, 2-carbomethoxy-4-cyano-4-(3-cyclopropylmethoxy-4-difluoromethoxyphenyl)cyclohexan-1-one and *cis*-[4-cyano-4-(3-cyclopropylmethoxy-4-difluoromethoxyphenyl)cyclohexan-1-ol]; these are examples of compounds which bind preferentially to the low affinity binding site and which have an IC₅₀ ratio of 0.1 or greater.

### Other compounds of interest include:

Compounds set out in U.S. patent 5,552,438 issued 03 September, 1996; this patent and the compounds it discloses are incorporated herein in full by reference. The compound of particular interest, which is disclosed in U.S. patent 5,552,438, is *cis*-4-cyano-4-[3-(cyclopentyloxy)-4-methoxyphenyl]cyclohexane-1-carboxylic acid (also known as cilomalast) and its salts, esters, pro-drugs or physical forms; AWD-12-281 from elbion (Hofgen, N. et al. 15th EFMC Int Symp Med Chem (Sept 6-10, Edinburgh) 1998, Abst P.98; CAS reference No. 247584020-9); a 9-benzyladenine derivative nominated NCS-613 (INSERM); D-4418 from Chiroscience and Schering-Plough; a benzodiazepine PDE4 inhibitor identified as CI-1018 (PD-168787) and attributed to Pfizer; a benzodioxole derivative disclosed by Kyowa Hakko in WO99/16766; K-34 from Kyowa Hakko; V-11294A from Napp (Landells, L.J. et al. Eur Resp J [Annu Cong Eur Resp Soc (Sept 19-23, Geneva) 1998] 1998, 12 (Suppl. 28): Abst P2393); roflumilast (CAS reference No 162401-32-3) and a pthalazinone (WO99/47505, the disclosure of which is hereby incorporated by reference) from Byk-Gulden; Pumafentrine, (-)-p-[(4aR*,10*bS**)-9-ethoxy-1,2,3,4,4a,10b-hexahydro-8-methoxy-2-methylbenzo[c][1,6]naphthyridin-6-yl]-N,N-diisopropylbenzamide which is a mixed PDE3/PDE4 inhibitor which has been prepared and published on by Byk-Gulden, now Altana; arofylline under development by Almirall-Prodesfarma; VM554/UM565 from Vernalis; or T-440 (Tanabe Seiyaku; Fuji, K. et al. J Pharmacol Exp Ther,1998, 284(1): 162), and T2585.

Other possible PDE-4 and mixed PDE3/PDE4 inhibitors include those listed in WO01/13953, the disclosure of which is hereby incorporated by reference.

Suitable anticholinergic agents are those compounds that act as antagonists at the muscarinic receptor, in particular those compounds which are antagonists of the M₁ and M₂ receptors. Exemplary compounds include the alkaloids of the belladonna plants as illustrated by the likes of atropine, scopolamine, homatropine, hyoscyamine; these compounds are normally administered as a salt, being tertiary amines. These drugs, particularly the salt forms, are readily available from a number of commercial sources or can be made or prepared from literature data via, to wit:
Atropine - CAS-51-55-8 or CAS-51-48-1 (anhydrous form), atropine sulfate - CAS-5908-99-6; atropine oxide - CAS-4438-22-6 or its HCl salt - CAS-4574-60-1 and methylatropine nitrate - CAS-52-88-0.
Homatropine - CAS-87-00-3, hydrobromide salt - CAS-51-56-9, methylbromide salt - CAS-80-49-9.
Hyoscyamine (*d, l*) - CAS-101-31-5, hydrobromide salt - CAS-306-03-6 and sulfate salt - CAS-6835-16-1.
Scopolamine - CAS-51-34-3, hydrobromide salt - CAS-6533-68-2, methylbromide salt-CAS-155-41-9.

Preferred anticholinergics include ipratropium (e.g. as the bromide), sold under the name Atrovent, oxitropium (e.g. as the bromide) and tiotropium (e.g. as the bromide) (CAS-139404-48-1). Also of interest are: methantheline (CAS-53-46-3), propantheline bromide (CAS- 50-34-9), anisotropine methyl bromide or Valpin 50 (CAS- 80-50-2), clidinium bromide (Quarzan, CAS-3485-62-9), copyrrolate (Robinul), isopropamide iodide (CAS-71-81-8), mepenzolate bromide (U.S. patent 2,918,408), tridihexethyl chloride (Pathilone, CAS-4310-35-4), and hexocyclium methylsulfate (Tral, CAS-115-63-9). See also cyclopentolate hydrochloride (CAS-5870-29-1), tropicamide (CAS-1508-75-4), trihexyphenidyl hydrochloride (CAS-144-11-6), pirenzepine (CAS-29868-97-1), telenzepine (CAS-80880-90-9), AF-DX 116, or methoctramine, and the compounds disclosed in WO01/04118, the disclosure of which is hereby incorporated by reference.

Suitable antihistamines (also referred to as H₁-receptor antagonists) include any one or more of the numerous antagonists known which inhibit H₁-receptors, and are safe for human use. All are reversible, competitive inhibitors of the interaction of histamine with H₁-receptors. The majority of these inhibitors, mostly first generation antagonists, have a core structure, which can be represented by the following formula:

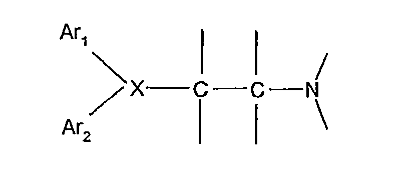

This generalized structure represents three types of antihistamines generally available: ethanolamines, ethylenediamines, and alkylamines. In addition, other first generation antihistamines include those which can be characterized as based on piperizine and phenothiazines. Second generation antagonists, which are non-sedating, have a similar structure-activity relationship in that they retain the core ethylene group (the alkylamines) or mimic the tertiary amine group with piperizine or piperidine. Exemplary antagonists are as follows:
Ethanolamines: carbinoxamine maleate, clemastine fumarate, diphenylhydramine hydrochloride, and dimenhydrinate.
Ethylenediamines: pyrilamine amleate, tripelennamine HCl, and tripelennamine citrate. Alkylamines: chloropheniramine and its salts such as the maleate salt, and acrivastine.
Piperazines: hydroxyzine HCI, hydroxyzine pamoate, cyclizine HCI, cyclizine lactate, meclizine HCI, and cetirizine HCl.
Piperidines: Astemizole, levocabastine HCI, loratadine or its descarboethoxy analogue, and terfenadine and fexofenadine hydrochloride or another pharmaceutically acceptable salt.
Azelastine hydrochloride is yet another H₁ receptor antagonist which may be used in combination with a PDE4 inhibitor.
Examples of preferred anti-histamines include methapyrilene and loratadine.

The invention thus provides, in a further aspect, a combination comprising a compound of formula (I) a pharmaceutically acceptable salt, solvate together with a PDE4 inhibitor.

The invention thus provides, in a further aspect, a combination comprising a compound of formula (I) a pharmaceutically acceptable salt, solvate together with a corticosteroid.

The invention thus provides, in a further aspect, a combination comprising a compound of formula (I) a pharmaceutically acceptable salt, solvate together with an anticholinergic.

The invention thus provides, in a further aspect, a combination comprising a compound of formula (I) a pharmaceutically acceptable salt, solvate together with an antihistamine.

The invention thus provides, in a further aspect, a combination comprising a compound of formula (I) a pharmaceutically acceptable salt, solvate together with a PDE4 inhibitor and a corticosteroid.

The invention thus provides, in a further aspect, a combination comprising a compound of formula (I) a pharmaceutically acceptable salt, solvate together with an anticholinergic and a PDE-4 inhibitor.

The combinations referred to above may conveniently be presented for use in the form of a pharmaceutical formulation and thus pharmaceutical formulations comprising a combination as defined above together with a physiologically acceptable diluent or carrier represent a further aspect of the invention.

The individual compounds of such combinations may be administered either sequentially or simultaneously in separate or combined pharmaceutical formulations. Appropriate doses of known therapeutic agents will be readily appreciated by those skilled in the art.

According to a further aspect of the invention, there is provided a process for preparing a compound of formula (I) or a salt, solvate, which comprises a process (a), or (b) as defined below, followed by the following steps in any order:
(i) optional removal of any protecting groups;
(ii) optional separation of an enantiomer from a mixture of enantiomers;
(iii) optional conversion of the product to a corresponding salt, solvate, or physiologically functional derivative thereof.

In one general process (a), a compound of formula (I), may be obtained by deprotection of a protected intermediate, for example of formula (II): 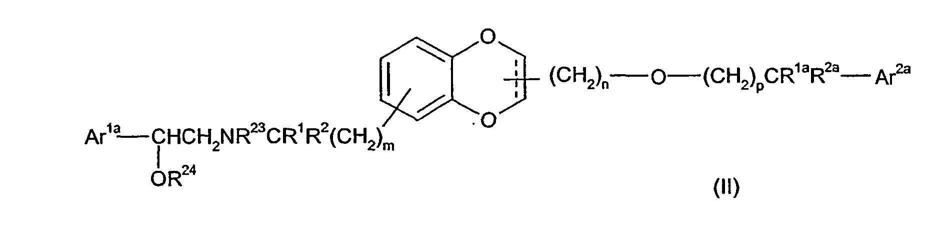 or a salt or solvate thereof, wherein R¹, R², R^{1a}, R^{2a}, m, n, p and ---- are as defined for the compound of formula (I), Ar^{1a} represents an optionally protected form of Ar¹; Ar^{2a} represents an optionally protected form of Ar² and R²³ and R²⁴ are each independently either hydrogen or a protecting group, provided that the compound of formula (II) contains at least one protecting group.

Protected forms Ar^{1a} of the preferred groups Ar¹ may be selected from: 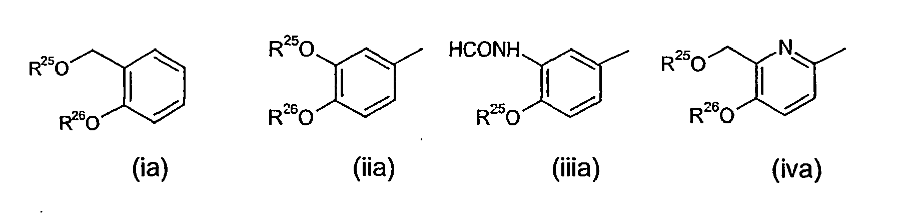 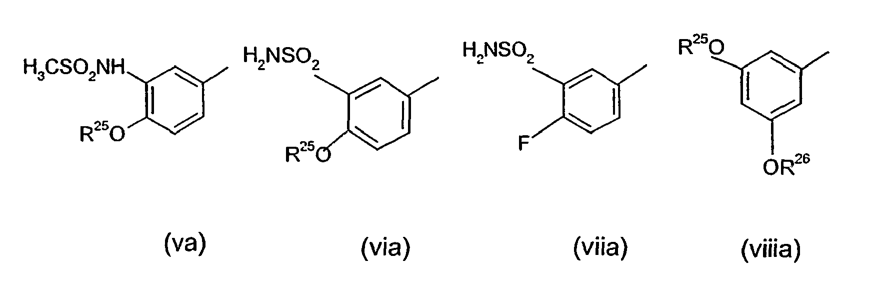 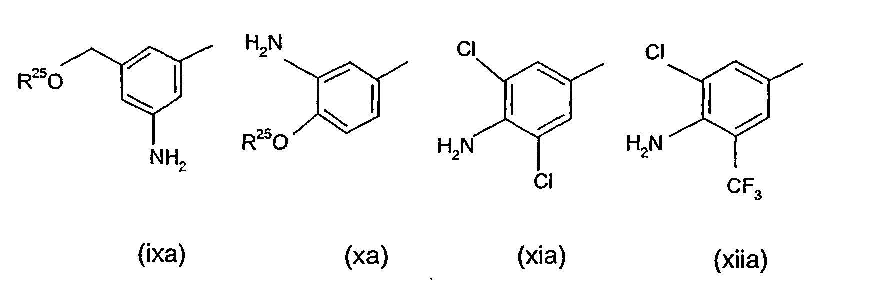 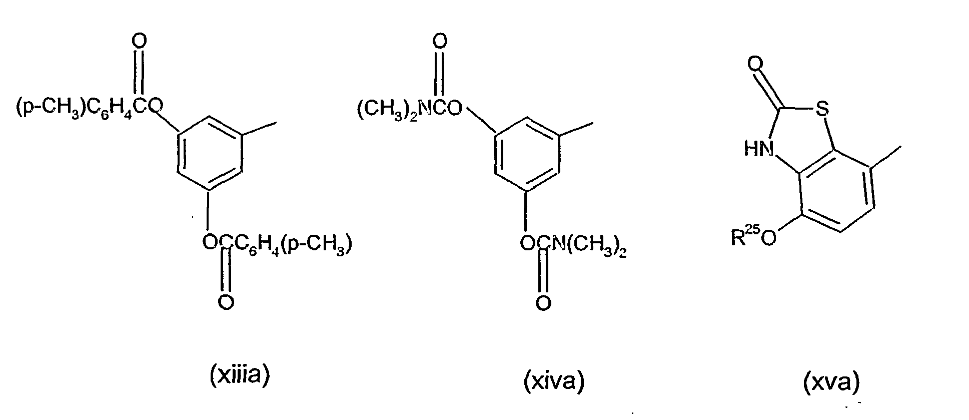 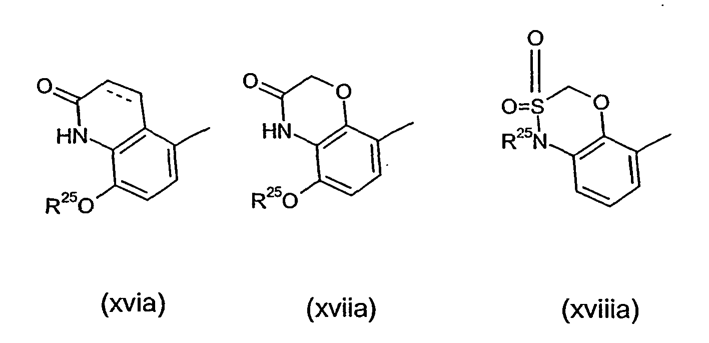 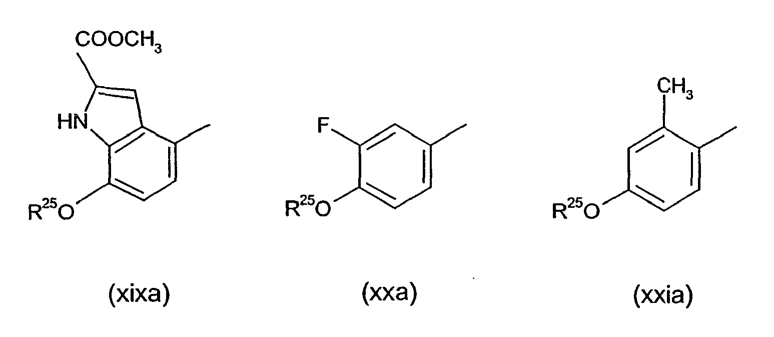 wherein R²⁵ and R²⁶ are each independently either hydrogen or a protecting group provided that at least one of R²⁵ and R²⁶ is a protecting group. It will be appreciated that when Ar¹ represents a group (vii), (xi), (xii), (xiii) or (xiv), no protection of Ar¹ is required.

Suitable protecting groups may be any conventional protecting group such as those described in "Protective Groups in Organic Synthesis" by Theodora W Greene and Peter G M Wuts, 3rd edition (John Wiley & Sons, 1999). Examples of suitable hydroxyl protecting groups represented by R²⁵ and R²⁶ are esters such as acetate ester, aralkyl groups such as benzyl, diphenylmethyl, or triphenylmethyl, and tetrahydropyranyl. Examples of suitable amino protecting groups represented by R²³ include benzyl, α-methylbenzyl, diphenylmethyl, triphenylmethyl, benzyloxycarbonyl, tert-butoxycarbonyl, and acyl groups such as trichloroacetyl or trifluoroacetyl.

As will be appreciated by the person skilled in the art, use of such protecting groups may include orthogonal protection of groups in the compounds of formula (II) to facilitate the selective removal of one group in the presence of another, thus enabling selective functionalisation of a single amino or hydroxyl function. For example, the -CH(OH) group may be orthogonally protected as - CH(OR²⁴) using, for example, a trialkylsilyl group such as triethylsilyl. A person skilled in the art will also appreciate other orthogonal protection strategies, available by conventional means as described in Theodora W Greene and Peter G M Wuts (see above).

The deprotection to yield a compound of formula (I), may be effected using conventional techniques. Thus, for example, when R²⁵, R²⁶, and/or R²³ is an aralkyl group, this may be cleaved by hydrogenolysis in the presence of a metal catalyst (e.g. palladium on charcoal).

When R²⁵ and/or R²⁶ is tetrahydropyranyl this may be cleaved by hydrolysis under acidic conditions. Acyl groups represented by R²³ may be removed by hydrolysis, for example with a base such as sodium hydroxide, or a group such as trichloroethoxycarbonyl may be removed by reduction with, for example, zinc and acetic acid. Other deprotection methods may be found in Theodora W Greene and Peter G M Wuts (see above). In a particular embodiment of process (a), R²⁵ and R²⁶ may together represent a protecting group as in the compound of formula (III): 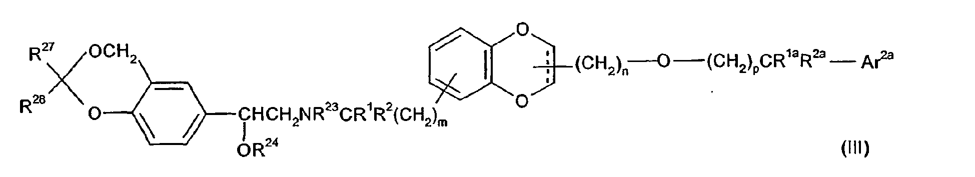 or a salt or solvate thereof, wherein R¹, R², R^{1a}, R^{2a}, R²³, R²⁴, m, n and p are as defined for the compound of formula (II), and R²⁷ and R²⁸ are independently selected from hydrogen, C₁₋₆alkyl, C₃₋₇cycloalkyl or aryl or R²⁷ and R²⁸ together form a C₃₋₇cycloalkyl ring. In a preferred aspect, both R²⁷ and R²⁸ are methyl.

A compound of formula (III) may be converted to a compound of formula (I), by hydrolysis with dilute aqueous acid, for example acetic acid or hydrochloric acid in a suitable solvent or by transketalisation in an alcohol, for example ethanol, in the presence of a catalyst such as an acid (for example, toluenesulphonic acid) or a salt (such as pyridinium tosylate) at normal or elevated temperature.

It will be appreciated that the protecting groups R²⁵, R²⁶, R²³ and R²⁴ (including the cyclised protecting group formed by R²⁵ and R²⁶ as depicted in formula (III) may be removed in a single step or sequentially. The precise order in which protecting groups are removed will in part depend upon the nature of said groups and will be readily apparent to the skilled worker. Preferably, when R²⁵ and R²⁶ together form a protecting group as in formula (III) this protecting group is removed together with any protecting group on the CH(OH) moiety, followed by removal of R²³.

A compound of formula (II) or formula (III) wherein R²³ is hydrogen may be prepared from a corresponding compound of formula (IV): 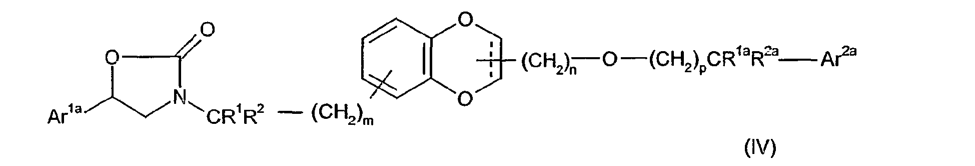 or a salt or solvate thereof, wherein ----, Ar^{1a}, Ar^{2a}, R¹, R², R^{1a}, R^{2a}, m, n and p are as defined for the compound of formula (II) or (III).

The conversion of a compound of formula (IV) to a compound of formula (II) or (III) may be effected by treatment with a base, for example a non-aqueous base, such as potassium trimethylsilanolate, or an aqueous base such as aqueous sodium hydroxide, in a suitable solvent such as tetrahydrofuran.

A compound of formula (IV) may be prepared from a corresponding compound of formula (V): 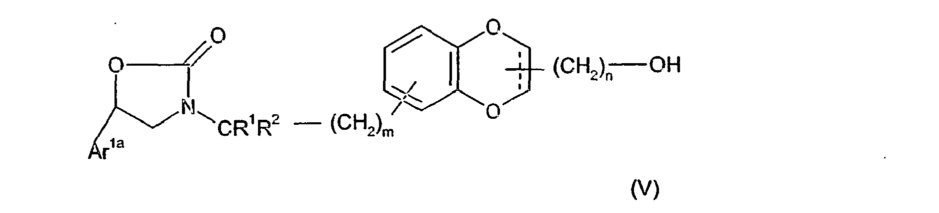 or a salt or solvate thereof, wherein Ar^{1a}, R¹, R², m, and n are as defined for compounds of formula (II). by reaction with a compound of formula (VI):

L(CH₂)ₚCR^{1a}R^{2a}Ar^{2a} (VI)

wherein R^{1a} , R^{2a}, p and Ar^{2a} are as defined for compounds (IV) and L is a leaving group.

The reaction of a compound of formula (V) with a compound of formula (VI) is conveniently effected in the presence of a base such as sodium hydride in a suitable solvent for example dimethylformamide.

Compounds of formula (VI) are commercially available or may be prepared by methods well known to the person skilled in the art.

A compound of formula (V) may be prepared from a corresponding compound of formula (VII): 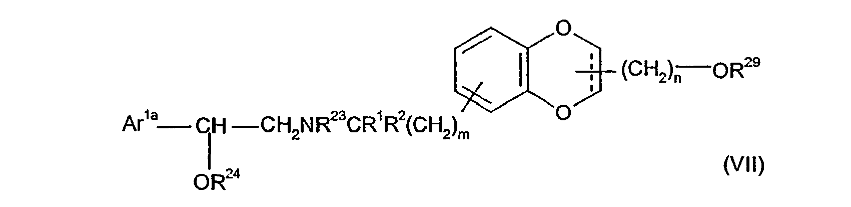 wherein ----, Ar^{1a}, R¹, R², R²³, R²⁴, m and n are as hereinbefore defined for compounds of formula (II), and R²⁹ is a protecting group, for example an ester-forming group such as pivaloyl.

A compound of formula (VII) may be converted into a compound of formula (V) by reaction with a reagant such as 1,1-carbonyldiimidazole, in a solvent such as tetrahydrofuran. The protecting group R²⁹ may be removed by any suitable method known in the art, for example by reaction with a trialkylsilanolate eg. potassium trimethylsilanolate, in a solvent such as tetrahydrofuran.

A compound of formula (VII) may be prepared by reacting a compound of formula (VIII): 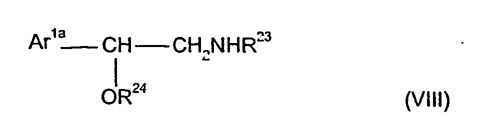 wherein Ar^{1a}, R²³ and R²⁴ are as defined for formula (II);
with a compound of formula (IX): - 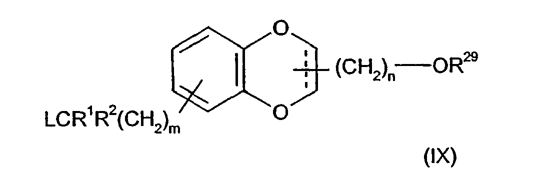 wherein ----, R¹, R², m and n are as defined for formula (II), L is a leaving group for example a halo group, (typically bromo or iodo) or a sulfonate such as an alkyl sulfonate (typically methanesulfonate) an aryl sulfonate (typically toluenesulfonate) or a haloalkylsulfonate (typically trifluoromethanesulfonate), and R²⁹ is a protecting group, eg. pivaloyl.

The reaction of a compound for formula (VIII) with a compound of formula (IX) may conveniently be effected in a solvent such as N,N-dimethylformamide.

Compounds of formula (VIII) are known in the art (for example EPA 0947498) or may be readily prepared by a person skilled in the art using known methods, for example as described in WO 02/066422.

Further details concerning preparation of compounds (VIII) wherein Ar¹ is a group (v) can be found in DE3524990; concerning the preparation of compounds (VIII) wherein Ar¹ is a group (ii), (viii), and (xvi) in EP-A-162576; concerning the preparation of compounds (VIII)
wherein Ar¹ is a group (iv) in EP-A-220054; concerning the preparation of compounds (VIII) wherein Ar¹ is a group (xi) in GB2165542 and concerning the preparation of compounds (VIII) wherein Ar¹ is a group (c) in GB2230523.

A compound of formula (IX) may be prepared from a corresponding compound of formula (X) : 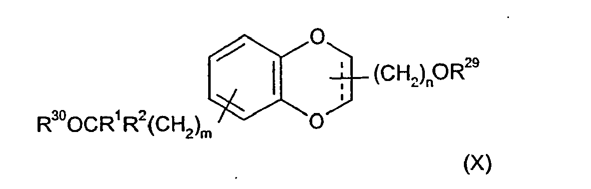 wherein R³⁰ is hydrogen or a protecting group, such as a trialkylsilyl group eg. t-butyl dimethylsilyl, and ----, R¹, R², m, and n are as defined for formula (IX), and R²⁹ is a protecting group eg. benzyl.

Thus for example a compound of formula (X) wherein R³⁰ represents hydrogen may be reacted with a sulfonyl halide, eg. methanesulfonyl chloride or p-toluenesulfonyl chloride to give a compound of formula (IX) wherein L is a sulfonate. Said sulfonate may if desired be converted into a corresponding halide by reaction with a tetraalkylammonium halide, such as tetrabutylammonium iodide, in a suitable solvent, for example acetonitrile. Where R³⁰ represents a protecting group this may be removed prior to formation of the group L, by coventional methods. Thus for example when R³⁰ represents a trialkylsilyl group this may be removed eg. by reaction with tetrabutylammonium fluoride. The protecting group R²⁹ may if desired by exchanged for a different protecting group to facilitate the subsequent reaction steps. Thus, where R²⁹ in the compound of formula (IX) is a benzyl group, this may if desired be removed and replaced with eg. a pivaloyl group. Selection of suitable protecting groups will be evident to those skilled in the art.

A compound of formula (X) may be prepared from a compound of formula (XI): 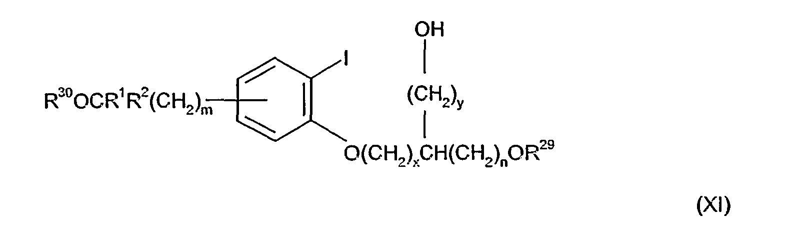 wherein R¹, R², R³⁰, m and n are as defined for formula (X), R²⁹ is a protecting group such as benzyl, and x and y are each zero or 1 such that the sum of x+y = 1.

Cyclisation of a compound (XI) may be effected by reaction with a base such as caesium carbonate or potassium carbonate in the presence of cuprous iodide and 1,10-phenanthroline in a solvent such as toluene. If necessary or desired, the product of the cyclisation reaction may be further reacted to introduce the group R³⁰.

If desired the protecting group R²⁹ may be replaced with a different protecting group, using standard deprotection and protection techniques, eg. to facilitate further reaction of a compound (X). Thus for example, in compounds of formula (XI) R²⁹ is conveniently benzyl, but in subsequent stages an acyl group such as pivaloyl is preferred.

A compound of formula (XI) wherein x = 1 and y = zero may be prepared by reacting a compound of formula (XII): 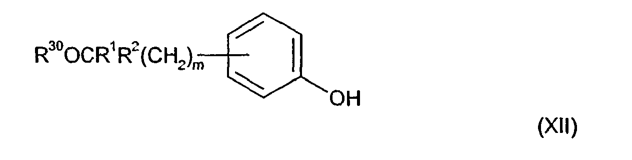 wherein R¹, R², R³⁰ and m are as defined for formula (XI) with a solution of iodine, in the presence of a base such as sodium hydroxide and in a solvent such as ethanol, to form the iodo-substituted compound. This may then be reacted with a compound of formula (XIII): 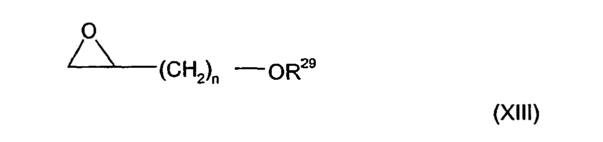 under basic conditions eg. using caesium carbonate in a solvent such as N,N-dimethylformamide.

It will be appreciated that the configuration of the moiety: 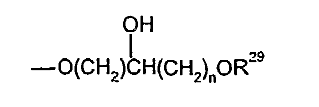 in formula (XI) and ultimately of the compound of formula (I) in respect of the asymmetric carbon atom in the benzodioxan ring will depend upon the configuration of the compound of formula (XIII) employed. In one embodiment, the compound of formula (XIII) may be for example (S)-(+)-benzyl glycidyl ether.

A compound of formula (XI) wherein x = zero and y = 1 may be prepared from a compound of formula (XIV): 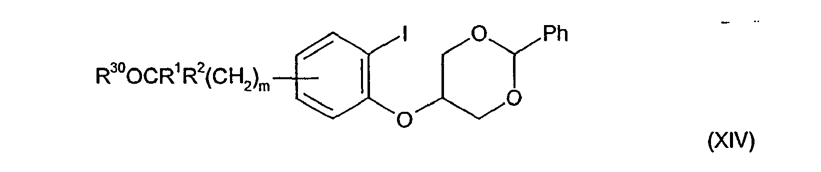 wherein R¹, R², R³⁰ and n are as defined hereinbefore for compounds (XI);
by cleavage of the 1,3-dioxane ring, using eg. a hydride reducing agent acting as a Lewis acid such as diisobutylaluminium hydride, or an organic acid such a trifluoroacetic acid in the presence of a reducing agent such as triethylsilane.

A compound of formula (XIV) may be prepared by reacting a compound of formula (XII) as defined hereinabove, with a solution of iodine in the presence of a base to form the iodo-substituted compound, as described hereinabove, followed by reaction with a compound of formula (XV): 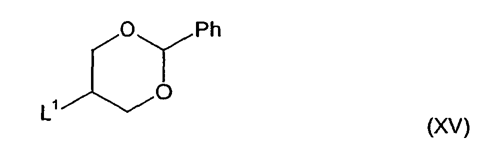 wherein L¹ is a leaving group eg. a sulponate such as methane sulphonate.

Compounds of formula (XV) may be prepared by standard methods from commerically available precursors.

Compounds of formula (XII) and (XIII) are commercially available or may be prepared by standard methods.

A compound of formula (IV) may also be prepared by reaction of a compound of formula (XVI): 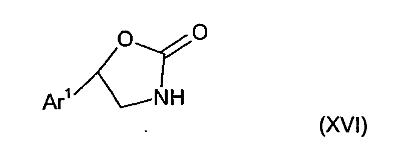 or a salt or solvate thereof, wherein Ar¹ is as defined for the compound of formula (II) with a compound of formula (XVII): 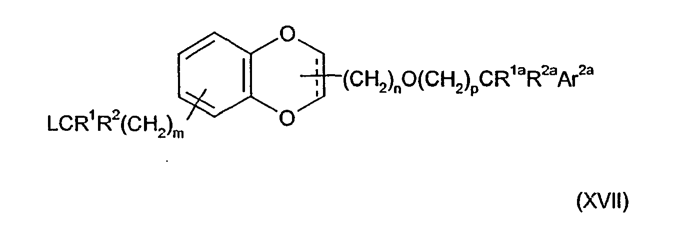 wherein ----, R¹, R², R^{1a}, R^{2a}, m, n and p are as defined for the compound of formula (II) and L is a leaving group as defined for formula (IX)

The coupling of a compound of formula (XIV) with a compound of formula (XVII) may be effected in the presence of a base, such as a metal hydride, for example sodium hydride, or an inorganic base such as cesium carbonate, in an aprotic solvent, for example N,N-dimethylformamide or tetrahydrofuran.

Compounds of formula (XVI) may be prepared as described in WO 02/066422.

A compound of formula (XVII) may be prepared by reacting a compound of formula (IX) as hereinbefore defined with a compound of formula (VI) as hereinbefore defined, for example using conditions described hereinabove for the reaction of a compound (V) with a compound (VI).

In a further process (b) a compound of formula (I), (II) or (III) may be prepared by alkylation of an amine of formula 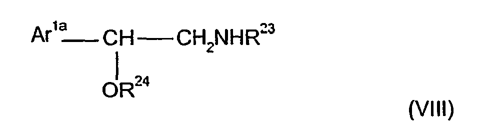 wherein Ar^{1a}, R²³ and R²⁴ are as defined for formula (II) with a compound of formula (XVII): 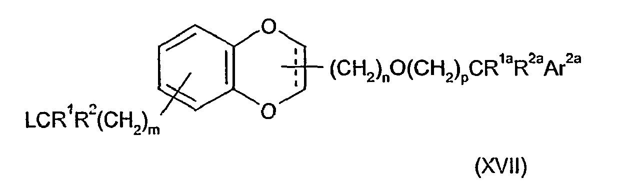 as defined above
followed by removal of any protecting groups present by conventional methods as derscribed above for the deprotection of compounds of formula (II) and (III).

The reaction of compounds (VIII) and (XVII) is optionally effected in the presence of an organic base such as trialkylamine and in a suitable solvent, for example N,N-dimethyl formamide or acetonitrile.

It will be appreciated that in either of the routes (a) or (b) described above, the precise order of the synthetic steps by which the various groups and moieties are introduced into the molecule may be varied. It will be within the skill of the practitioner in the art to ensure that groups or moieties introduced at one stage of the process will not be affected by subsequent transformations and reactions, and to select the order of synthetic steps accordingly.

The enantiomeric compounds of the invention may be obtained (i) by separation of the components of the corresponding racemic mixture, for example, by means of a chiral chromatography column, enzymic resolution methods, or preparing and separating suitable diastereoisomers, or (ii) by direct synthesis from the appropriate chiral intermediates by the methods described above.

Optional conversions of a compound of formula (I) to a corresponding salt may conveniently be effected by reaction with the appropriate acid or base. Optional conversion of a compound of formula (I) to a corresponding solvate may be effected by methods known to those skilled in the art.

According to a further aspect, the present invention provides novel intermediates for the preparation of compounds of formula (I) for example:
compounds of formula (II), (III) and (IV) as defined above, or an optical isomer, a salt, or a protected derivative thereof

For a better understanding of the invention, the following Examples are given by way of illustration.

### SYNTHETIC EXAMPLES

Throughout the examples, the following abbreviations are used:
LCMS: Liquid Chromatography Mass Spectrometry
MS : mass spectrum
TSP+ve : thermospray mass spectrum positive mode
HPLC: high pressure liquid chromatography
RT : retention time
THF : tetrahydofuran
DCM : dichloromethane
DMF: N,N-dimethylformamide
EtOAc : ethyl acetate
Et₂O : diethyl ether
EtOH : ethanol
MeOH : methanol
MeCN : acetonitrile
AcOH : glacial acetic acid
PE: petroleum ether
bp : boiling point
ca : circa
h : hour(s)
min : minute(s)
All temperatures are given in degrees centigrade.
Silica gel refers to Merck silica gel 60 Art number 7734.
Flash silica gel refers to Merck silica gel 60 Art number 9385.
Biotage refers to prepacked silica gel cartridges containing KP-Sil run on flash 12i chromatography module.
SPE Bond Elut are prepacked cartridges used in parallel purifications, normally under vacuum. These are commercially available from Varian.

LCMS was conducted on a Supelcosil LCABZ+PLUS column (3.3 cm x 4.6 mm ID) eluting with 0.1 % HCO₂H and 0.01 M ammonium acetate in water (solvent A), and 0.05% HCO₂H 5% water in acetonitrile (solvent B), using the following elution gradient 0-0.7 min O%B, 0.7-4.2 min 100%B, 4.2-5.3 min 0%B, 5.3-5.5 min 0%B at a flow rate of 3 ml/min. The mass spectra were recorded on a Fisons VG Platform spectrometer using electrospray positive and negative mode (ES+ve and ES-ve).

HPLC was conducted on a LCABZ+PLUS column (3.3 cm x 4.6 mm ID) eluting with 0.1% formic acid and 0.01 M ammonium acetate in water (solvent A), and 0.05% formic acid 5% water in acetonitrile (solvent B) using the following elution gradient 0-1 min O%B, 1-10 min100%B, 10-13 min 100%B, 13-15 min O%B at a flow rate of 1ml/min

Preparative mass directed HPLC was conducted on a Waters FractionLynx system comprising of a Waters 600 pump with extended pump heads, Waters 2700 autosampler, Waters 996 diode array and Gilson 202 fraction collector on a 10 cm X 2.54 cm ID ABZ+ column, eluting with 0.1% formic acid in water (solvent A) and 0.1% formic acid in acetonitrile (solvent B), using the following elution gradient: 0.0-1.0 min 15%B, 1.0-10.0 min 55%B, 10.0-14.5 min 99%B, 14.5-14.9 min 99%B, 14.9-15.0 min 15%B at a flow rate of 20 ml/min and detecting at 200-320 nm at room temperature. Mass spectra were recorded on Micromass ZMD mass spectrometer using electrospray positive and negative mode, alternate scans. The software used was *MassLynx* 3.5 with *OpenLynx* and *FractionLynx* options.

### Example 1

### 4-((1R)-2-{[2-((3R)-3-{[(2,6-Dichlorobenzyl)oxy]methyl}-2,3-dihydro-1,4-benzodioxin-6-yl)ethyl]amino}-1-hydroxyethyl)-2-(hydroxymethyl)phenol with (2E)-but-2-enedioic acid (1:2)

### i) 4-(2-Hydroxyethyl)-2-iodophenol

A solution of iodine (100g) in EtOH (900ml) was added slowly over 2h to a stirred solution of 4-hydroxyphenethylalcohol (54.8g) in 2M NaOH solution (900ml) at room temperature. The solution was concentrated (ca 50% vol), acidified with 50% HCl and extracted with EtOAc. The extracts were washed with aqueous sodium sulphite (300ml), water and dried (Na₂SO₄ The solvent was evaporated to dryness and the residue was recrystallised from PE - EtOAc (2:1, 700ml) to give required compound (34.7g). The mother liquors were evaporated onto silica and purified by column chromatography on silica (500g) eluting with DCM - isopropanol (95:5). The appropriate fractions were combined with the first batch and re-crystallised from PE - EtOAc (2:1, 500ml) to give *the title compound* (27.6g). LCMS RT = 2.75 min. Concentration of the mother liquors gave a second crop (24.3g).

### ii) (2R)-1-(Benzyloxy)-3-14-(2-hydroxyethyl)-2-iodophenoxy]propan-2-ol

A stirred mixture of 4-(2-hydroxyethyl)-2-iodophenol (36g), (S)-(+)-benzyl glycidyl ether (25g) and caesium carbonate (53g) in DMF (600ml) was heated at 90° for 18h. The mixture was cooled, poured into 2M HCl and extracted into EtOAc. The combined extracts were washed with 2M NaOH, water, dried (Na₂SO₄) and evaporated. The residual solid was triturated with diethyl ether to give *the title compound* (44g). LCMS RT = 3.35 min.

### iii) 2-{(3R)-3-[(Benzyloxy)methyl]-2,3-dihydro-1,4-benzodioxin-6-yl}ethanol

A stirred mixture of (2*R*)-1-(benzyloxy)-3-[4-(2-hydroxyethyl)-2-iodophenoxy]propan-2-ol (44g), caesium carbonate (66g) , cuprous iodide (1.9g) and 1,10-phenanthroline (1.9g) in toluene (400ml) was heated at 110° for 18h. The mixture was cooled, filtered through celite and the filtrate evaporated with silica gel (200g). The residue was purified by column chromatography on silica (600g) eluting with diethyl ether to give *the title compound* (25g). LCMS RT = 3.37 min.

### iv 2-{(3R)-3-[(Benzyloxy)methyl]-2,3-dihydro-1,4-benzodioxin-6-yl}ethyl methanesulfonate

Methanesulphonyl chloride (8.8ml) was added to a stirred, cooled (ice-bath) solution of 2-{(3*R*)-3-[(benzyloxy)methyl]-2,3-dihydro-1,4-benzodioxin-6-yl}ethanol (19g) in pyridine (120ml). The solution was then stirred for 1.5 h at room temperature. The solution was poured into 5M HCl (600ml) and extracted into DCM. The extracts were washed with saturated NaHCO₃, water and dried (Na₂SO₄). The solvent was evaporated and the residual oil was purified by column chromatography on silica eluting with diethyl ether-cyclohexane (4:1). The appropriate fractions were evaporated to give *the title compound* (19g). LCMS RT = 3.44 min.

### v) 2-[(3R)-3-(Hydroxymethyl)-2,3-dihydro-1,4-benzodioxin-6-yl]ethyl methanesulfonate

A solution of 2-{(3*R*)-3-[(benzyloxy)methyl]-2,3-dihydro-1,4-benzodioxin-6-yl}ethyl methanesulfonate (19g) in EtOH (400ml) and EtOAc (400ml) was hydrogenated over 10% palladium on carbon (3g) and 20% palladium hydroxide on carbon (60% paste in water, 7g) for 24 h at room temperature. The mixture was filtered through celite and the filtrate was evaporated to dryness to give the *title compound* (15g). LCMS RT = 2.42 min.

### vi) ((2S)-7-{2-[(Methylsulfonyl)oxylethyl}-2,3-dihydro-1,4-benzodioxin-2-yl)methyl pivalate

Pivaloyl chloride (15.6ml) was added to a stirred, and cooled (ice-bath) solution of 2-[(3R)-3-(hydroxymethyl)-2,3-dihydro-1,4-benzodioxin-6-yl]ethyl methanesulfonate (15g) in pyridine (120ml). The solution was stirred at room temperature for 1h, poured into 5M HCI (500ml) and extracted into DCM. The extracts were washed with saturated NaHCO₃, water, dried (Na₂SO₄) and evaporated to give the *title compound* (19g). LCMS RT = 3.3 min.

### vii) [(2S)-7-(2-lodoethyl)-2,3-dihydro-1,4-benzodioxin-2-yl]methyl pivalate

A solution of ((2S)-7-{2-[(methylsulfonyl)oxy]ethyl}-2,3-dihydro-1,4-benzodioxin-2-yl)methyl pivalate (13g) and tetrabutylammonium iodide (52g) in MeCN (200ml) was heated at 70° for 4h. The solvent was concentrated to near dryness, diluted with water (800ml) and extracted with diethyl ether (500ml). The organic extract was washed with water, dried (Na₂SO₄) and evaporated to give *the title compound* (13.5g). LCMS RT = 3.88 min.

### viii) [(2S)-7-(2-{[(2R)-2-(2,2-Dimethyl-4H-1,3-benzodioxin-6-yl)-2-hydroxyethyl]amino}ethyl)-2,3-dihydro-1,4-benzodioxin-2-yl]methyl pivalate

A mixture of [(2*S*)-7-(2-iodoethyl)-2,3-dihydro-1,4-benzodioxin-2-yl]methyl pivalate (4.4g) and (1*R*)-2-amino-1-(2,2-dimethyl-4*H*-1,3-benzodioxin-6-yl)ethanol (WO 0266422 A1) (4.8g) in DMF (15ml)was stirred for 20 h at room temperature. The mixture was poured into water and extracted into diethyl ether. The combined extracts were washed with water, dried (Na₂SO₄) and evaporated to dryness. The residual oil was purified on a biotage™ cartridge of silica (90g) eluting with DCM-EtOH-0.88 ammonia (200:8:1) to give the *title compound* (1.7g). LCMS RT = 2.66 min.

### ix) ((2S)-7-{2-[(5R)-5-(2,2-Dimethyl-4H-1,3-benzodioxin-6-yl)-2-oxo-13-oxazolidin-3-yl]ethyl}-2,3-dihydro-1,4-benzodioxin-2-yl)methyl pivalate

1,1-Carbonyl diimidazole (1.6g) was added to a stirred solution of [(2S)-7-(2-{[(2*R*)-2-(2,2-dimethyl-4*H*-1,3-benzodioxin-6-yl)-2-hydroxyethyl]amino}ethyl)-2,3-dihydro-1,4-benzodioxin-2-yl]methyl pivalate (3.3g) in dry THF (50ml). After 18h the solution was evaporated to dryness, diluted with water (100ml) and extracted into diethyl ether. The extracts were dried (Na₂SO₄), evaporated and the residual oil was purified on a biotage™ cartridge of silica (90g) using diethyl ether as eluent. The fractions were evaporated to give the *title compound* (2.9g). LCMS RT = 3.64 min.

### x) (5R)-5-(2.2-Dimethyl-4H-1.3-benzodioxin-6-yl)-3-{2-[(3R)-3-(hydroxymethyl)-2,3-dihydro-1,4-benzodioxin-6-yllethyl}-1,3-oxazolidin-2-one

A solution of ((2S)-7-{2-[(5R)-5-(2,2-dimethyl-4H-1,3-benzodioxin-6-yl)-2-oxo-1,3-oxazolidin-3-yl]ethyl}-2,3-dihydro-1,4-benzodioxin-2-yl)methyl pivalate (2.9g) and potassium trimethylsilanolate (2.8g) in THF(50ml) was stirred for 2 h at room temperature. The solution was poured into water and extracted into DCM. The extracts were dried (Na₂SO₄) and evaporated to give the *title compound* (1.7g). LCMS RT = 2.91 min.

### xi) (5R)-3-[2-((3R)-3{[(2.6-Dichlorobenzyl)oxylmethyl)-2.3-dihydro-1,4-benzodioxin-6-yl)ethyll-5-(2,2-dimethyl-4H-1,3-benzodioxin-6-yl)-1,3-oxazolidin-2-one

Sodium hydride (60% dispersion in oil, 0.02g) was added to a solution of (5R)-5-(2,2-dimethyl-4H-1,3-benzodioxin-6-yl)-3-{2-[(3R)-3-(hydroxymethyl)-2,3-dihydro-1,4-benzodioxin-5-yl]ethyl}-1,3-oxazolidin-2-one (0.121g) in DMF (4ml) at room temperature. After 15 min 2,6-dichlorobenzyl bromide (0.1g) was added and the mixture was stirred for 1h. The mixture was then poured into aqueous ammonium chloride solution and extracted into EtOAc. The extracts were washed with water, dried (Na₂SO₄) and evaporated. The residual oil was purified on a biotage™ cartridge (8g) eluting with diethyl ether. The appropriate fractions were evaporated to give *the title compound* (0.108g). LCMS RT = 3.87 min.

### xii) (1R)-2-{[2-((3R)-3-{[(2,6-Dichlorobenzyl)oxylmethyl-2,3-dihydro-1,4-benzodioxin-6-yl)ethyl]amino}-1-(2,2-dimethyl-4H-1,3-benzodioxin-6-yl)ethanol

A stirred solution of (5*R*)-3-[2-((3*R*)-3-{[(2,6-dichlorobenzyl)oxy]methyl}-2,3-dihydro-1,4-benzodioxin-6-yl)ethyl]-5-(2,2-dimethyl-4H-1 ,3-benzodioxin-6-yl)-1 ,3-oxazolidin-2-one (0.108g) and potassium trimethylsilanolate (0.248g) in THF (5ml) was heated at 80° for 1 h. The mixture was poured into phosphate buffer solution (pH 5) and extracted into EtOAc. The extracts were washed with water, dried (Na₂SO₄) and evaporated. The residual oil was purified on a biotage™ cartridge of silica (4g) eluting with DCM-EtOH-0.88 ammonia. The appropriate fractions were evaporated to give *the title compound* (0.058g) LCMS RT = 3.00 min.

### xiii 4-((1R)-2-{[2-((3R)-3-{[(2.6-Dichlorobenzyl)oxy]methyl)-2,3-dihydro-1,4-benzodioxin-6-yl)ethyl]amino}-1-hydroxyethyl)-2-(hydroxymethyl)phenol acetate

A stirred solution of (1*R*)-2-{[2-((3*R*)-3-{[(2,6-dichlorobenzyl)oxy]methyl}-2,3-dihydro-1,4-benzodioxin-6-yl)ethyl]amino}-1-(2,2-dimethyl-4H-1,3-benzodioxin-6-yl)ethanol (0.05g) in glacial AcOH (5ml) and water (0.3ml) was heated at 80° for 1h. The solvent was evaporated to give the *title compound.* ¹H NMR CD₃OD = 7.41 (d,8Hz,2H), 7.34 - 7.30(m,2H), 7.15(dd,8,2Hz, 1H),6.81-6.77(m,3H), 6.72(dd,8,2 Hz, 1H), 4.85(m, obscured by H₂O) 4.65(s,2H), 4.31(m,1H), 4.28(m,1H), 4.03(dd,11,7Hz,1H), 3.81(dd,10.5,5Hz,1H), 3.77(dd10.5,5Hz, 1H), 3.21(t,8Hz,2H), 3.13(m,1H), 3.11(m,1H), 2.89(m,2H)

### xiv) 4-((1R)-2-{[2-((3R)-3-{[(2 6-Dichlorobenzyl)oxylmethyl)-2.3-dihydro-1,4-benzodioxin-6-yl)ethyl]amino}-1-hydroxyethyl)-2-(hydroxymethyl)phenol with (2E)-but-2-enedioic acid (1:2)

4-((1*R*)-2-{[2-((3R)-3-{[(2,6-dichlorobenzyl)oxy]methyl}-2,3-dihydro-1,4-benzodioxin-6-yl)ethyl]amino)-1-hydroxyethyl)-2-(hydroxymethyl)phenol acetate was purified on a biotage™ cartridge of silica (4g) eluting with DCM-EtOH-0.88 ammonia (25:8:1). The appropriate fractions were evaporated and the residual oil was dissolved in MeOH (5ml) and treated with 0.5 equivalent of fumaric acid. The solvent was removed to give *the title compound* (0.25g). LCMS RT = 2.66 min; ES+ve 537 (MH⁺).

### Example 2

### 4-{(1R)-2-[(2-{(3R)-3-[(Benzyloxy)methyl]-2,3-dihydro-1,4-benzodioxin-6-ul}ethyl)amino]-1-hydroxyethyl}-2-(hydroxymethyl)phenol acetate

### i) (1R)-2-[(2-{(3R)-3-[(Benzyloxy)methyl]-2,3-dihydro-1,4-benzodioxin-6-yl}ethyl)amino]-1-(2,2-dimethyl-4H-1,3-benzodioxin-6-yl)ethanol

Prepared using methods similar to those described in Example 1 viii) using (1*R*)-2-amino-1-(2,2-dimethyl-4H-1,3-benzodioxin-6-yl)ethanol (WO 0266422 A1) and benzyl alcohol. LCMS RT = 2.72 min.

### ii) 4-{(1R)-2-[(2-{(3R)-3-[(Benzyloxy)methyl]-2,3-dihydro-1,4-benzodioxin-6-yl)ethyl)amino]-1-hydroxyethyl)-2-(hydroxymethyl)phenol acetate

Prepared using methods similar to those described in Example 1 xiii) LCMS RT = 2.60 min; ES+ve 466 (MH)⁺

### Example 3

### 4-{(1R)-2-[(2-{(3S)-3-[(Benzyloxy)methyl]-2,3-dihydro-1,4-benzodioxin-6-yl)ethyl)amino]-1-hydroxyethyl}-2-(hydroxymethyl)phenol acetate

### i) 4-(2-{[tert-Butyl(dimethyl)silyl]oxy}ethyl)-2-iodophenol

*tert*-Butyldimethylsilyl chloride (1.21g) was added to a stirred solution of 4-(2-hydroxyethyl)-2-iodophenol (2.0g) and imidazole (0.54g) in DMF (15ml). After 1 h the solution was poured into water and extracted into diethyl ether. The extracts were dried (Na₂SO₄ evaporated and the residual oil was purified on a biotage™ cartridge of silica (40g) using hexane-diethyl ether (7:3) as the eluent. The fractions were evaporated to give the *title compound* (2.2g). LCMS RT = 4.07 min.

### ii) (2S)-1-(Benzyloxy)-3-[4-(2-{[tert-butyl(dimethyl)silyl]oxy)ethyl)-2-iodophenoxy]propan-2-ol

A stirred mixture of 4-(2-{[*tert*-butyl(dimethyl)silyl]oxy)ethyl)-2-iodophenol (0.5g), (R)-(-)-benzyl glycidyl ether (0.26g) and potassium carbonate (0.219g) in DMF (1ml) was heated at 90° for 1 h. The mixture was cooled, diluted with DCM (20ml) and filtered through celite. The filtrate was evaporated and the residual oil was purified on a biotage™ cartridge of silica (8g) using hexane-diethyl ether (4:1) as eluent. The fractions were evaporated to give the *title compound* as a clear oil (0.306g). LCMS RT = 4.31 min.

### iii) (2-{(3S)-3-[(Benzyloxy)methyl]-2,3-dihydro-1,4-benzodioxin-6-yl}ethoxy)(tert-butyl)dimethylsilane

Prepared using methods similar to those described in Example 1 iii) LCMS RT = 4.31 min.

### iv 2-{(3S)-3-[(Benzyloxy)methyl]-2,3-dihydro-1,4-benzodioxin-6-yl)ethanol

A solution of (2-{(3S)-3-[(benzyloxy)methyl]-2,3-dihydro-1,4-benzodioxin-6-yl}ethoxy)(tert butyl)dimethylsilane (0.15g) and tetrabutylammonium fluoride on silica (1.6g) in THF (10ml) was stirred for 2h at room temperature. The mixture was filtered through celite and the filtrate evaporated to dryness. The residual oil was purified on a biotage™ cartridge of silica (4g) eluting with hexane-diethyl ether (3:2). The fractions were evaporated to give the *title compound* (0.07g). LCMS RT = 3.11 min.

### v) 2-{(3S)-3-[(Benzyloxy)methyl]-2,3-dihydro-1,4-benzodioxin-6-yl}ethyl 4-methylbenzenesulfonate

Prepared using methods similar to those described in Example 1 iv) using 4-methylbenzenesulfyl chloride. LCMS RT = 3.87min.

### vi) (1R)-2-[(2-{(3S)-3[(Benzyloxy)methyl]-2,3-dihydro-1,4-benzodioxin-6-yl}ethyl)amino]-1-(2,2-dimethyl-4H-1,3-benzodioxin-6-yl)ethanol

Prepared using methods similar to those described in Example 1 viii). LCMS RT = 2.86 min.

### vii) 4-{(1R)-2-[(2-{(3S)-3-[(Benzyloxy)methyl]-2,3-dihydro-1,4-benzodioxin-6-yl}ethyl)amino]-1-hydroxyethyl}-2-(hydroxymethyl)phenol acetate

Prepared using methods similar to those described in Example 1 xiii). LCMS RT = 2.50 min; ES+ve 466 (MH⁺).

### Example 4

### 2-(Hydroxymethyl)-4-{(1R)-1-hydroxy-2-[(2-{(3R)-3-[(pyridin-3-ylmethoxy)methyl]-2,3-dihydro-1,4-benzodioxin-6-yl}ethyl)amino]ethyl}phenol acetate

### i) (5R)-5-(2,2-Dimethyl-4H-1,3-benzodioxin-6-yl)-3-(2-{(3R)-3-[(pyridin-3-ylmethoxy)methyl]-2,3-dihydro-1,4-benzodioxin-8-yl}ethyl)-1,3-oxazolidin-2-one

Prepared using methods similar to those described in Example 1 xi). LCMS RT = 3.00 min.

### ii) (1R)-1-(2,2-Dimethyl-4H-1,3-benzodioxin-6-yl)-2-[(2-{(3R)-3-[(pyridin-3-ylmethoxy)methyl]-2,3-dihydro-1,4-benzodioxin-6-yl}ethyl)amino]ethanol

Prepared using methods similar to those described in Exampe 1 xii) LCMS RT = 2.30 min.

### iii) 2-(Hydroxymethyl)-4-{(1R)-1-hydroxy-2-[(2-[(23R)-3-[(pyridin-3-ylmethoxy)methyl]-2 3-dihydro-1,4-benzodioxin-6-yl)ethyl)amino]ethyl}phenol acetate

Prepared using methods similar to those described in Example 1 xiii) LCMS RT = 1.93 min ES+ve 467 (MH)⁺.

### Example 5 5

### 4-((1R)-2-{2-((3R)-3-{[(6-Chloropyridin-3-yl)methoxylmethyl}-2,3-dihydro-1,4-benzodioxin-6-yl)ethyllamino}-1-hydroxyethyl)-2-(hydroxymethyl)phenol acetate

### i) (5R)-3-[2-((3R)-3-{[(6-Chloropyridin-3-yl)methoxy]methyl}-2,3-dihydro-1,4-benzodioxin-6-yl)ethyl]-5-(2,2-dimethyl-4H-1,3-benzodioxin-6-yl)-1,3-oxazolidin-2-one

Prepared using methods similar to those described in Example 1 xi) LCMS RT = 3.52 min.

### ii) (1R)-2-{[2-((3R)-3-{[(6-Chloropyridin-3-yl)methoxylmethyl}-2-3-dihydro-1,4-benzodioxin-6-yl)ethyl]amino}-1-(2,2-dimethyl-4H-1,3-benzodioxin-6-yl)ethanol

Prepared using methods similar to those described in Example 1 xii) LCMS RT = 2.70 min.

### iii) 4-((1R)-2-{[2-((3R)-3-{[(6-Chloropyridin-3-yl)methoxylmethyl}-2,3-dihydro-1,4-benzodioxin-6-yl)ethyl]amino}-1-hydroxyethyl)-2-(hydroxymethyl)phenol acetate

Prepared using methods similar to those described in Example 1 xiii) LCMS RT = 2.36 min ES+ve 500 (MH)⁺.

### Example 6

### 4-((1 R)-2-{[2-((3R)-3-{[(2,6-Dichloropyridin-3-yl)methoxylmethyl)-2,3-dihydro-1,4-benzodioxin-6-yl)ethyllamino)-1-hydroxyethyl)-2-(hydroxymethyl)phenol acetate

### i) 5R)-3-[2-((3R)-3-{[(2,6-Dichloropyridin-3-yl)methoxylmethyl)-2,3-dihydro-1,4-benzodioxin-6-yl)ethyl]-5-(2,2-dimethyl-4H-1,3-benzodioxin-6-yl)-1,3-oxazolidin-2-one

Sodium hydride (60%) dispersion in oil, 0.04g) was added to a stirred solution of (5R)-5-(2,2-dimethyl-4*H*-1,3-benzodioxin-6-yl)-3-{2-[(3*R*)-3-(hydroxymethyl)-2,3-dihydro-1,4-benzodioxin-6-yl]ethyl}-1,3-oxazolidin-2-one (Example 1x) (0.15g) in DMF (3ml) at room temperature. After 10 min 2,6-dichloro-3-(chloromethyl)pyridine hydrochloride (0.2g) (Helvetica chimica Acta 1976, 59,179-90) was then added and the solution was stirred for 1h. The mixture was poured into a phosphate buffer solution (ph5 30ml) and extracted into DCM (3x25ml). The combined extracts were dried ( Na₂SO₄) and evaporated to dryness. The residual oil was purified on a biotage™ cartridge of silica (8g) eluting with diethyl ether. The appropriate fractions were evaporated to give the *title compound* (0.076g) LCMS RT = 3.71 min.

### ii) (1R)-2-{[2-((3R)-3-{[(2,6-Dichloropyridin-3-yl)methoxy]methyl}-2,3-dihydro-1,4-benzodioxin-6-yl)ethyl]amino}-1-(2,2-dimethyl-4H-1,3-benzodioxin-6-yl)ethanol

Prepared using methods similar to those described in Example 1xii. LCMS RT =2.86 min.

### iii) 4-((1R)-2-{[2-((3R)-3-{[(2,6-Dichloropyridin-3-yl)methoxy]methyl}-2,3-dihydro-1,4-benzodioxin-6-yl)ethylamino}-1-hydroxyethyl)-2-(hydroxymethyl)phenol acetate

Prepared using methods similar to those described in Example 1xiii.
LCMS RT =2.86 min; ES +ve 535 (MH⁺).

### Example 7

### 4-{(1R)-2-[(2-{2-[(Benzyloxy)methyl)]-2,3-dihydro-1,4-benzodioxin-6-y]}ethyl)amino]-1-hydroxyethyl}-2-(hydroxymethyl)phenol acetate

### i) 2-Phenyl-1,3-dioxan-5-yl methanesulfonate

Methanesulphonyl chloride (2.3ml) was added dropwise to a solution of 1,3-*O-*benzylideneglycerol (1.8g)(Fluka) in DCM (50ml) and triethylamine (2.1ml) at ~-5°, under nitrogen. The reaction mixture was stirred at room temperature for 18h. The reaction was quenched with 2N HCI and the organic layer separated. The organic layer was washed with saturated NaHCO₃ brine and dried (MgSO₄). The solvent was evaporated *in vacuo,* and the residue purified by silica SPE bond elut, eluting with EtOAc-cyclohexane mixtures to give the *title compound* (0.47g). LCMS RT=2.84min

### ii) 2-{3-Iodo-4-[(2-phenyl-1,3-dioxan-5-yl)oxy]phenyl}ethanol

A stirred mixture of 4-(2-hydroxyethyl)-2-iodophenol (0.7g) (Example 1i), 2-phenyl-1,3-dioxan-5-yl methanesulfonate (0.47g) and cesium carbonate (0.65g) in DMF (6ml) was heated at 80°, under nitrogen, for 66h. The reaction mixture was cooled to room temperature, treated with 2M sodium carbonate and extracted with EtOAc. The EtOAc extracts were combined, dried (MgSO₄) and evaporated *in vacuo* to give a residue which was purified by silica SPE bond elut, eluting with EtOAc-cyclohexane mixtures to give the *title compound* (0.26g) LCMS RT=3.28min

### iii) tert-Butyl(2-{3-iodo-4-[(2-phenyl-1,3-dioxan-5-yl)oxy]phenyl}ethoxy)dimethylsilane

A solution of 2-{3-iodo-4-[(2-phenyl-1,3-dioxan-5-yl)oxy]phenyl}ethanol (0.26g), imidazole (0.08g), tert-butyldimethylsilyl chloride (0.169g) in DMF (2ml), was stirred at room temperature under nitrogen for 18h. The reaction mixture was quenched with water and extracted with EtOAc. The extracts were combined, dried (MgSO₄) and evaporated *in vacuo.* The residue was purified by silica SPE bond elut, eluting with EtOAc-cyclohexane mixtures to give the *title compound* (0.26g) LCMS RT= 4.33min

### iv) 3-(Benzyloxy)-2-[4-(2-hydroxyethyl)-2-iodophenoxy]propan-1-ol

Diisobutylaluminium hydride in toluene (1M, 0.7ml) was added to a solution of *tert*-butyl(2-{3-iodo-4-[(2-phenyl-1,3-dioxan-5-yl)oxy]phenyl}ethoxy)dimethylsilane (0.25g) in dry DCM (5ml), at 5° under nitrogen. The solution was stirred at room temperature for 1h. The reaction was quenched with saturated NaHCO₃ and extracted with EtOAc. The extracts were combined, washed with brine, dried (MgSO₄), and evaporated *in vacuo.* The residue was purified by silica SPE bond elut, eluting with EtOAc-cyclohexane mixtures to give the *title compound* (0.167g) LCMS RT=3.14min

### v) 2-{2-[(Benzyloxy)methyl]-2,3-dihydro-1,4-benzodioxin-6-yl}ethanol

A mixture of 3-(benzyloxy)-2-[4-(2-hydroxyethyl)-2-iodophenoxy]propan-1-ol (0.167g), cesium carbonate (0.254g), cuprous iodide (8mg) and 1,10-phenanthraline (8mg), in toluene (5ml) was heated at 115°, under nitrogen, for 18h. The reaction was quenched with water and extracted with EtOAc. The combined extracts were dried, (MgSO₄) and evaporated *in vacuo.* The residue was purified on a silica SPE bond elut, eluting with EtOAc- cyclohexane mixtures to give the *title compound* (52mg) LCMS RT=3.23min

### vi) 2-[(Benzyloxy)methyl]-6-(2-bromoethyl)-2,3-dihydro-1,4-benzodioxine

A mixture of 2-{2-[(benzyloxy)methyl]-2,3-dihydro-1,4-benzodioxin-6-yl}ethanol (52mg), PPh₃ (81mg), and carbon tetrabromide (103mg) in DCM (5ml) was stirred at room temperature under nitrogen for 18h. Water was added and the reaction mixture extracted with EtOAc. The combined extracts were dried, (MgSO₄), and evaporated *in vacuo.* The residue was purified by silica SPE bond elut, eluting with EtOAc-cyclohexane mixtures to give the *title compound* (53mg) LCMS RT=3.83min

### vii) (1R)-2-[(2-{2-[(Benzyloxy)methyl]-2,3-dihydro-1.4-benzodioxin-6-yl)ethyl)aminol-1-(2,2-dimethyl-4H-1,3-benzodioxin-6-yl)ethanol

Prepared using methods similar to those described in Example 1 viii) LCMS RT=2.76min

### viii) 4-{(1R)-2-[(2-{2-[(Benzyloxy)methyl]-2,3-dihydro-1,4-benzodioxin-6-yl]ethyl)amino]-1-hydroxyethyl}-2-(hydroxymethyl)phenol acetate

Prepared using methods similar to those described in Example 1 xiii)-LCMS RT = 2.46 min, ES+ve 466 (MH)⁺.

### Example 8

### 4-((1R)-2-{[2-((3R)-3-{[(5-Bromopyridin-3-yl)methoxy]methyl}-2,3-dihydro-1,4-benzodioxin-6-yl)ethyl]amino}-1-hydroxyethyl)-2-(hydroxymethyl)phenol acetate

### i) (5R)-3[2-((3R)-3-{[(5-Bromopyridin-3-yl)methoxy]methyl)-2,3-dihydro-1,4-benzodioxin-6-yl)ethyl]-5-(2,2-dimethyl-4H-1,3-benzodioxin-6-yl)-1,3-oxazolidin-2-one

Prepared using methods similar to those described in Example 1 xi. using 3-bromo-5-chloromethyl pyridine hydrochloride (Nucleosides & Nucleotides (1994), 13(10), 2345-66). LCMS RT = 3.41 min.

### ii) (1R)-2-{[2-((3R)-3-{[(5-Bromopyridin-3-yl)methoxy]methyl)-2,3-dihydro-1,4-benzodioxin-6-yl)ethyl]amino}-1-(2,2-dimethyl-4H-1,3-benzodioxin-6-yl)ethanol

Prepared using methods similar to those described in Example 1xii. LCMS RT = 2.72 min.

### iii) 4-((1R)-2-{[2-((3R)-3-{[(5-Bromopyridin-3-yl)methoxylmethyl)-2,3-dihydro-1,4-benzodioxin-6-yl)ethyl]amino}-1-hydroxyethyl)-2-(hydroxymethyl)phenol acetate

Prepared using methods similar to those described in Example 1 xiii. LCMS RT = 2.32 min ; ES+ve 547 (MH)⁺

### Example 9

### 3-[({(2R)-7-[2-({(2R)-2-Hydroxy-2-[4-hydroxy-3-(hydroxymethyl)phenyl]ethyl}amino)ethyl]-2,3-dihydro-1,4-benzodioxin-2-yl}methoxy)methyl]benzonitrile acetate

### i) 3-{[((2R)-7-{2-[(5R)-5-(2,2-Dimethyl-4H-1,3-benzodioxin-6-yl)-2-oxo-1,3-oxazolidin-3-yl]ethyl}-2,3-dihydro-1,4-benzodioxin-2-yl)methoxy]methyl}benzonitrile

A solution of (5R)-5-(2,2-dimethyl-4*H*-1,3-benzodioxin-6-yl)-3-{2-[(3R)-3-(hydroxymethyl)-2,3-dihydro-1,4-benzodioxin-6-yl]ethyl}-1,3-oxazolidin-2-one (Example 1x.) (184.4mg) in DMF (5ml) under nitrogen was treated with sodium hydride (60% dispersion in mineral oil, 20mg) and stirred at 20° for 15 min. A solution of 3-(bromomethyl)benzonitrile (90.0mg) in DMF (3ml) was added and the mixture was stirred at 20° for 18 hours. The mixture was quenched with phosphate buffer (pH6.5, 10ml) and water (10ml) and the mixture extracted with EtOAc (2x15ml). The combined organic layers were washed with water (5ml), brine (2ml), dried (Na₂SO₄) and filtered. The solvent was evaporated *in vacuo* and the residue was chromatographed on silica (Flashmaster^{™} column chromatography, 10g), eluting with EtOAc-PE(1:3) to give the *title compound* (154mg). LCMS RT = 3.65min

### ii) 3-({[(2R)-7-(2-{[(2R)-2-(2,2-Dimethyl-4H-1,3-benzodioxin-6-yl)-2-hydroxyethyl]amino}ethyl)-2,3-dihydro-1,4-benzodioxin-2-yl]methoxy}methyl)benzonitrile,

A solution of 3-{[((2*R*)-7-{2-[(5R)-5-(2,2-dimethyl-4*H*-1,3-benzodioxin-6-yl)-2-oxo-1,3-oxazolidin-3-yl]ethyl}-2,3-dihydro-1,4-benzodioxin-2-yl)methoxy]methyl}benzonitrile (154mg) in THF (5ml) under nitrogen was treated with potassium trimethylsilanolate (180mg) and heated at 70° for 4h. The mixture was cooled to 20° and quenched with phosphate buffer (pH6.5ml, 15ml) and water (15ml). The mixture was extracted with EtOAc (30ml) and the combined organic extracts were washed with water (10ml), brine (2ml), and dried (Na₂SO₄). This was evaporated *in vacuo* to give a 1:1 mixture of the *title compound* and compound 10ii. The mixture was purified by chromatography on silica (Flashmaster Column chromatography^{™}, 10g) eluting with DCM-EtOH-ammonia (150:8:1, then 100:8:1) to give the *title compound* (61.6mg). LCMS RT=2.68min

### iii) 3-[({2R)-7-[2-({[2R)-2-Hydroxy-2-[4-hydroxy-3-(hydroxymethyl)phenyl]ethyl}amino)ethyl]-2,3-dihydro-1,4-benzodioxin-2-yl}methoxy)methyl]benzonitrile acetate

3-({[(2*R*)-7-(2-{[(2*R*)-2-(2,2-Dimethyl-4*H*-1,3-benzodioxin-6-yl)-2-hydroxyethyl]amino}ethyl)-2,3-dihydro-1,4-benzodioxin-2-yl]methoxy}methyl)benzonitrile, (61.6mg) was dissolved in glacial AcOH (5ml) and water (5ml) and the solution was heated to 70° for 0.5h. The mixture was cooled to 20° and the solvent was evaporated in *vacuo* to give a yellow oil. This was purified by Mass Directed Auto Preparative HPLC acetic acid was added and the solvant was removed to give the *title compound* (40.9mg). LCMS RT 2.39 min, ES+ve 491 (MH⁺)

### Example 10

### 3-[({(2R)-7-[2-({(2R)-2-Hydroxy-2-[4-hydroxy-3-(hydroxymethyl)phenyl]ethyl}amino)ethyl]-2,3-dihydro-1,4-benzodioxin-2-yl}methoxy)methyl)benzamide acetate

### i) 3-({[(2R)-7-(2-{[(2R)-2-(2,2-Dimethyl-4H-1,3-benzodioxin-6-yl)-2-hydroxyethyl]amino}ethyl)-2,3-dihydro-1,4-benzodioxin-2-yl]methoxy}methyl)benzamide

A solution of 3-{[((2*R*)-7-{2-[(5*R*)-5-(2,2-dimethyl-4*H*-1,3-benzodioxin-6-yl)-2-oxo-1,3-oxazolidin-3-yl]ethyl}-2,3-dihydro-1,4-benzodioxin-2-yl)methoxy]methyl}benzonitrile (Example 9i.) (154mg) in THF (5ml) under nitrogen was treated with potassium trimethylsilanolate (180mg) and heated at 70° for 4h. The mixture was cooled to 20° and quenched with phosphate buffer (pH6.5ml, 15ml) and water (15ml). The mixture was extracted with EtOAc (30ml) and the combined organic extracts were washed with water (10ml), brine (2ml), dried (Na₂SO₄) and filtered under vacuum. Solvent was evaporated *in vacuo* to give a 1:1 mixture of the *title compound* and compound (9ii). The mixture was purified by chromatography on silica (Flashmaster Column chromatography^{™}, 10g) eluting with DCM-EtOH-ammonia (150:8:1, then 100:8:1) to give the *title compound* (37.8mg. LCMS RT = 2.44min

### ii) 3-[({(2R)-7[2-({(2R)-2-Hydroxy-2-[4-hydroxy-3-(hydroxymethyl)phenyl]ethyl}amino)ethyl]-2,3-dihydro-1,4-benzodioxin-2-yl}methoxy)methyl]benzamide acetate

Prepared using methods similar to those described in Example 1(xiii) to give the *title compound* (23.9mg). LCMS RT 2.23min, ES+ve 509 (MH⁺)

### Example 11

### 4-[(1 R)-2-({2-[(3R)-3-({[3-(Cyclopentylthio)benzylloxy}methyl)-2,3-dihydro-1,4-benzodioxin-6-yl]ethyl}amino)-1-hydroxyethyl]-2-(hydroxymethyl)phenol acetate

### i) 1-(Bromomethyl)-3-(cyclopentylthio)benzene

A solution of 1-(hydroxymethyl)-3-(cyclopentylthio)benzene (WO 0324439 A1, 4.6g) in dry DCM (100mL) was treated with PPh₃ (14.5g) and portionwise with carbon tetrabromide (18.4g). The reaction mixture was stirred at room temperature for 3 h prior to partitioning between EtOAc and water. The organic phase was dried (MgSO₄) and concentrated *in vacuo.* The residue was purified by chromatography (SPE bond elut) using a gradient of EtOAc in cyclohexane (0-50%) to give the *title compound.* (1.1g) LCMS RT= 3.98 min.

### ii) (5R)-3-{2-[(3R)-3-({[3-(Cyclopentylthio)benzylloxy)methyl)-2,3-dihydro-14-benzodioxin-6-yl]ethyl}-5-(2,2-dimethyl-4H-1,3-benzodioxin-6-yl)-1,3-oxazolidin-2-one

A solution of (5*R*)-5-(2,2-dimethyl-4H-1,3-benzodioxin-6-yl)-3-{2-[(3R)-3-(hydroxymethyl)-2,3-dihydro-1,4-benzodioxin-6-yl]ethyl}-1,3-oxazolidin-2-one (Example 1x.) (340mg) in DMF (10ml) under nitrogen was treated with sodium hydride (60% dispersion in mineral oil, 46.21 mg) and stirred at 20° for 15 min. A solution of 1-(bromomethyl)-3-(cyclopentylthio)benzene (230mg) in DMF (3ml) was added and the mixture was stirred at 20° for 18 hours. The mixture was quenched with phosphate buffer (pH6.5, 10ml) and water and the mixture extracted with EtOAc. The combined organic layers were washed with water brine, dried (Na₂SO₄) and filtered. The solvent was evaporated *in vacuo* to give a yellow oil, which was chromatographed on silica (Flashmaster Column chromatography^{™}, 70g) eluting with EtOAc-cyclohexane (1:1) to give the *title compound* (361.4mg). LCMS RT 4.09min

### iii) (1R)-2-({2-[(3R)-3-({[3-(Cyclopentylthio)benzyl]oxy]methyl)-2,3-dihydro-1,4-benzodioxin-6-yl]ethyl}amino)-1-(2,2-dimethyl-4H-1,3-benzodioxin-6-yl)ethanol

Prepared using methods similar to those described in Example 9(ii) to give the *title compound* (38.7mg). LCMS RT 3.15min

### iv) 4-[(1R)-2-({2-[(3R)-3-({[3-(Cyclopentylthio)benzyl]oxy]methyl)-2,3-dihydro-1,4-benzodioxin-6-yl]ethyl}amino)-1-hydroxyethyl]-2-(hydroxymethyl)phenol acetate

Prepared using methods similar to those described in Example 9(iii) to give the *title compound* (33.3mg). LCMS RT 2.96min, ES+ve 566 (MH⁺)

### Example 12

### 4-[(1R)-2-({2-[(3R)-3-({[3-(Cyclopentylsulfonyl)benzylloxy)methyl)-2,3-dihydro-1,4-benzodioxin-6-yl]ethyl}amino)-1-hydroxyethyl]-2-(hydroxymethyl)phenol acetate

### i) (5R)-3-{2-[(3R)-3-({[3-(Cyclopentylsulfonyl)benzyl]oxy}methyl)-2,3-dihydro-1,4-benzodioxin-6-yl]ethyl}-5-(2,2-dimethyl-4H-1,3-benzodioxin-8-yl)-1,3-oxazolidin-2-one

A stirred cooled solution of (5*R*)-3-{2-[(3*R*)-3-({[3-(Cyclopentylthio)benzyl]oxy}methyl)-2,3-dihydro-1,4-benzodioxin-6-yl]ethyl}-5-(2,2-dimethyl-4*H*-1,3-benzodioxin-6-yl)-1,3-oxazolidin-2-one (Example 11 ii) (296.4mg) in dry DCM (20ml) was treated with 3-chloroperoxybenzoic acid (57-86%, 320mg). The solution was diluted with DCM (30ml) and washed with aqueous sodium sulphite. The extracts were washed with water (50ml), dried (Na₂SO₄) and evaporated to dryness *in vacuo* to give the *title compound* (330.2mg). LCMS RT 3.57 min

### ii) (1R)-2-({2-[(3R)-3-({[3-(Cyclopentylsulfonyl)benzyl]oxy}methyl)-2,3-dihydro-1,4-benzodioxin-6-yl]ethyl}amino)-1-(2,2-dimethyl-4H-1,3-benzodioxin-6-yl)ethanol

Prepared using methods similar to those described in Example 9(ii) and chromatographed on silica (Biotage^{™}, 8g) eluting with DCM-MeOH-ammonia (250:8:1) to give the *title compound* (114.9mg). LCMS RT 2.85min

### iii) 4-[(1R)-2-({2-[(3R)-3-({[3-(Cyclopentylsulfonyl)benzyl]oxy}methyl)-2,3-dihydro-1,4-benzodioxin-6-yl]ethyl}amino)-1-hydroxyethyl]-2-(hydroxymethyl)phenol acetate

Prepared using methods similar to those described in Example 9(iii) to give the *title compound* (102.7mg). LCMS RT 2.4.8min, ES+ve 598 (MH⁺)

### Example 13

### 2-(Hydroxymethyl)-4-{(1R)-1-hydroxy-2-[(2-{(3R)-3-[({5-[4-(methylsulfinyl)phenyl]pyridin-3-yl}methoxy)methyl]-2,3-dihydro-1,4-benzodioxin-6-yl}ethyl)amino]ethyl}phenol acetate

### i) (5R)-5-(2,2-Dimethyl-4H-1,3-benzodioxin-6-yl)-3-(2-{(3R)-3-[({5-[4-(methylsulfinyl)phenyl]pyridin-3-yl}methoxy)methyl]-2,3-dihydro-1,4-benzodioxin-6-yl}ethy)-1,3-oxazolidin-2-one

A solution of (5R)-3-[2-((3*R*)-3-{[(5-bromopyridin-3-yl)methoxy]methyl}-2,3-dihydro-1,4-benzodioxin-6-yl)ethyl]-5-(2,2-dimethyl-4H-1,3-benzodioxin-6-yl)-1,3-oxazolidin-2-one (Example 8i.) (0.072g), 4-(methylsulfinyl)phenylboronic acid (0.045g) and tetrakis(triphenylphosphine)palladium(0) (0.02g) in dimethoxyethane (4ml) and 2N sodium carbonate solution (3ml) was heated at 85° for 20 min. The mixture was poured into 2N sodium carbonate solution (30ml) and extracted into DCM (3x20ml).The extracts were dried (Na₂SO₄) and evaporated. The residual oil was purified on a biotage™ silica cartridge (4g) using 10% MeOH-EtOAc as the eluent. The appropriate fractions were evaporated to give the *title compound (0.067g)* LCMS RT = 3.09 min.

### ii) (1R)-1-(2,2-Dimethyl-4H-1,3-benzodioxin-6-yl)-2-[(2-{(3R)-3-[({5-[4-(methylsulfinyl)phenyl]pyridin-3yl}methoxy)methyl]-2,3-dihydro-1,4-benzodioxin-6-yl}ethyl)amino]ethanol

Prepared using methods similar to those described in Example 1xii.
LCMS RT = 2.48 min.

### iii) 2-(Hydroxymethyl)-4-{(1R)-1=hydroxy-2-[(2-{(3R)-3-[({5-[4-(methylsulfinyl)phenyl]pyridin-3-yl)methoxy)methy(1-2,3-dihydro-1,4-benzodioxin-6-methyl]1-2,3-dihydro-1.4-benzodioxin-6-yl}ethyl)aminolethyl}phenol acetate

Prepared using methods similar to those described in Example 1xiii. LCMS RT = 2.17 min: ES+ ve 605(MH⁺)

### Example 14

### N-{3-[({2R)-7-[2-({(2R)-2-Hydroxy-2-r4-hydroxy-3-(hydroxymethyl)phenyllethyl]amino)ethyl]-2,3-dihydro-1,4-benzodioxin-2-yl}methoxy)methyl]phenyl}urea formate

### i) N-[3-(Hydroxymethyl)phenyl]urea

3-[(Aminocarbonyl)amino]benzoic acid (500mg) was suspended in dry THF (25ml) and the mixture was cooled to 0° under nitrogen. Borane-THF complex (1M, 8.3ml) was added slowly over 20min and the mixture was stirred at room temperature for 16h. The mixture was once again cooled to 0° and water was cautiously added, followed by HCI (2N). The reaction mixture was extracted with EtOAc and the organic layers were washed with brine and dried (Na₂SO₄). The solvent was removed by evaporation, MeOH was added and the mixture was filtered to give a white solid. The solid was purified using an isolute^{™} SPE aminopropyl column. This gave the *title compound* (65mg). LCMS RT=1.5min

### ii) N-[3-(Bromomethyl)phenyl]urea

N-[3-(hydroxymethyl)phenyl]urea (25mg) was suspended in dry DCM (1ml) under an atmosphere of nitrogen. Phosphorus tribromide (18µl) was added and the mixture was stirred for 2h at 20°. The solution was used without purification. LCMS RT=2.5min

### iii) N-(3-{[((2R)-7-{2-[(5R)-5-(2,2-Dimethyl-4H-1,3-benzodioxin-6-yl)-2-oxo-1,3-oxazolidin-3-yl]ethyl}-2,3-dihydro-1,4-benzodioxin-2-yl)methoxy]methyl}phenyl)urea

(5*R*)-5-(2,2-dimethyl-4*H*-9,3-benzodioxin-6-yl)-3-{2-[(3*R*)-3-(hydroxymethyl)-2,3-dihydro-1,4-benzodioxin-6-yl]ethyl}-1,3-oxazolidin-2-one (Example 1x.) (31 mg) and tetrabutylammonium hydrogen sulphate (3mg) were added to a solution of NaOH (250mg) in water (0.5ml). N-[3-(Bromomethyl)phenyl]urea in solution in DCM (1 ml) was added to the stirred mixture and the reaction mixture was heated at 45° for 16h. Water (0.5ml) was added and the organic phase was separated. The solvent was removed to give the *title compound* which was used without further purification in Example 14iv. LCMS RT=3.23min

### iv) N-[3-({[(2R)-7-(2-{[(2R)-2-(2,2-Dimethyl-4H-1,3-benzodioxin-6-yl)-2-hydroxyethyl]amino}ethyl)-2,3-dihydro-1,4-benzodioxin-2-yl]methoxy)methyl)phenyl]urea

To a stirred solution of *N*-(3-{[((2*R*)-7-{2-[(5*R*)-5-(2,2-dimethyl-4*H*-1,3-benzodioxin-6-yl)-2-oxo-1,3-oxazolidin-3-yl]ethyl}-2,3-dihydro-1,4-benzodioxin-2-yl)methoxy]methyl}phenyl)urea (Example 14iv) in dry THF (1ml) was added potassium trimethylsilanolate (80mg) and the reaction mixture was heated at 65° for 2h. Water was added and the mixture was extracted with DCM. The solvent was removed giving the *title compound.* LCMS RT=2.5min

### v) N-{3-[({(2R)-7-[2-({(2R)-2-Hydroxy-2-[4-hydroxy-3-(hydroxymethyl}phenyl]ethyl}amino)ethyl]-2,3-dihydro-1,4-benzodioxin-2-yl}methoxy)methyl]phenyl}urea formate

A solution of *N*-[3-({[(2*R*)-7-(2-{[(2*R*)-2-(2,2-dimethyl-4H-1,3-benzodioxin-6-yl)-2-hydroxyethyl]amino}ethyl)-2,3-dihydro-1,4-benzodioxin-2-yl]methoxy}methyl)phenyl]urea in glacial AcOH (1ml) and water (0.5ml) was heated at 70° for 1h. The solvent was removed and the residue was purified by mass-directed autopreparative HPLC to give the *title compound* (1.4mg). LCMS RT=2.23min ES+ve 524 (MH)⁺

### Example 15

### 4-((1R)-2-{[2-((3R)-3-{[(4-Chlorobenzyl)oxy]methyl}-2,3-dihydro-1,4-benzodioxin-6-yl)ethyl]amino}-1-hydroxyethyl)-2-(hydroxymethyl)phenol

### i) (5R)-3-[2-((3R)-3-{[(4-Chlorobenzyl)oxy]methyl}-2,3-dihydro-1,4-benzodioxin-6-yl)ethyl]-5-(2,2-dimethyl-4H-1,3-benzodioxin-6-yl)-1,3-oxazolidin-2-one

Prepared using methods similar to those used in Example 14 iii) using 1-(bromomethyl)-4-chlorobenzene. LCMS RT=3.8min

### ii) (1R)-2-{[2-((3R)-3-{[(4-Chlorobenzyl)oxy]methyl)-2,3-dihydro-1,4-benzodioxin-6-yl)ethyl]amino}-1-(2,2-dimethyl-4H-1,3-benzodioxin-6-yl)ethanol

Prepared using methods similar to those used in Example 14 iv). LCMS RT=2.8min

### iii) 4-((1R)2-(f2-((3R)-3-{((4-Chlorobenzyl)oxy]methyl)-2,3-dihydro-1,4-benzodioxin-6-yl)ethl]amino)-1-hydroxyethyl)-2-(hydroxymethyl)phenol

Prepared using methods similar to those used in Example 14 v). The crude product was purified using mass-directed autopreparative HPLC followed by chromatography on a SPE bond elut column using DCM-MeOH-aqueous ammonia (94:6:1). This gave the *title compound* (4.3mg). LCMS RT=2.6min ES+ve 500, 502 (MH)⁺

### Example 16

### 4-((1 R)-2-{([2-((3R)-3-{[(4-Fluorobenzyl)oxylmethyl}-2,3-dihydro-1,4-benzodioxin-6-yl)ethyl]amino}-1-hydroxyethyl)-2-(hydroxymethyl)phenol formate

### i) (5R)-5-(2,2-Dimethyl-4H-1,3-benzodioxin-6-yl)-3-[2-((3R)-3-[[(4-fluorobenzyl)oxy]methyl}-2,3-dihydro-1,4-benzodioxin-6-y))ethyn-1.3-oxazolidin-2-one

Prepared using methods similar to those used in Example 14 iii) using 1-(bromomethyl)-4-fluorobenzene. LCMS RT=3.7min

### ii (1R)-1-(2,2-Dimethyl-4H-1,3-benzodioxin-6-yl)-2-[[2-((3R)-3-[[(4-fluorobenzyl)oxy]methyl}-2,3-dihydro-1,4-benzodioxin-6-yl)ethyllamino)ethanol

Prepared using methods similar to those used in Example 14iv). LCMS RT=2.7min

### iii) 4-((1R)-2-{[2-((3R)-3-{[(4-Fluorobenzyl)oxylmethyl}-2,3-dihydro-1,4-benzodioxin-6-yl)ethyl]amino}-1-hydroxyethyl)-2-(hydroxymethyl)phenol formate

Prepared using methods similar to those used in Example 14 v). LCMS RT=2.5min ES+ve 484 (MH)⁺

### Example 17

### 4-((l R)-2-{[2-((3R)-3-{[(3,5-Dimethylbenzyl)oxy]methyl}-2,3-dihydro-1,4-benzodioxin-6-yl)ethyl]amino}-1-hydroxyethyl)-2-(hydroxymethyl)phenol formate

### i) (5R)-5-(2,2-Dimethyl-4H-1,3-benzodioxin-6-yl)-3-[2-((3R)-3-{[(3,5 dimethylbenzyl]oxy]methyl}-2,3-dihydro-1,4-benzodioxin-6-yl)ethyl]-1,3-oxazolidin-2-one

Prepared using methods similar to those used in Example 14. iii) using 1-(bromomethyl)-3,5-dimethylbenzene. LCMS RT=3.9min

### ii) (1R)-1-(2,2-Dimethly-4H-1.3-benzodioxin-6-yl)-2-{[2-((3R)-3-{[(3,5-dimethylbenzyl)oxy]methyl}-2,3-dihydro-1,4-benzodioxin-6-yl)ethyl]amino}ethanol

Prepared using methods similar to those used in Example 14 iv). LCMS RT=2.8min

### iii) 4-((1 R)-2-{[2-((3R)-3-{[(3,5-Dimethylbenzyl)oxy]methyl}-2,3-dihydro-1,4-benzodioxin-6-yl)ethyl]amino}-1-hydroxyethyl)-2-(hydroxymethyl)phenol formate

Prepared using methods similar to those used in Example 14 v). LCMS RT=2.6min ES+ve 494 (MH)⁺

### Example 18

### 2-(Hydroxymethyl)-4-{(1R)-1-hydroxy-2-[(2-{(3R)-3-[(1-phenylethoxy)methyl]-2,3-dihydro-1,4-benzodioxin-6-yl}ethyl)amino]ethyl}phenol formate

### i) (5R)-5-(2,2-Dimethyl-4H-1,3-benzodioxin-6-yl)-3-(2-{(3R)-3-[(1-phenylethoxy)methyl]-2,3-dihydro-1,4-benzodioxin-6-yl}ethyl)-1,3-oxazolidin-2-one

Prepared using methods similar to those used in Example 14 iii) using (1-bromoethyl)benzene. LCMS RT=3.8min

### ii) (1R)-1-(2,2-Dimethyl-4H-1,3-benzodioxin-6-yl)-2-[(2-((3R)-3-[(1-phenylethoxy)methyl]-2,3-dihydro-1,4-benzodioxin-6-yl)ethyl)aminolethanol

Prepared using methods similar to those used in Example 14 iv) LCMS RT=2.7min

### iii 2-(Hydroxymethyl)-4-{(1R)-1-hydroxy-2-[(2-{(3R)-3-[(1-phenylethoxy)methyl]-2,3-dihydro-1,4-benzodioxin-6-yl}ethyl)amino]ethyl}phenol formate

Prepared using methods similar to those used in Example 14 v) LCMS RT=2.5min ES+ve 480 (MH)⁺

### Example 19.

### 2-(Hydroxymethyl)-4-[(1R)-1-hydroxy-2-({2-{(3R)-3-({[3-(methylsulfonyl)benzyl]oxy}methyl)-2,3-dihydro-1,4-benzodioxin-6-yl]ethyl}amino)ethyl]phenol formate

### i) (5R)-5-(2,2-Dimethyl-4H-1,3-benzodioxin-6-yl)-3-{2-[(3R)-3-({(3-(methylsulfonyl)benzyl]oxy}methyl)-2,3-dihydro-1,4-benzodioxin-6-yl]ethyl}-1,3-oxazolidin-2-one

Prepared using methods similar to those used in Example 14 iii) using 1-(bromomethyl)-3-(methylsulfonyl)benzene (WO 9910316 A1). LCMS RT=3.4min

### ii) (1R)-1-(2,2-Dimethyl-4H-1.3-benzodioxin-6-yl)-2-({2-[(3R)-3-({[3-(methylsulfonyl)benzyl]oxy}methyl)-2,3-dihydro-1,4-benzodioxin-6-yl]ethyl}amino)ethanol

Prepared using methods similar to those used in Example 14 iv). LCMS RT = 2.5min

### iii) 2-(Hydroxymethyl)-4-[(1R)-1-hydroxy-2-({2-[(3R)-3-({[3-(methylsulfonyl)benzylloxy}methyl)-2,3-dihydro-1,4-benzodioxin-6-yl]ethyl}amino)ethyl]phenol formate

Prepared using methods similar to those used in Example 14 v). LCMS RT= 2.3min ES+ve 544 (MH)⁺

### Example 20

### 4-((1 R)-2-{[2-((3R)-3-{[3-(2,6-Dichlorophenyl)propoxylmethyl}-2,3-dihydro-1,4-benzodioxin-6-yl)ethyllaminol-1-hydroxyethyl)-2-(hydroxymethyl)phenol formate

### i) (5R)-3-[2-((3R)-3-{[3-(2,6-Dichlorophenyl)propoxy]methyl)-2,3-dihydro-1,4-benzodioxin-6-yl)ethyl]-5-(2,2-dimethyl-4H-1,3-benzodioxin-6,yl)-1,3-oxazolidin-2-one -

Prepared using methods similar to those used in Example 14 iii) using 2-(3-bromopropyl)-1,3-dichlorobenzene Journal of Medicinal Chemistry 1967, 10, 391-400). LCMS RT=4.1 min

### ii) (1R)-2-{[2-((3R)-3-{[3-(2,6-Dichlorophenyl)propoxylmethyl]-2,3-dihydro-1,4-benzodioxin-6-yl)ethyllamino}-1-(2.2-dimethyl-4H-1,3-benzodioxin-6-yl)ethanol -

Prepared using methods similar to those used in Example 14 iv). LCMS RT=3.3min

### iii) 4-((1R)-2-{[2-((3R)-3-{[3-(2.6-Dichlorophenyl)propoxylmethyl-2,3-dihydro-1,4-benzodioxin-6-yl)ethyl]amino}-1-hydroxyethyl)-2-(hydroxymethyl)phenol formate

Prepared using methods similar to those used in Example 14 v). LCMS RT=2.9min ES+ve 562, 564(MH)⁺

### Example 21

### 3-[({(2R)-7-[2-({(2R)-2-Hydroxy-2-[4-hydroxy-3-(hydroxymethyl)phenyl]ethyl}amino)ethyl]-2,3-dihydro-1,4-benzodioxin-2-yl}methoxy)methyl]benzenesulfonamide formate

### i) 3-(Bromomethyl)-N,N-bis{[2-(trimethylsilyl)ethoxylmethyl)benzenesulfonamide

Zinc bromide (690mg) was added to a solution of 3-(hydroxymethyl)-*N,N-*bis{[2-(trimethylsilyl)ethoxy]methyl}benzenesulfonamide (WO 0324439 A1) (910mg) and PPh₃ (1.1g) in toluene (20ml). Diisopropylazodicarboxylate (0.8ml) was added dropwise and the resulting mixture was stirred at room temperature under nitrogen for 16h. The solvent was removed and the residue was purified by silica SPE bond elut using cyclohexane - EtOAc (8:1) as eluent. This gave the *title compound* (200mg). LCMS RT = 4.29min

### ii) 3-{[((2R)-7-{2-[(5R)-5-(2.2-Dimethyl-4H-1,3-benzodioxin-6-yl)-2-oxo-1,3-oxazolidin-3-yl]ethyl}-2,3-dihydro-1.4-benzodioxin-2-yl)methoxylmethyl)-N,N-bis{[2-(trimethylsilyl)ethoxy]methyl}benzenesulfonamide

Prepared using methods similar to those used in Example 14 iii) using 3-(bromomethyl)-*N*,*N*-bis{[2-(trimethylsilyl)ethoxy]methyl)benzenesulfonamide.LCMS RT=4.4min

### iii 3-({[(2R)-7-(2-{[(2R)-2-(2.2-Dimethyl-4H-1,3-benzodioxin-6-yl)-2-hydroxyethyl]amino}ethyl)-2,3-dihydro-1,4-benzodioxin-2-yl]methoxy)methyl)-N,N-bis{[2-(trimethylsilyl)ethoxy]methyl}benzenesulfonamide)

Prepared using methods similar to those used in Example 14 iv).. LCMS RT=3.5min

### iv) 3-[({(2R)-7-[2-({(2R)-2-Hydroxy-2-(4-hydroxy-3-(hydroxymethyl)phenyl]ethyl)amino)ethyl]-2,3-dihydro-1,4-benzodioxin-2-yl}methoxy)methyl]benzenesulfonamide formate

A solution of 3-({[(2*R*)-7-(2-{[(2*R*)-2-(2,2-dimethyl-4*H*-1,3-benzodioxin-6-yl)-2-hydroxyethyl]amino}ethyl)-2,3-dihydro-1,4-benzodioxin-2-yl]methoxy}methyl)-*N,N*-bis{[2-(trimethylsilyl)ethoxy]methyl}benzenesulfonamide (132mg) in glacial AcOH (5ml) and water (2.5ml) was heated at 70° for 24h. The solvent was removed to give a residue, which was purified using mass-directed autopreparative HPLC. This gave the *title compound* (21 mg). LCMS RT=2.18min ES+ve 545(MH)⁺

### Example 22

### 6-{2-{(2-{(3R)-3-[(Benzyloxy)methyl]-2,3-dihydro-1,4-benzodioxin-6-yl]ethyl)amino]-1-hydroxyethyl}-2-(hydroxymethyl)pyridin-3-ol

### i) (2R)-2-[(Benzyloxy)methyl]-7-(2-iodoethyl)-2,3-dihydro-1,4-benzodioxine

A stirred solution of 2-{(2R)-2-[(benzyloxy)methyl]-2,3-dihydro-1,4-benzodioxin-6-yl}ethyl methanesulfonate (Example 1iv.) (11g) and tetrabutylammonium iodide (34 g) in MeCN (150ml) was heated at 70° for 3 h. The mixture was concentrated in vacuo and the residue was partitioned between diethyl ether and water. The organic extracts were washed with aqueous sodium metabisulphite, dried (Na₂SO₄) and evaporated to give the title compound (8.6 g). LCMS RT = 3.84 min.

### ii) 2-[(2-{(3R)-3-[(Benzyloxy)methyl]-2,3-dihydro-1,4-benzodioxin-6-yl}ethyl)amino]-1-(2-phenyl-4H-[1,3]dioxino[5,4-b]pyridin-6-yl)ethanol

(2*R*)-2-[(benzyloxy)methyl]-7-(2-iodoethyl)-2,3-dihydro-1,4-benzodioxine (71 mg) was added to a solution of 2-amino-1-(2-phenyl-4*H*-[1,3]dioxino[5,4-b]pyridin-6-yl)ethanol (54mg) (EP220054A2) and *N,N*-diisopropylethylamine (0.084ml) in DMF (2ml). The resulting mixture was stirred at room temperature under nitrogen for 72h. The DMF was blown off at 40° with nitrogen, and the residue purified by silica SPE bond elut, eluting with MeOH-DCM mixtures to give the *title compound* (48mg) LCMS RT = 2.9 min

### iii) 6-{2-[(2-{(3R)-3-[(Benzyloxy)methyl]-2,3-dihydro-1,4-benzodioxin-6-ylethyl)amino]-1-hydroxyl}ethyl]-2-(hydroxymethyl)pyridin-3-ol

2-[(2-{(3*R*)-3-[(Benzyloxy)methyl]-2,3-dihydro-1,4-benzodioxin-6-yl}ethyl)amino]-1-(2-phenyl-4*H*-[1,3]dioxino[5,4-b]pyridin-6-yl)ethanol (48mg) was dissolved in glacial AcOH (2ml) and water (1ml). The solution was heated for 30 min at 80°. The solvent was removed, and the residue was purified by silica SPE bond elut, eluting with MeOH-DCM mixtures, to give the *title compound* (31 mg). LCMS RT = 2.4 min, ES+ve 467 (MH)⁺

### Example 23

### N-(5-{(1R)-2-[(2-{(3R)-3-[(Benzyloxy)methyl]-2,3-dihydro-1,4-benzodioxin-6-yl}ethyl)amino]-1-hydroxyethyl}-2-hydroxyphenyl)methanesulfonamide

### i) N-[2-(Benzyloxy)-5-((1R)-1-hydroxy-2-{[(1S)-2-hydroxy-1-phenylethyl]amino}ethyl)phenyl]methanesulfonamide

A solution of N-[2-(benzyloxy)-5-(bromoacetyl)phenyl]methanesulfonamide (Journal of Medicinal Chemistry (1980), 23(7), 738-44) (1.15g) in dry DMF (30ml) was treated with diisopropylethylamine (1.06ml) and (S)-phenylglycinol (474mg) and the reaction mixture stirred at room temperature for 4h. The reaction mixture was concentrated *in vacuo* and the residue re-suspended in MeOH (50ml). The reaction mixture was cooled to 0° and treated with CaCl₂ (1.27g). The reaction mixture was stirred at 0° for 30 min prior to portionwise addition of NaBH₄ (218mg) ensuring that the temperature did not rise above 10° . After complete addition the reaction mixture was allowed to warm to room temperature and stirred for a further 74h. The reaction mixture was concentrated *in vacuo* and the residue partitioned between EtOAc and water. The organic phase was dried and concentrated *in vacuo.* Chromatography on silica (SPE, gradient from DCM to DCM-MeOH-NH₃(aq) 100:10:1) afforded *the title compound* (85mg). LCMS RT= 2.48min

### ii) N-{5-[(1R)-2-Amino-1-hydroxyethyl]-2-hydroxyphenyl}methanesulfonamide

Palladium hydroxide (40mg, 50% water) was flushed with nitrogen and treated with a solution of *N*-[2-(benzyloxy)-5-((1*R*)-1-hydroxy-2-{[(1*S*)-2-hydroxy-1-phenylethyl]amino} ethyl)phenyl]methanesulfonamide (400mg) in MeOH (80ml) and glacial AcOH (0.5ml). The reaction mixture was stirred under hydrogen for 16 h prior to flushing the reaction mixture with nitrogen and filtering to remove the catalyst and concentrating *in vacuo.* The residue was purified by chromatography (OASIS cartridge, eluted with water, 5% MeOH in water 50% MeOH in water and MeOH) to give *the title compound* (182mg). δ_{H} (400MHz, CD₃OD) 7.38 (1 H, d, J 2Hz), 7.12 (1 H, dd, J 2, 8Hz), 6.90 (1 H, d, J 8Hz), 4.78 (1 H, dd, J 2, 10Hz), 3.08 (1 H, dd, J 2, 15Hz), 2.98 (1 H, bd, J 10Hz), 2.93 (3H, s).

### iii) N-(5-{(1R)-2-[(2-{(3R)-3-[(Benzyloxylmethyl]-2,3-dihydro-1.4-benzodioxin-6-yl}ethyl)amino]-1-hydroxyethyl}-2-hydroxyphenyl)methanesulfonamide

Prepared using methods similar to those described in Example 22ii. LCMS RT = 2.6 min ES+ve 529 (MH)⁺.

### Example 24

### 4-{(1 1R)-2-[(2-f(3R)-3-[(Benzyloxy)methyl]-2,3-d ihydro-1.4-benzodioxin-6-yl)ethyl)aminol-1-hydroxyethyl}-2-fluorophenol

### i) 2-Azido-1-[4-(benzyloxy)-3-fluorophenyl]ethanone

A solution of 2-bromo-1-[4-(benzyloxy)-3-fluorophenyl]ethanone *(*J. Med. Chem. 1980, 23, 738-744) (1g) in dry DMF (2.5ml) was cooled to 15° and treated portionwise with sodium azide (220mg). After complete addition the reaction mixture was stirred for a further 1h. The reaction mixture was partitioned between EtOAc and water. The organic phase was washed with water and the combined aqueous phase back extracted with EtOAc. The combined organic phase was washed with sat. NaHCO_{3(aq)} three times and the combined washes back extracted with EtOAc. The combined organic phase was washed with brine, dried and concentrated *in vacuo.* The residue was purified by column chromatography on silica, eluting with hexane-EtOAc (4:1 and 2:1) to give the *title compound* (810mg). LCMS RT= 3.61 min.

### ii) (1R)-2-Azido-1-[4-(benzyloxy)-3-fluorophenyl]ethanol

Borane-dimethylsulphide solution in THF (2M, 0.03 ml) was added to a solution of (R)-2-methyl-CBS-oxazaborolidine in toluene (1M, 0.06ml) at 0° with stirring. The reaction mixture was stirred for 15 min prior to the dropwise addition of a solution of 2-azido-1-[4-(benzyloxy)-3-fluorophenyl]ethanone (100mg) in THF. Further Borane-dimethylsulphide in THF (2M, 0.03ml) was added dropwise and the reaction mixture stirred at 0° for 2h. 2M HCl_{(aq)} (2ml) was added dropwise and the reaction mixture stirred for 10 min prior to partitioning the reaction mixture between diethyl ether and water. The organic phase was washed twice with 2M HCl_{(aq)}, three times with sat. NaHCO_{3(aq)}, water and brine. The organic phase was dried and concentrated *in vacuo.* The residue was purified by column chromatography on silica gel, eluting with DCM to give the *title compound* (470mg). LCMS RT= 3.36 min.

### iii) (1R)-2-Amino-1-[4-(benzyloxy)-3-fluorophenyl]ethanol

A solution of (1*R*)-2-azido-1-[4-(benzyloxy)-3-fluorophenyl]ethanol (410mg) in THF (8mL) and water (2ml) was treated with PPh₃ (410mg) and stirred for 1h prior to addition of further PPh₃ (220mg). After stirring for a further 4h the reaction mixture was concentrated *in vacuo* and the residue partitioned between EtOAc and water. The organic phase was washed three times with 5% NaHCO_{3(aq)} dried and concentrated *in vacuo.* The residue was purified by chromatography on silica gel, eluting with DCM, 1% MeOH in DCM, 2% MeOH in DCM, 5% MeOH containing 0.5% Et₃N in DCM, and finally 20% MeOH containing 1 % Et₃N in DCM) to give *the title compound* (260mg). LCMS RT= 2.16 min.

### iv) 4-[(1R)-2-Amino-1-hydroxyethyl]-2-fluorophenol

Palladium on carbon (10% Pd by weight, wet, 50mg) was flushed with nitrogen and treated with a solution of (1*R*)-2-amino-1-[4-(benzyloxy)-3-fluorophenyl]ethanol (500mg) in EtOH (25ml), EtOAc (25ml) and glacial AcOH (10ml). The reaction mixture was stirred under hydrogen for 5 h prior to flushing the reaction mixture with nitrogen and filtering to remove the catalyst and concentrating *in vacuo.* The residue was purified by chromatography (SCX bond elut cartridge) eluting with DCM, MeOH and DCM-MeOH-NH₃(aq) 100:10:1) to give the *title compound* (308mg). ¹H NMR (CD₃OD)= 7.05 (1H, dd, J 2, 12Hz), 6.94 (1H, dd, J 2, 9Hz), 6.86 (1 H, t, *J* 9Hz), 4.54 (1 H, dd, J 5, 8Hz), 2.78 (1H, d, J 5Hz), 2.77 (1 H, d, J 8Hz).

### v) 4-{(1R)-2-[(2-{(3R)-3-[(Benzyloxy)methyl]-2,3-dihydro-1,4-benzodioxin-6-yl}ethyl)aminol-1-hydroxyethyl)-2-fluorophenol

Prepared using methods similar to those described in Example 22ii). LCMS RT = 2.6 min; ES+ve 454 (MH)⁺.

### Example 25

### 4-{(1R)-2-[(2-{(3R)-3-{(Benzyloxy)methyll-2,3-dihydro-1,4-benzodioxin-6-yl}ethyl)amino]-1-hydroxyethyl}-3-methylphenol

### i) 1-{2-Methyl-4-[(phenylmethyl)oxy]phenyl]ethanone

Benzyl bromide (20ml, 28.76g, 168.2mmol) was added to a stirred mixture of (1-(4-hydroxy-2-methylphenyl)ethanone (25.57g, 168.3mmol) and potassium carbonate (34.7g, 251.07mmol) in 2-butanone (250ml). The reaction mixture was heated at reflux for 17h then cooled to room temperature and filtered. The precipitate was washed with 2-butanone and the combined filtrate and washings concentrated *in vacuo.* The residue was suspended in cyclohexane and cooled to 0 °. Filtration of the precipitate afforded the *title compound.* (35.3g). LCMS RT = 3.46min

### ii) 1-[4-(Benzyloxy)-2-methylphenyl]-2-bromoethanone

Phenyltrimethylammonium bromide (7.8g) was added portionwise to a stirred solution of 1-[4-(Benzyloxy)-2-methylphenyl]ethanone (5.7g) in dry THF (50ml) ensuring the temperature did not exceed 10°. A further portion of phenyltrimethylammonium bromide (1.2g) was added. Water was added to the reaction mixture and the precipitate was collected by filtration. The precipitate was purified by column chromatography on silica using cyclohexane - DCM (1:1) to give the *title compound* (5.2g). LCMS RT = 3.60 min

### iii) (1R)-1-[4-(Benzyloxy)-2-methylphenyl}-2-{[(1S)-2-hydroxy-1-phenylethyl]amino)ethanol

Prepared using methods similar to those described in Example 23i. LCMS RT = 2.64 min

### iv) 4-[(1R)-2-Amino-1-hydroxymethyl]1-3-methypheno

Prepared using methods similar to those described in Example 23ii.
LCMS RT = 1.91 min

### v) 4-{(1R)-2-[(2-{(3R)-3-[(Benzyloxy)methyl]-2,3-dihydro-1,4-benzodioxin-6-yl}ethyl)amino]-1-hydroxyethyl}-3-methylphenol

Prepared using methods similar to those described in Example 22ii. LCMS RT = 2.6 min; ES+ve 450 (MH)⁺.

### Example 26

### (1R)-1-(4-Amino-3,5-dichlorophenyl)-2-[(2-{(3R)-3-[(benzyloxy)methyl]-2,3-dihydro-1,4-benzodioxin-6-yl}ethyl)amino]ethanol

i) Prepared using methods similar to those described in Example 22ii using (1 R)-2-amino-1-(4-amino-3,5-dichlorophenyl)ethanol (EP 460924 A1). LCMS RT = 2.8 min; ES+ve 503 (MH)⁺.

### Example 27

### 5-{(1 R)-2-[(2-{(3R)-3-{(Benzyloxy)methyll-2,3-dihydro-1,4-benzodioxin-6-yl)ethyl)aminol-1-hydroxyethyl}-2-hydroxyphenylformamide

### i) {5-{(1R)-2-[Bis(phenylmethyl)amino]-1-hydroxyethyl)-2-[(phenylmethyl)oxy]phenyl}formamide

A mixture of {5-[(2R)-2-oxiranyl]-2-[(phenylmethyl)oxy]phenyl}formamide (WO 0276933) (200mg) and dibenzylamine (0.75ml) were heated in a microwave oven at 150° for 30 min. The mixture was allowed to cool to 20° and was purified on a silica SPE bond elut cartridge (10g) using a gradient of 0% to 50% EtOAc in cyclohexane (Gradmaste^{™}). The appropriate fractions were evaporated *in vacuo* and the residue further purified by mass-directed autopreparative HPLC to give the *title compound* (123mg). LCMS RT = 2.75min.

### ii) (5-{(1R)-2-[Bis(phenylmethyl)amino]-1-hydroxyethyl}-2-hydroxyphenyl)formamide

A solution of {5-{(1*R*)-2-[bis(phenylmethyl)amino]-1-hydroxyethyl}-2-[(phenylmethyl)oxy]phenyl}formamide (1.40g) in EtOAc (15ml) and EtOH (15ml) was hydrogenated over 10% palladium on carbon (140mg). When hydrogen uptake had ceased the mixture was filtered through celite, the solvent evaporated *in vacuo* and the residue purified on a silica SPE bond elut cartridge (70g) using a gradient of 0% to 5% MeOH in DCM (Gradmaster^{™}). The appropriate fractions were evaporated *in vacuo* to give the *title compound* (380mg). LCMS RT = 2.17min.

### iii) {5-{(1R)-2-[Bis(phenylmethyl)amino]-1-hydroxyethyl)-2-[({[2-(trimethylsilyl)ethyl]oxy}methyl)oxy]phenyl}formamide

A solution of (5-{(1*R*)-2-[bis(phenylmethyl)amino]-1-hydroxyethyl}-2-hydroxyphenyl)formamide (354mg) in DMF (10ml) under nitrogen was treated with sodium hydride (60% dispersion in mineral oil, 38mg) and the mixture stirred at 20° for 10 min. 2-(Trimethylsilyl)ethoxymethyl chloride (0.17m1) was added and the mixture was stirred at 20° for 3h. Phosphate buffer solution (pH 6.5) and water were added and the mixture was extracted with EtOAc. The extract was washed with water and dried (Na₂SO₄). Solvent evaporation *in vacuo* gave a residue which was purified on a silica SPE bond elut cartridge (20g) using a gradient of 0% to 2% MeOH in DCM (Gradmaster^{™}). The appropriate fractions were evaporated *in vacuo* to give the *title compound* (286mg). LCMS RT = 3.08min.

### iv) {5-[(1R)-2-Amino-1-hydroxyethyl]-2-[({[2-{trimethylsilyl)ethyl]oxy}methyl)oxy]phenyl}formamide

A solution of {5-{(1*R*)-2-[bis(phenylmethyl)amino]-1-hydroxyethyl}-2-[({[2-(trimethylsilyl)ethyl]oxy}methyl)oxy]phenyl}formamide (90mg) in EtOAc (8ml) and EtOH (8ml) was hydrogenated over 10% palladium on carbon (40mg) and 20% palladium hydroxide on carbon (20mg). When hydrogen uptake had ceased the mixture was filtered through celite and the solvent evaporated *in vacuo* to give the *title compound* (52mg). LCMS RT = 2.31 min.

### v) (2R)-2-[(Benzyloxy)methyl]-7-(2-bromoethyl)-2,3-dihydro-1,4-benzodioxine

Tetrabutylammonium bromide (148mg) was added to a solution of 2-{(3R)-3-[(benzyloxy)methyl]-2,3-dihydro-1,4-benzodioxin-6-yl}ethyl methanesulfonate (Example 1iv.) (58mg) in MeCN (1ml) and the resulting solution was heated at 70° for 2h. The mixture was partitioned between DCM and water and the organic extract was concentrated *in vacuo.* The resulting residue was purified by chromatography on a silica SPE bond elut cartridge eluting with cyclohexane - EtOAc mixtures to give the *title compound* (37mg). LCMS RT = 3.8min

### vi) 5-{(1R)-2-[(2-{(3R)-3-[(Benzyloxy)methyl]-2,3-dihydro-1,4-benzodioxin-6-yl}ethyl)amino]-1-hydroxyethyl}-2-{[2-(trimethylsilyl)ethoxy]methoxy}phenylformamide

A solution of (2*R*)-2-[(Benzyloxy)methyl]-7-(2-bromoethyl)-2,3-dihydro-1,4-benzodioxine (28mg), {5-[(1*R*)-2-Amino-1-hydroxyethyl]-2-[({[2-(trimethylsilyl)ethyl]oxy}methyl) oxy]phenyl}formamide (25mg) and N,N-diisopropylethylamine (28µl) in DMF(0.5ml) was heated at 70° for 72h. The reaction mixture was partitioned between aqeuous NaHCO₃ and EtOAc and the combined organic extracts were washed with brine and dried (Na₂SO₄). The solvent was removed to give the *title compound* (55mg) LCMS RT = 3.1 min

### vii) 5-{(1R)-2-[(2-{(3R)-3-[(Benzyloxy)methyl]-2,3-dihydro-1,4-benzodioxin-6-yl}ethyl)amino]-1-hydroxymethyl}-2-hydroxyphenylformamide

5-{(1R)-2-[(2-{(3R)-3-[(benzyloxy)methyl]-2,3-dihydro-1,4-benzodioxin-6-yl}ethyl)amino]-1-hydroxyethyl}-2-{[2-(trimethylsilyl)ethoxy]methoxy}phenylformamide (55mg) was dissolved in glacial AcOH (2ml) and water (1ml) and the solution was heated at 70° for 7h. The solvent was removed and the residue was azeotroped with MeOH to give the *title compound.* LCMS RT = 2.4min ES+ve 479 (MH)⁺

### BIOLOGICAL ACTIVITY

### In vitro measurements of compound potency and intrinsic activity at the human Beta 1, 2 and 3 receptors.

### Method 1

The potencies of the compounds of Examples 1-4 were determined using frog melanophores transfected with the human beta 2 adrenoreceptor. The cells were incubated with melatonin to induce pigment aggregation. Pigment dispersal was induced by compounds acting on the human beta 2 adrenoreceptor. The beta 2 agonist activity of test compounds was assessed by their ability to induce a change in light transmittance across a melanophore monolayer (a consequence of pigment dispersal). At the human beta 2 adrenoreceptor, compounds of said examples had IC₅₀ values below 1 µM.

### Method 2

Potency of compounds of the invention at the human beta 2, 1 and 3 receptors was also determined using Chinese hamster ovary cells co-expressing the human receptor with a reporter gene. Studies were performed using either whole cells or membranes derived from those cells.

The three beta-receptors are coupled via the Gs G-protein to cause a stimulation of adenylate cyclase resulting in increased levels of cAMP in the cell. For direct cAMP measurements either membranes or cells have been used with either the HitHunter enzyme fragment complementation kit (DiscoveRx) or the FP² fluorescence polarisation kit (Perkin Elmer) to quantify the levels of cAMP present. These assays provide a measure of agonist potency and intrinsic activity of the compounds at the various receptors.

The reporter gene in the cells has also been used to quantify potency at the beta 1 and 3 receptors. This is a reporter of cAMP levels using the cAMP response element upstream of a firefly luciferase gene. After stimulation of the receptor with an agonist an increase in the level of luciferase is measured as a quantification of the level of cAMP in the cell.

In this assay the potency of compounds at the human beta-2 receptor is expressed as a pEC₅₀ value. Compounds of Examples 1-15, 17-19 and 22-26 had a pEC₅₀ of >6.

The application of which this description and claims forms part may be used as a basis for priority in respect of any subsequent application. The claims of such subsequent application may be directed to any feature or combination of features described herein. They may take the form of product, composition, process, or use claims and may include, by way of example and without limitation, the following claims:

## Claims

1. A compound of formula (I): 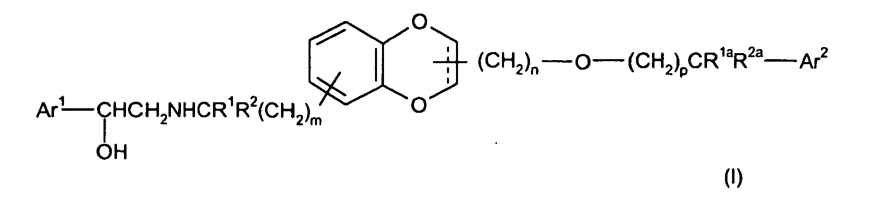 or a salt or solvate thereof, wherein:
Ar¹ is a group selected from 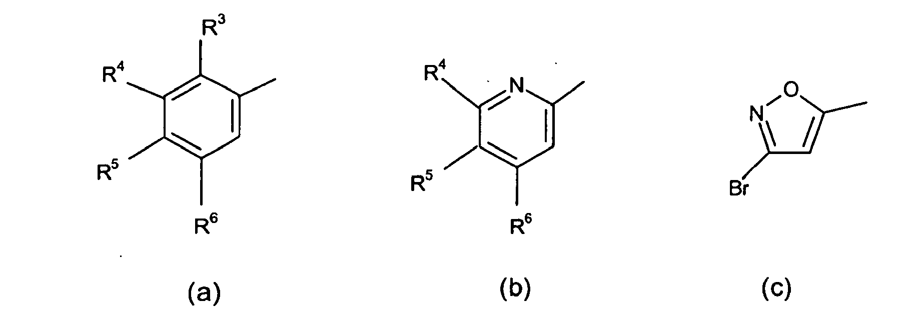 and 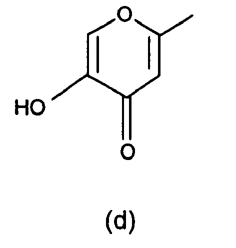
wherein R⁴ represents hydrogen, halogen, -(CH₂)_{q}OR⁷, -NR⁷C(O)R⁸, -NR⁷SO₂R⁸, -SO₂NR⁷R⁸, -NR⁷R⁸, -OC(O)R⁹ or OC(O)NR⁷R⁸, and R³ represents hydrogen, halogen or C₁₋₄ alkyl;
or R⁴ represents -NHR¹⁰ and R³ and -NHR¹⁰ together form a 5- or 6- membered heterocyclic ring;
R⁵ represents hydrogen, halogen, -OR⁷ or -NR⁷R⁸;
R⁶ represents hydrogen, halogen, haloC₁₋₄alkyl, -OR⁷, -NR⁷R⁸, -OC(O)R⁹ or OC(O)NR⁷R⁸;
R⁷ and R⁸ each independently represents hydrogen or C₁₋₄ alkyl, or in the groups -NR⁷R⁸, -SO₂NR⁷R⁸ and -OC(O)NR⁷R⁸, R⁷ and R⁸ independently represent hydrogen or C₁₋₄ alkyl or together with the nitrogen atom to which they are attached form a 5-, 6- or 7- membered nitrogen-containing ring,
R⁹ represents an aryl (eg phenyl or naphthyl) group which may be unsubstituted or substituted by one or more substituents selected from halogen, C₁₋₄ alkyl, hydroxy, C₁₋₄ alkoxy or halo C₁₋₄ alkyl; and
q is zero or an integer from 1 to 4;
Ar² is a group: 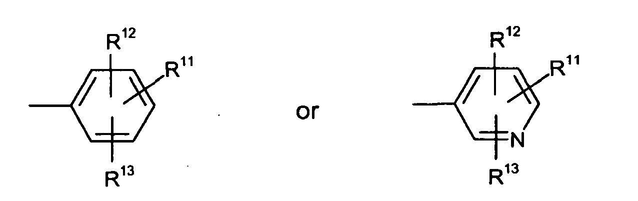 wherein
R¹¹ is selected from hydrogen, C₁₋₆alkyl, hydroxy, C₁₋₆ alkoxy, cyano, nitro, halo, C₁₋₆haloalkyl, XCO₂R¹⁶, -XC(O)NR¹⁵R¹⁶, -XNR¹⁴C(O)R¹⁵, -XNR¹⁴C(O)NR¹⁵R¹⁶, -XNR¹⁴C(O)NC(O)NR¹⁵R¹⁶, -XNR¹⁴SO₂R¹⁵, -XSO₂NR¹⁷R¹⁸, XSR¹⁴, XSOR¹⁴, XSO₂R¹⁴, -XNR¹⁵R¹⁶, -XNR¹⁴C(O)OR¹⁵, or XNR¹⁴SO₂NR¹⁵R¹⁶,
or R¹¹ is selected from -X-aryl, -X-hetaryl, or -X-(aryloxy), each optionally substituted by 1 or 2 groups independently selected from hydroxy, C₁₋₆alkoxy, halo, C₁₋₆alkyl, C₁₋₆haloalkyl, cyano, nitro, CONR¹⁵R¹⁶,
-NR¹⁴C(O)R¹⁵, SR¹⁴, SOR¹⁴, -SO₂R¹⁴, -SO₂NR¹⁷R¹⁸, -CO₂R¹⁶, -NR¹⁵R¹⁶, or hetaryl optionally substituted by 1 or 2 groups independently selected from hydroxy, C₁₋₆alkoxy, halo, C₁₋₆alkyl, or C₁₋₆haloalkyl;
X is -(CH₂)ᵣ- or C₂₋₆ alkenylene;
r is an integer from 0 to 6, preferably 0 to 4;
R¹⁴ and R¹⁵ are independently selected from hydrogen, C₁₋₆alkyl, C₃₋₇cycloalkyl, aryl, hetaryl, hetaryl(C₁₋₆alkyl)- and aryl(C₁₋₆alkyl)- and R¹⁴ and R¹⁵ are each independently optionally substituted by 1 or 2 groups independently selected from halo, C₁₋₆alkyl, C₃₋₇ cycloalkyl, C₁₋₆ alkoxy, C₁₋₆haloalkyl -NHC(O)(C₁₋₆alkyl), -SO₂(C₁₋₆alkyl), -SO₂(aryl), -CO₂H, and -CO₂(C₁₋₄alkyl), -NH₂, -NH(C₁₋₆alkyl), aryl(C₁₋₆alkyl)-, aryl(C₂₋₆alkenyl)-, aryl(C₂₋₆alkynyl)-, hetaryl(C₁₋₆alkyl)-, -NHSO₂aryl, -NH(hetarylC₁₋₆alkyl), -NHSO₂hetaryl, -NHSO₂(C₁₋₆alkyl), -NHC(O)aryl, or -NHC(O)hetaryl:
or R¹⁴ and R¹⁵, together with the nitrogen atom to which they are bonded, form a 5-, 6- or 7-membered nitrogen- containing ring;
or where R¹¹ is -XNR¹⁴C(O)NR¹⁵R¹⁶, R¹⁴ and R¹⁵ may, together with the -NC(O)N- portion of the group R¹ to which they are bonded, form a saturated or unsaturated ring, preferably a 5-, 6-, or 7- membered ring, for example an imidazolidine ring, such as imidazolidine-2,4-dione;
or where R¹¹ is -XNR¹⁴C(O)OR¹⁵,R¹⁴ and R¹⁵ may, together with the -NC(O)O- portion of the group R¹¹ to which they are bonded, form a saturated or unsaturated ring, preferably a 5-, 6-, or 7- membered ring, for example an oxazolidine ring, such as oxazolidine-2,4-dione;
R¹⁶ is selected from hydrogen, C₁₋₆alkyl and C₃₋₇ cycloalkyl;
or where R¹¹ is -XC(O)NR¹⁵R¹⁶ or -XNR¹⁴C(O)NR¹⁵R¹⁶, R¹⁵ and R¹⁶ may, together with the nitrogen to which they are bonded, form a 5-, 6-, or 7- membered nitrogen containing ring;
R¹⁷ and R¹⁸ are independently selected from hydrogen, C₁₋₆alkyl, C₃₋₇cycloalkyl, aryl, hetaryl, hetaryl(C₁₋₆alkyl)- and aryl(C₁₋₆alkyl)-, or R¹⁷ and R¹⁸, together with the nitrogen to which they are bonded, form a 5-, 6-, or 7- membered nitrogen containing ring;
and R¹⁷ and R¹⁸ are each optionally substituted by one or two groups independently selected from halo, C₁₋₆alkyl, and C₃₋₇cycloalkyl, C₁₋₆haloalkyl ;
R¹² is selected from hydrogen, hydroxy, C₁₋₆alkyl, C₁₋₆alkoxy, halo, aryl, aryl(C₁₋₆alkyl)-, C₁₋₆haloalkoxy, and C₁₋₆haloalkyl ;
R¹³ is selected from hydrogen, hydroxy, C₁₋₆alkyl, C₁₋₆alkoxy, halo, aryl, aryl(C₁₋₆alkyl)-, C₁₋₆haloalkoxy, and C₁₋₆haloalkyl ;
R¹ and R² are independently selected from hydrogen and C₁₋₄ alkyl with the proviso that the total number of carbon atoms in R¹ and R² is not more than 4;
one of R^{1a} and R^{2a} is selected from hydrogen and C₁₋₄alkyl, and the other of R^{1a} and R^{2a} represents hydrogen or C₁₋₄alkyl ;
m is an integer of from 1 to 3;
n is an integer of from 1 to 4; and
p is zero or an integer of from 1 to 3;
and ---- represents a single or double bond.

2. A compound of formula (I) as defined in claim 1, or a salt or solvate thereof, wherein R^{1a} and R^{2a} each represent hydrogen;
and in the group Ar¹ :
R⁴ represents halogen, -(CH₂)qOR₇, -NR⁷C(O)R⁸, -NR⁷SO₂R⁸, -SO₂NR⁷R⁸, -NR⁷R⁸, -OC(O)R⁹ or OC(O)NR⁷R⁸, and R³ represents hydrogen or C₁₋₄ alkyl;

3. A compound of formula (I) as defined in claim 1, or a salt or solvate thereof, wherein R^{1a} and R^{2a} each represent hydrogen;
and in the group Ar¹:
R⁴ represents -NHR¹⁰ and R³ and -NHR¹⁰ together form a 5- or 6- membered heterocyclic ring.

4. A compound of formula (I) according to any one of claims 1 to 3 wherein the group Ar¹ is selected from groups (a) and (b) as defined in claim 1.

5. A compound of formula (I) according to any of claims 1 to 4 wherein, in the group Ar², R¹¹ is selected from hydrogen, C₁₋₄alkyl, hydroxy, halo, -NR¹⁴C(O)NR¹⁵R¹⁶, -NR¹⁴SO₂R¹⁵ and XSO₂NR¹⁷R¹⁸ wherein R¹⁴ to R¹⁸ are as defined in claim 1.

6. A compound of formula (I) according to any of claims 1 to 4 wherein, in the group Ar², R¹¹ is selected from cyano, -CONR¹⁵R¹⁶, SR¹⁴, SOR¹⁴ and SO₂R¹⁴, wherein R¹⁴, R¹⁵ and R¹⁶ are as defined in claim 1.

7. A compound of formula (I) according to any of claims 1 to 6 wherein R¹² and R¹³ each represent hydrogen.

8. A compound of formula (I) according to any of claims 1 to 6 wherein R¹¹ represents hydrogen and R¹² and R¹³ each represent halogen or C₁₋₆alkyl.

9. A compund of formula (I) according to any of claims 1 to 8 wherein R¹ and R² are both hydrogen.

10. A compound of formula (I) according to any of claims 1 to 9 wherein each of m and n is independently 1 or 2, and p is zero or 1.

11. A compound of formula (I) selected from:
4-((1*R*)-2-{[2-((3*R*)3-{[(2,6-Dichlorobenzyl)oxy]methyl}-2,3-dihydro-1,4-benzodioxin-6-yl)ethyl]amino}-1-hydroxyethyl)-2-(hydroxymethyl)phenol;
4-{(1*R*)-2-[(2-{(3*R*)-3-[(Benzyloxy)methyl]-2,3-dihydro-1,4-benzodioxin-6-yl}ethyl)amino]-1-hydroxyethyl}-2-(hydroxymethyl)phenol;
4-{(1*R*)-2-[(2-{(3*S*)-3-[(Benzyloxy)methyl]-2,3-dihydro-1,4-benzodioxin-6-yl}ethyl)amino]-1-hydroxyethyl}-2-(hydroxymethyl)phenol;
2-(Hydroxymethyl)-4-{(1*R*)-1-hydroxy-2-[(2-{(3R)-3-[(pyridin-3-ylmethoxy)methyl]-2,3-dihydro-1,4-benzodioxin-6-yl}ethyl)amino]ethyl}phenol;
4-((1*R*)-2-{[2-((3*R*)-3-{[(6-Chloropyridin-3-yl)methoxy]methyl}-2,3-dihydro-1,4-benzodioxin-6-yl)ethyl]amino}-1-hydroxyethyl)-2-(hydroxymethyl)phenol;
4-((1R)-2-{[2-((3R}-3-{[(2,6-Dichloropyridin-3-yl)methoxy]methyl}-2,3-dihydro-1,4-benzodioxin-6-yl)ethyl]amino}-1-hydroxyethyl)-2-(hydroxymethyl)phenol;
4-{(1*R*)-2-[(2-{2-[(Benzyloxy)methyl]-2,3-dihydro-1,4-benzodioxin-6-yl)ethyl)amino]-1-hydroxyethyl}-2-(hydroxymethyl)phenol;
4-((1*R*)-2-([2-((3*R*)-3-([(5-Bromopyridin-3-yl)methoxy]methyl)-2,3-dihydro-1,4-benzodioxin-6-yl)ethyl]amino}-1-hydroxyethyl)-2-(hydroxymethyl)phenol;
3-[({(2*R*)-7-[2-({(2*R*)-2-Hydroxy-2-[4-hydroxy-3-(hydroxymethyl)phenyl]ethyl}amino)ethyl]-2,3-dihydro-1,4-benzodioxin-2-yl}methoxy)methyl]benzonitrile;
3-[({(2*R*)-7-[2-({(2*R*)-2-Hydroxy-2-[4-hydroxy-3-(hydroxymethyl)phenyl]ethyl}amino)ethyl]-2,3-dihydro-1,4-benzodioxin-2-yl}methoxy)methyl]benzamide;
4-[(1*R*)-2-({2-[(3*R*)-3-({[3-(Cyclopentylthio)benzyl]oxy}methyl)-2,3-dihydro-1,4-benzodioxin-6-yl]ethyl}amino)-1-hydroxyethyl]-2-(hydroxymethyl)phenol;
4-[(1*R*)-2-({2-[(3*R*)-3-({[3-(Cyclopentylsulfonyl)benzyl]oxy}methyl)-2,3-dihydro-1,4-benzodioxin-6-yl]ethyl}amino)-1-hydroxyethyl]-2-(hydroxymethyl)phenol;
2-(Hydroxymethyl)-4-{(1*R*)-1-hydroxy-2-[(2-{(3*R*)-3-[({5-[4-(methylsulfinyl)phenyl]pyridin-3-yl}methoxy)methyl]-2,3-dihydro-1,4-benzodioxin-6-yl}ethyl)amino]ethyl}phenol;
N-{3-[({(2*R*)-7-[2-({(2*R*)-2-Hydroxy-2-[4-hydroxy-3-(hydroxymethyl)phenyl]ethyl}amino)ethyl]-2,3-dihydro-1,4-benzodioxin-2-yl}methoxy)methyl]phenyl}urea;
4-((1 *R*)-2-{[2-((3*R*)-3-{[(4-Chlorobenzyl)oxy]methyl}-2,3-dihydro-1,4-benzodioxin-6-yl)ethyl]amino}-1-hydroxyethyl)-2-(hydroxymethyl)phenol;
4-((1*R*)-2-{[2-((3*R*}-3-{[(4-Fluorobenzyl)oxy]methyl}-2,3-dihydro-1,4-benzodioxin-6-yl)ethyl]amino}-1-hydroxyethyl)-2-(hydroxymethyl)phenol;
4-((1 *R*)-2-{[2-((3*R*)-3-{[(3,5-Dimethylbenzyl)oxy]methyl}-2,3-dihydro-1,4-benzodioxin-6-yl)ethyl]amino}-1-hydroxyethyl)-2-(hydroxymethyl)phenol;
2-(Hydroxymethyl)-4-{(1*R*)-1-hydroxy-2-[(2-{(3*R*)-3-[(1-phenylethoxy)methyl]-2,3-dihydro-1,4-benzodioxin-6-yl}ethyl)amino]ethyl}phenol;
2-(Hydroxymethyl)-4-[(1*R*)-1-hydroxy-2-({2-[(3*R*)-3({[3-(methylsulfonyl)benzyl]oxy)methyl)-2,3-dihydro-1,4-benzodioxin-6-yl]ethyl}amino)ethyl]phenol;
4-((1 *R*)-2-{[2-((3*R*)-3-{[3-(2,6-Dichlorophenyl)propoxy]methyl}-2,3-dihydro-1,4-benzodioxin-6-yl)ethyl]amino}-1-hydroxyethyl)-2-(hydroxymethyl)phenol;
3-[({(2*R*)-7-[2-({(2*R*)-2-Hydroxy-2-[4-hydroxy-3-(hydroxymethyl)phenyl]ethyl}amino)ethyl]-2,3-dihydro-1,4-benzodioxin-2-yl}methoxy)methyl]benzenesulfonamide;
6-{2-[(2-{(3*R*)-3-[(Benzyloxy)methyl]-2,3-dihydro-1,4-benzodioxin-6-yl}ethyl)amino]-1-hydroxyethyl}-2-(hydroxymethyl)pyridin-3-ol;
N-(5-{(1*R*)-2-[(2-{(3*R*)-3-[(Benzyloxy)methyl]-2,3-dihydro-1,4-benzodioxin-6-yl}ethyl)amino]-1-hydroxyethyl}-2-hydroxyphenyl)methanesulfonamide;
4-{(1*R*)-2-[(2-{(3*R*)-3-[(Benzyloxy)methyl]-2,3-dihydro-1,4-benzodioxin-6-yl}ethyl)amino]-1-hydroxyethyl}-2-fluorophenol;
4-{(1*R*)-2-[(2-{(3R)-3-[(Benzyloxy)methyl]-2,3-dihydro-1,4-benzodioxin-6-yl}ethyl)amino]-1-hydroxyethyl}-3-methylphenol;
(1 *R*)-1-(4-Amino-3,5-dichlorophenyl)-2-[(2-((3*R*)-3-[(benzyloxy)methyl]-2,3-dihydro-1,4-benzodioxin-6-yl}ethyl)amino]ethanol;
5-{(1*R*)-2-[(2-{(3R)-3-[(Benzyloxy)methyl]-2,3-dihydro-1,4-benzodioxin-6-yl)ethyl)amino]-1-hydroxyethyl}-2-hydroxyphenylformamide;
or a salt or solvate thereof.

12. A compound of formula (I) according to any of claims 1 to 10, or a pharmaceutically acceptable salt, solvate for use in medical therapy.

13. A pharmaceutical formulation comprising a compound of formula (I) according to any of claims 1 to 10, or a pharmaceutically acceptable salt, solvate, or physiologically functional derivative thereof, and a pharmaceutically acceptable carrier or excipient, and optionally one or more other therapeutic ingredients.

14. The use of a compound of formula (I) according to any of claims 1 to 10, or a pharmaceutically acceptable salt, solvate, or physiologically functional derivative thereof in the manufacture of a medicament for the prophylaxis or treatment of a clinical condition for which a selective β₂-adrenoreceptor agonist is indicated.

15. A process for the preparation of a compound of formula (I), according to any of claims 1 to 10, or a salt, solvate, or physiologically functional derivative thereof, which comprises:
(a) deprotection of a protected intermediate, for example of formula (II). 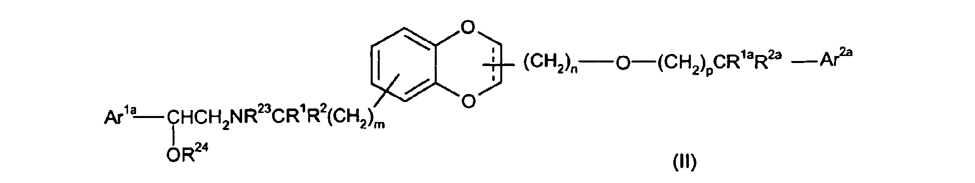 or a salt or solvate thereof, wherein R¹, R², R^{1a}, R^{2a}, m, n, p and ---- are as defined for the compound of formula (I), Ar^{1a} represents an optionally protected form of Ar¹ ; Ar^{2a} represents an optionally protected form of Ar² and R²³ and R²⁴ are each independently either hydrogen or a protecting group, provided that the compound of formula (II) contains at least one protecting group;
(b) alkylation of an amine of formula 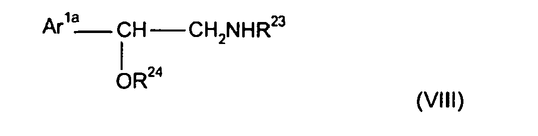
wherein Ar^{1a}, R²³ and R²⁴ are as defined for formula (II) with a compound of formula (XV): 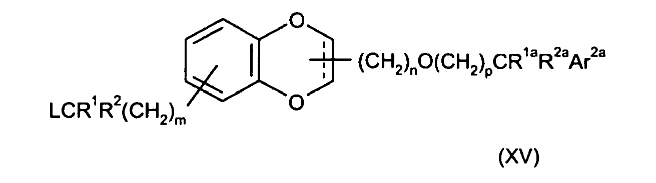 wherein ----, Ar², R¹, R², R^{1a}, R^{2a}, m, n and p are as defined for the compound of formula (II) and L is a leaving group as defined for formula (IX);
followed by the following steps in any order:
(i) optional removal of any protecting groups;
(ii) optional separation of an enantiomer from a mixture of enantiomers;
(iii) optional conversion of the product to a corresponding salt, solvate,
or physiologically functional derivative thereof.

## Patentansprüche

1. Verbindung der Formel (I): 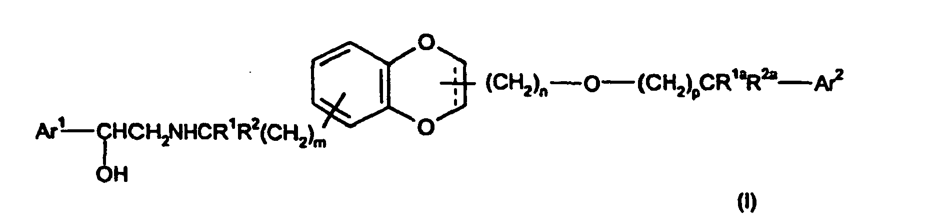 oder ein Salz oder ein Solvat davon, worin:
Ar¹ eine Gruppe ist, die ausgewählt ist aus 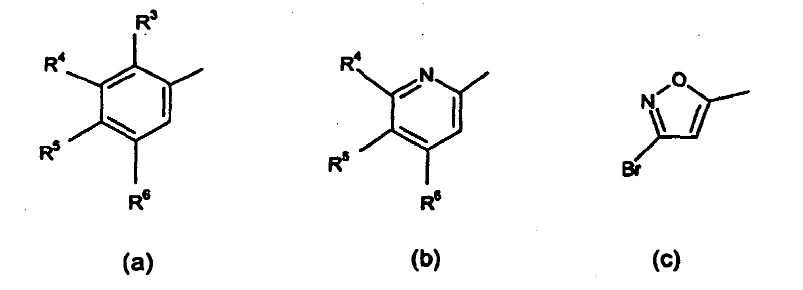 und 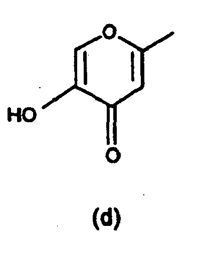
worin R⁴ Wasserstoff, Halogen, -(CH₂)_{q}OR⁷, -NR⁷C(O)R⁸, -NR⁷SO₂R⁸, -SO₂NR⁷R⁸, -NR⁷R⁸, -OC(O)R⁹ oder OC(O)NR⁷R⁸ darstellt,
und R³ Wasserstoff, Halogen oder C₁₋₄-Alkyl darstellt;
oder R⁴ -NHR¹⁰ darstellt und R³ und -NHR¹⁰ zusammen einen Heterocyclyl-Ring mit 5 oder 6 Gliedern bilden;
R⁵ Wasserstoff, Halogen, -OR⁷ oder -NR⁷R⁸ darstellt;
R⁶ Wasserstoff, Halogen, Halogen-C₁₋₄-alkyl, -OR⁷, -NR⁷R⁸, -OC(O)R⁹ oder OC(O)NR⁷R⁸ darstellt;
R⁷ und R⁸ jeweils unabhängig Wasserstoff oder C₁₋₄-Alkyl darstellen, oder R⁷ und R⁸ in den Gruppen -NR⁷R⁸, -SO₂ NR⁷R⁸ und -OC(O)NR⁷R⁸ unabhängig Wasserstoff oder C₁₋₄-Alkyl darstellen oder zusammen mit dem Stickstoffatom, an das sie gebunden sind, einen Stickstoff enthaltenden Ring mit 5, 6 oder 7 Gliedern bilden;
R⁹ eine Aryl-Gruppe (z.B. Phenyl- oder Naphthyl-Gruppe) darstellt, die unsubstituiert oder mit einem oder mehreren Substituenten substituiert sein kann, die ausgewählt sind aus Halogen, C₁₋₄-Alkyl, Hydroxy, C₁₋₄-Alkoxy oder Halogen-C₁₋₄-alkyl; und
q Null oder eine ganze Zahl von 1 bis 4 ist;
Ar² eine Gruppe ist: 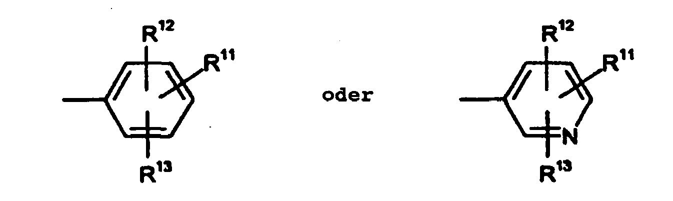 worin
R¹¹ ausgewählt ist aus Wasserstoff, C₁₋₆-Alkyl, Hydroxy, C₁₋₆-Alkoxy, Cyano, Nitro, Halogen, C₁₋₆-Halogenalkyl, XCO₂R¹⁶, -XC(O)NR¹⁵R¹⁶, -XNR¹⁴C(O)R¹⁵, -XNR¹⁴C(O)NR¹⁵R¹⁶, -XNR¹⁴C(O)NC(O)NR¹⁵R¹⁶, -XNR¹⁴SO₂R¹⁵, -XSO₂NR¹⁷R¹⁸, XSR¹⁴, XSOR¹⁴, XSO₂R¹⁴, -XNR¹⁵R¹⁶, -XN-R¹⁴C(O)OR¹⁵ oder XNR¹⁴SO₂NR¹⁵R¹⁶, oder R¹¹ ausgewählt ist aus -X-Aryl, -X-Hetaryl oder -X-(Aryloxy), wobei jedes gegebenenfalls mit 1 oder 2 Gruppen substituiert ist, die unabhängig ausgewählt sind aus Hydroxy, C₁₋₆-Alkoxy, Halogen, C₁₋₆-Alkyl, C₁₋₆-Halogenalkyl, Cyano, Nitro, CONR¹⁵R¹⁶, -NR¹⁴C(O)R¹⁵, SR¹⁴, SOR¹⁴, -SO₂R¹⁴, -SO₂NR¹⁷R¹⁸, -CO₂R¹⁶, -NR¹⁵R¹⁶ oder Hetaryl, das gegebenenfalls mit 1 oder 2 Gruppen substituiert ist, die unabhängig ausgewählt sind aus Hydroxy, C₁₋₆-Alkoxy, Halogen, C₁₋₆-Alkyl oder C₁₋₆-Halogenalkyl;
X -(CH₂)ᵣ- oder C₂₋₆-Alkenylen ist;
r eine ganze Zahl von 0 bis 6, vorzugsweise 0 bis 4, ist;
R¹⁴ und R¹⁵ unabhängig ausgewählt sind aus Wasserstoff, C₁₋₆-Alkyl, C₃₋₇-Cycloalkyl, Aryl, Hetaryl, Hetaryl(C₁₋₆-alkyl)- und Aryl(C₁₋₆-alkyl)- und R¹⁴ und R¹⁵ jeweils unabhängig gegebenenfalls mit 1 oder 2 Gruppen substituiert sind, die unabhängig ausgewählt sind aus Halogen, C₁₋₆-Alkyl, C₃₋₇-Cycloalkyl, C₁₋₆-Alkoxy, C₁₋₆-Halogenalkyl, -NHC(O)(C₁₋₆-Alkyl), -SO₂(C₁₋₆-Alkyl), -SO₂(Aryl), -CO₂H und -CO₂(C₁₋₄-Alkyl), -NH₂, -NH(C₁₋₆-Alkyl), Aryl(C₁₋₆-alkyl)-, Aryl(C₂₋₆-alkenyl)-, Aryl(C₂₋₆-alkinyl)-, Hetaryl(C₁₋₆-alkyl)-, -NHSO₂-Aryl, -NH(Hetaryl-C₁₋₆-alkyl), -NHSO₂-Hetaryl, -NHSO₂(C₁₋₆-Alkyl), -NHC(O)Aryl oder -NHC(O)Hetaryl ;
oder R¹⁴ und R¹⁵ zusammen mit dem Stickstoffatom, an das sie gebunden sind, einen Stickstoff enthaltenden Ring mit 5, 6 oder 7 Gliedern bilden;
oder, wenn R¹¹ -XNR¹⁴C(O)NR¹⁵R¹⁶ ist, R¹⁴ und R¹⁵ zusammen mit dem -NC(O)N-Teil der R¹-Gruppe, an die sie gebunden sind, einen gesättigten oder ungesättigten Ring bilden können, vorzugsweise einen Ring mit 5, 6 oder 7 Gliedern, zum Beispiel einen Imidazolidin-Ring wie Imidazolidin-2,4-dion;
oder, wenn R¹¹ -XNR¹⁴C(O)OR¹⁵ ist, R¹⁴ und R¹⁵ zusammen mit dem -NC(O)O-Teil der R¹¹-Gruppe, an die sie gebunden sind, einen gesättigten oder ungesättigten Ring bilden können, vorzugsweise einen Ring mit 5, 6 oder 7 Gliedern, zum Beispiel einen Oxazolidin-Ring wie Oxazolidin-2,4-dion;
R¹⁶ ausgewählt ist aus Wasserstoff, C₁₋₆-Alkyl und C₃₋₇-Cycloalkyl;
oder, wenn R¹¹ -XC(O)NR¹⁵R¹⁶ oder -XNR¹⁴C(O)NR¹⁵R¹⁶ ist, R¹⁵ und R¹⁶ zusammen mit dem Stickstoff, an den sie gebunden sind, einen Stickstoff enthaltenden Ring mit 5, 6 oder 7 Gliedern bilden können;
R¹⁷ und R¹⁸ unabhängig ausgewählt sind aus Wasserstoff, C₁₋₆-Alkyl, C₃₋₇-Cycloalkyl, Aryl, Hetaryl, Hetaryl(C₁₋₆-alkyl)- und Aryl(C₁₋₆-alkyl)-, oder R¹⁷
und R¹⁸ zusammen mit dem Stickstoff, an den sie gebunden sind, einen Stickstoff enthaltenden Ring mit 5, 6 oder 7 Gliedern bilden;
und R¹⁷ und R¹⁸ jeweils gegebenenfalls mit 1 oder 2 Gruppen substituiert sind, die unabhängig ausgewählt sind aus Halogen, C₁₋₆₋Alkyl, C₃₋₇-Cycloalkyl und C₁₋₆-Halogenalkyl;
R¹² ausgewählt ist aus Wasserstoff, Hydroxy, C₁₋₆-Alkyl, C₁₋₆-Alkoxy, Halogen, Aryl, Aryl(C₁₋₆-alkyl)-, C₁₋₆-Halogenalkoxy und C₁₋₆-Halogenalkyl ;
R¹³ ausgewählt ist aus Wasserstoff, Hydroxy, C₁₋₆-Alkyl, C₁₋₆-Alkoxy, Halogen, Aryl, Aryl(C₁₋₆-alkyl)-, C₁₋₆-Halogenalkoxy und C₁₋₆-Halogenalkyl ;
R¹ und R² unabhängig ausgewählt sind aus Wasserstoff und C₁₋₄-Alkyl mit der Bedingung, daß die Gesamtzahl der Kohlenstoffatome in R¹ und R² nicht mehr als 4 ist;
eines von R^{1a} und R^{2a} ausgewählt ist aus Wasserstoff und C₁₋₄-Alkyl, und das andere von R^{1a} und R^{2a} Wasserstoff oder C₁₋₄-Alkyl darstellt;
m eine ganze Zahl von 1 bis 3 ist;
n eine ganze Zahl von 1 bis 4 ist; und
p Null oder eine ganze Zahl von 1 bis 3 ist;
und ---- eine Einfach- oder Doppelbindung darstellt.

2. Verbindung der Formel (I) gemäß Anspruch 1 oder ein Salz oder Solvat davon, worin R^{1a} und R^{2a} jeweils Wasserstoff darstellen;
und in der Ar¹-Gruppe:
R⁴ Halogen, -(CH₂)_{q}OR⁷, -NR⁷C(O)R⁸, -NR⁷SO₂R⁸, -SO₂ NR⁷R⁸, -NR⁷R⁸, -OC(O)R⁹ oder OC(O)NR⁷R⁸ darstellt und R³ Wasserstoff oder C₁₋₄-Alkyl darstellt.

3. Verbindung der Formel (I) gemäß Anspruch 1 oder ein Salz oder Solvat davon, worin R^{1a} und R^{2a} jeweils Wasserstoff darstellen;
und in der Ar¹-Gruppe:
R⁴ -NHR¹⁰ darstellt und R³ und -NHR¹⁰ zusammen einen Heterocyclyl-Ring mit 5 oder 6 Gliedern bilden.

4. Verbindung der Formel (I) gemäß einem der Ansprüche 1 bis 3, worin die Ar¹-Gruppe aus den in Anspruch 1 definierten Gruppen (a) und (b) ausgewählt ist.

5. Verbindung der Formel (I) gemäß einem der Ansprüche 1 bis 4, worin in der Ar²-Gruppe R¹¹ ausgewählt ist aus Wasserstoff, C₁₋₄-Alkyl, Hydroxy, Halogen, -NR¹⁴C(O)NR¹⁵R¹⁶, -NR¹⁴SO₂R¹⁵ und XSO₂NR¹⁷R¹⁸, worin R¹⁴ bis R¹⁸ wie in Anspruch 1 definiert sind.

6. Verbindung der Formel (I) gemäß einem der Ansprüche 1 bis 4, worin in der Ar²-Gruppe R¹¹ ausgewählt ist aus Cyano, -CONR¹⁵R¹⁶, SR¹⁴, SOR¹⁴ und SO₂R¹⁴, worin R¹⁴, R¹⁵ und R¹⁶ wie in Anspruch 1 definiert sind.

7. Verbindung der Formel (I) gemäß einem der Ansprüche 1 bis 6, worin R¹² und R¹³ jeweils Wasserstoff darstellen.

8. Verbindung der Formel (I) gemäß einem der Ansprüche 1 bis 6, worin R¹¹ Wasserstoff darstellt und R¹² und R¹³ jeweils Halogen oder C₁₋₆-Alkyl darstellen.

9. Verbindung der Formel (I) gemäß einem der Ansprüche 1 bis 8, worin R¹ und R² beide Wasserstoff sind.

10. Verbindung der Formel (I) gemäß einem der Ansprüche 1 bis 9, worin jedes von m und n unabhängig 1 oder 2 ist und p Null oder 1 ist.

11. Verbindung der Formel (I), die aus folgenden ausgewählt ist:
4-((lR)-2-{[2-((3R)-3-{[(2,6-Dichlorbenzyl)oxy]-methyl}-2,3-dihydro-1,4-benzodioxin-6-yl)ethyl]amino}-1-hydroxyethyl)-2-(hydroxymethyl)phenol;
4-{(1R)-2-[(2-{(3R)-3-[(Benzyloxy)methyl]-2,3-dihydro-1,4-benzodioxin-6-yl}ethyl)amino]-1-hydroxyethyl}-2-(hydroxymethyl)phenol;
4-{(1R)-2-[(2-{(3S)-3-[(Benzyloxy)methyl]-2,3-dihydro-1,4-benzodioxin-6-yl}ethyl)amino]-1-hydroxyethyl}-2-(hydroxymethyl)phenol;
2-(Hydroxymethyl)-4-{(1R)-1-hydroxy-2-[(2-{(3R)-3-[(pyridin-3-ylmethoxy)methl]-2,3-dihydro-1,4-benzodioxin-6-yl}ethyl)amino]ethyl}phenol;
4-((1R)-2-{[2-((3R)-3-{[(6-Chlorpyridin-3-yl)-methoxy]methyl}-2,3-dihydro-1,4-benzodioxin-6-yl)ethyl]amino}-1-hydroxyethyl)-2-(hydroxymethyl)phenol;
4-((1R)-2-{[2-((3R)-3-{[(2,6-Dichlorpyridin-3-yl)methoxy]methyl}-2,3-dihydro-1,4-benzodioxin-6-yl)ethyl]amino}-1-hydroxyethyl)-2-(hydroxymethyl)phenol ;
4-{(1R)-2-[(2-{2-[(Benzyloxy)methyl]-2,3-dihydro-1,4-benzodioxin-6-yl}ethyl)amino]-1-hydroxyethyl}-2-(hydroxymethyl)phenol;
4-((1R)-2-{[2-((3R)-3-{[(5-Brompyridin-3-yl) - methoxy]methyl}-2,3-dihydro-1,4-benzodioxin-6-yl)ethyl]amino}-1-hydroxyethyl)-2-(hydroxymethyl)phenol;
3-[({(2R)-7-[2-({(2R)-2-Hydroxy-2-[4-hydroxy-3-(hydroxymethyl)phenyl]ethyl}amino)ethyl]-2,3-dihydro-1,4-benzodioxin-2-yl}methoxy)methyl]benzonitril;
3-[({(2R)-7-[2-({(2R)-2-Hydroxy-2-[4-hydroxy-3-(hydroxymethyl)phenyl]ethyl}amino)ethyl]-2,3-dihydro-1,4-benzodioxin-2-yl}methoxy)methyl]benzamid;
4-[(1R)-2-({2-[(3R)-3-({[3-(Cyclopentylthio)-benzyl]oxy}methyl)-2,3-dihydro-1,4-benzodioxin-6-yl]ethyl}amino)-1-hydroxyethyl]-2-(hydroxymethyl)-phenol;
4-[(1R)-2-({2-[(3R)-3-({[3-(Cyclopentylsulfonyl)-benzyl]oxy}methyl)-2,3-dihydro-1,4-benzodioxin-6-yl]ethyl}amino)-1-hydroxyethyl]-2-(hydroxymethyl)-phenol;
2-(Hydroxymethyl)-4-{(1R)-1-hydroxy-2-[(2-[(3R)-3-[({5-[4-(methylsulfinyl)phenyl]pyridin-3-yl}methoxy)-methyl]-2,3-dihydro-1,4-benzodioxin-6-yl}ethyl)amino]-ethyl}phenol;
N-{3-[({(2R)-7-[2-({(2R)-2-Hydroxy-2-[4-hydroxy-3-(hydroxymethyl)phenyl]ethyl}amino)ethyl]-2,3-dihydro-1,4-benzodioxin-2-yl}methoxy)methyl]phenyl}harnstoff;
4-((1R)-2-{[2-((3R)-3-{[(4-Chlorbenzyl)oxy]methyl}-2,3-dihydro-1,4-benzodioxin-6-yl)ethyl]amino}-1-hydroxyethyl)-2-(hydroxymethyl)phenol;
4-((1R)-2-{[2-((3R)-3-{[(4-Fluorbenzyl)oxy]methyl}-2,3-dihydro-1,4-benzodioxin-6-yl)ethyl]amino}-1-hydroxyethyl)-2-(hydroxymethyl)phenol;
4-((1R)-2-{[2-((3R)-3-{[(3,5-Dimethylbenzyl)oxy]-methyl}-2,3-dihydro-1,4-benzodioxin-6-yl)ethyl]amino}-1-hydroxyethyl)-2-(hydroxymethyl)phenol;
2-(Hydroxymethyl)-4-{(1R)-1-hydroxy-2-[(2-{(3R)-3-[(1-phenylethoxy)methyl]-2,3-dihydro-1,4-benzodioxin-6-yl}ethyl)amino]ethyl}phenol;
2-(Hydroxymethyl)-4-[(1R)-1-hydroxy-2-({2-[(3R)-3-({3-(methylsulfonyl)benzyl}oxy}methyl)-2,3-dihydro-1,4-benzodioxin-6-yl]ethyl}amino)ethyl]phenol;
4-((1R)-2-{[2-((3R)-3-{[3-(2,6-Dichlorphenyl)-propoxy]methyl}-2,3-dihydro-1,4-benzodioxin-6-yl)ethyl]amino}-1-hydroxyethyl)-2-(hydroxymethyl)phenol;
3-[({(2R)-7-[2-({(2R)-2-Hydroxy-2-[4-hydroxy-3-(hydroxymethyl)phenyl]ethyl}amino)ethyl]-2,3-dihydro-1,4-benzodioxin-2-yl}methoxy)methyl]benzolsulfonamid;
6-{2-[(2-{(3R)-3-[(Benzyloxy)methyl]-2,3-dihydro-1,4-benzodioxin-6-yl}ethyl)amino]-1-hydroxyethyl}-2-(hydroxymethyl)pyridin-3-ol;
N-(5-{(1R)-2-[(2-{(3R)-3-[(Benzyloxy)methyl]-2,3-dihydro-1,4-benzodioxin-6-yl}ethyl)amino]-1-hydroxyethyl}-2-hydroxyphenyl)methansulfonamid;
4-{(1R)-2-[(2-{(3R)-3-[(Benzyloxy)methyl]-2,3-dihydro-1,4-benzodioxin-6-yl}ethyl)amino]-1-hydroxyethyl}-2-fluorphenol;
4-{(1R)-2-[(2-{(3R)-3-[(Benzyloxy)methyl]-2,3-dihydro-1,4-benzodioxin-6-yl}ethyl)amino]-1-hydroxyethyl}-3-methylphenol ;
(1R)-1-(4-Amino-3,5-dichlorphenyl)-2-[(2-{(3R)-3-[(benzyloxy)methyl]-2,3-dihydro-1,4-benzodioxin-6-yl}ethyl)amino]ethanol;
5{(1R)-2-[(2-{(3R)-3-[(Benzyloxy)methyl]-2,3-dihydro-1,4-benzodioxin-6-yl}ethyl)amino]-1-hydroxyethyl}-2-hydroxyphenylformamid;
oder ein Salz oder Solvat davon.

12. Verbindung der Formel (I) gemäß einem der Ansprüche 1 bis 10 oder ein pharmazeutisch annehmbares Salz oder Solvat zur Verwendung in der medizinischen Therapie.

13. Pharmazeutische Formulierung, die eine Verbindung der Formel (I) gemäß einem der Ansprüche 1 bis 10 oder ein pharmazeutisch annehmbares Salz, Solvat oder physiologisch funktionelles Derivat davon und einen pharmazeutisch annehmbaren Träger oder Exzipienten und gegebenenfalls einen oder mehrere andere therapeutische Bestandteile umfaßt.

14. Verwendung einer Verbindung der Formel (I) gemäß einem der Ansprüche 1 bis 10 oder eines pharmazeutisch annehmbaren Salzes, Solvats oder physiologisch funktionellen Derivats davon in der Herstellung eines Medikaments für die Prophylaxe oder Behandlung eines klinischen Zustands, für den ein selektiver β₂-Adrenorezeptoragonist angezeigt ist.

15. Verfahren zur Herstellung einer Verbindung der Formel (I) gemäß einem der Ansprüche 1 bis 10 oder eines Salzes, Solvats oder physiologisch funktionellen Derivats davon, das folgendes umfaßt:
(a) Entschützen eines geschützten Zwischenprodukts, zum Beispiel der Formel (II) 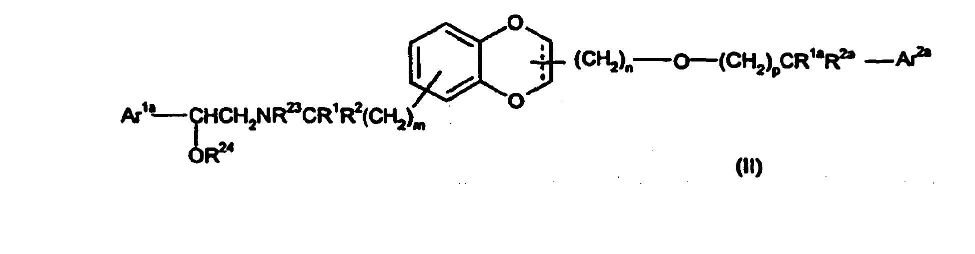 oder eines Salzes oder Solvats davon, worin R¹, R², R^{1a}, R^{2a}, m, n, p und ---- wie für die Verbindung der Formel (I) definiert sind, Ar^{1a} eine gegebenenfalls geschützte Form von Ar¹ darstellt; Ar^{2a} eine gegebenenfalls geschützte Form von Ar² darstellt, und R²³ und R²⁴ jeweils unabhängig entweder Wasserstoff oder ein Schutzgruppe sind, vorausgesetzt, daß die Verbindung der Formel (II) mindestens eine Schutzgruppe enthält;
(b) Alkylierung eines Amins der Formel 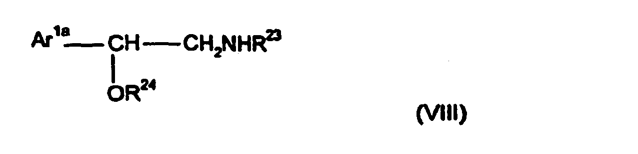 worin Ar^{1a}, R²³ und R²⁴ wie für Formel (II) definiert sind, mit einer Verbindung der Formel (XV): 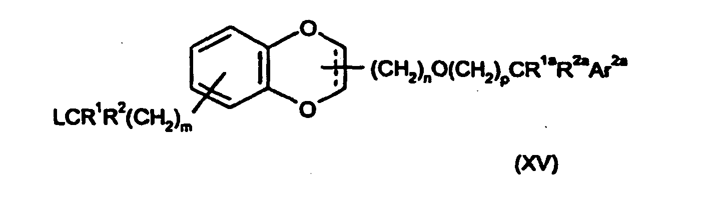 worin Ar², R¹, R², R^{1a}, R^{2a}, m, n und p wie für die Verbindung der Formel (II) definiert sind und L eine wie für Formel (IX) definierte Abgangsgruppe ist;
gefolgt von den nachfolgenden Schritten in beliebiger Reihenfolge:
(i) optionelles Entfernen jedweder Schutzgruppen;
(ii) optionelle Trennung eines Enantiomers von einem Gemisch aus Enantiomeren;
(iii) optionelles Umwandeln des Produkts in ein entsprechendes Salz, Solvat oder physiologisch funktionelles Derivat davon.

## Revendications

1. Composé de formule (I) : 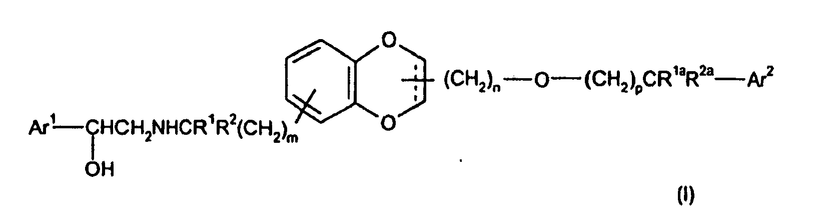 ou sel ou solvate de ceux-ci,
dans laquelle :
Ar¹ est un groupe choisi parmi 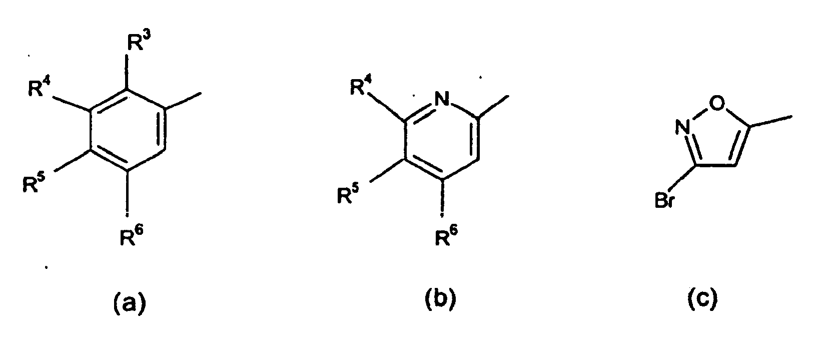 et 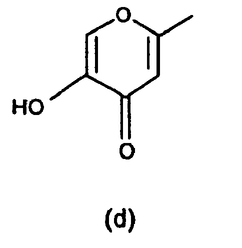
où R⁴ représente un atome d'hydrogène, d'halogène, un groupe -(CH₂)_{q}OR⁷, -NR⁷C(O)R⁸, -NR⁷SO₂R⁸, -SO₂NR⁷R⁸, -NR⁷R⁸, -OC(O)R⁹ ou OC(O)NR⁷R⁸,
et R³ représente un atome d'hydrogène, d'halogène ou un groupe alkyle en C₁₋₄ ;
ou bien R⁴ représente un groupe -NHR¹⁰ et R³ et NHR¹⁰ forment ensemble un noyau hétérocyclique de 5 ou 6 chaînons ;
R⁵ représente un atome d'hydrogène, d'halogène, un groupe-OR⁷ ou -NR⁷R⁸;
R⁶ représente un atome d'hydrogène, d'halogène, un groupe haloalkyle en C₁₋₄, -OR⁷, -NR⁷R⁸, -OC(O)R⁹ ou OC(O)NR⁷R⁸;
R⁷ et R⁸ représentent chacun indépendamment un atome d'hydrogène ou un groupe alkyle en C₁₋₄, ou bien dans les groupes -NR⁷R⁸, -SO₂NR⁷R⁸ et -OC(O)NR⁷R⁸, R⁷ et R⁸ représentent indépendamment un atome d'hydrogène ou un groupe alkyle en C₁₋₄ ou forment ensemble avec l'atome d'azote auquel ils sont attachés un noyau contenant de l'azote à 5, 6 ou 7 chaînons,
R⁹ représente un groupe aryle (par exemple phényle ou naphtyle) qui peut être non substitué ou substitué par un ou plusieurs substituants choisis parmi un atome d'halogène, un groupe alkyle en C₁₋₄, hydroxy, alcoxy en C₁₋₄, ou haloalkyle en C₁₋₄; et
q est zéro ou un entier de 1 à 4;
Ar² est un groupe 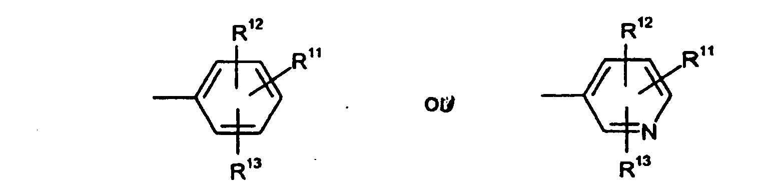 dans lesquels :
R¹¹ est choisi parmi un atome d'hydrogène, un groupe alkyle en C₁₋₆, hydroxy, alcoxy en C₁₋₆, cyano, nitro, halo, haloalkyle en C₁₋₆, XCO₂R¹⁶, -XC(O)NR¹⁵R¹⁶, -XNR¹⁴C(O)R¹⁵, -XNR¹⁴C(O)NR¹⁵R¹⁶, -XNR¹⁴C(O)NC(O)NR¹⁶sR¹⁶, -XNR¹⁴SO₂R¹⁵, -XSO₂NR¹⁷R¹⁸, XSR¹⁴ XSOR¹⁴, XSO₂R¹⁴, -XNR¹⁵R¹⁶, -XNR¹⁴C(O)OR¹⁵ ou XNR¹⁴SO₂NR¹⁵R¹⁶,
ou bien R¹¹ est choisi parmi les groupes -X aryle, -X-hétaryle ou -X-(aryloxy), chacun étant éventuellement substitué par 1 ou 2 groupes choisis indépendamment parmi un groupe hydroxy, alcoxy en C₁₋₆, halo, alkyle en C₁₋₆ haloalkyle en C₁₋₆, cyano, nitro, CONR¹⁵R¹⁶, -NR¹⁴C(O)R¹⁵, SR¹⁴ , SOR¹⁴, -SO_{2R}¹⁴, -SO₂NR¹⁷R¹⁸, -CO₂R¹⁶, -NR¹⁵R¹⁶ ou hétaryle éventuellement substitué par 1 ou 2 groupes choisis indépendamment parmi un groupe hydroxy, alcoxy en C₁₋₆, halo, alkyle en C₁₋₆ ou haloalkyle en C₁₋₆ ;
X est -(CH₂)ᵣ- ou alcénylène en C₂₋₆ ;
R est un entier de 0 à 6, de préférence de 0 à 4 ;
R¹⁴ et R¹⁵ sont choisis indépendamment parmi un atome d'hydrogène, un groupe alkyle en C₁₋₆, cycloalkyle en C₃₋₇, aryle, hétaryle, hétarylalkyl(en C₁₋₆)- et arylalkyl(en C₁₋₆)-, et R¹⁴et R¹⁵ sont chacun indépendamment éventuellement substitués par 1 ou 2 groupes choisis indépendamment parmi un atome d'halogène, un groupe alkyle en C₁₋₆, cycloalkyle en C₃₋₇ alcoxy en C₁₋₆, haloalkyle en C₁₋₆, -NHC(O)alkyle en C₁₋₆, -SO₂alkyle en C₁₋₆, -SO₂(aryle), -CO₂H et -CO₂alkyle en C₁₋₄, -NH₂, -NHalkyle en C₁₋₆, arylalkyl(en C₁₋₆)-, arylalcényl(en C₂₋₆)-, arylalcynyl(en C₂₋₆)-, hétarylalkyl(en C₁₋₆)-, -NHSO₂aryle,-NHhétarylalkyle en C₁₋₆, -NHSO₂hétaryle, -NHSO₂alkyle en C₁₋₆, -NHC(O)aryle ou -NHC(O)hétaryle ;
ou bien R¹⁴ et R¹⁵ forment ensemble avec l'atome d'azote auquel ils sont liés, un noyau contenant de l'azote à 5, 6 ou 7 chaînons ;
ou bien lorsque R¹¹ est un groupe -XNR¹⁴C(O)NR¹⁵R¹⁶, R¹⁴ et R¹⁵peuvent former ensemble avec la partie -NC(O)N- du groupe R¹ à laquelle ils sont liés, un noyau saturé ou insaturé, de préférence un noyau à 5, 6 ou 7 chaînons, par exemple, un noyau imidazolidine, comme imidazolidine-2,4-dione ;
ou bien lorsque R¹¹ est un groupe -XNR¹⁴C(O)OR¹⁵, R¹⁴ et R¹⁵ peuvent former ensemble avec la partie -NC(O)O- du groupe R¹¹ à laquelle ils sont liés, un noyau saturé ou insaturé, de préférence un noyau à 5, 6 ou 7 chaînons, par exemple, un noyau oxazolidine, comme oxazolidine-2,4-dione ;
R¹⁶ est choisi parmi un atome d'hydrogène, un groupe alkyle en C₁₋₆ et cycloalkyle en C₃₋₇ ;
ou bien lorsque R¹¹ est un groupe -XC(O)NR¹⁵R¹⁶ ou-XNR¹⁴C(O)NR¹⁵R¹⁶, R¹⁵ et R¹⁶ peuvent former ensemble avec l'atome d'azote auquel ils sont liés, un noyau contenant de l'azote à 5, 6 ou 7 chaînons ;
R¹⁷ et R¹⁸ sont choisis indépendamment parmi un atome d'hydrogène, un groupe alkyle en C₁₋₆, cycloalkyle en C₃₋₇, aryle, hétaryle, hétarylalkyl(en C₁₋₆)-, et arylalkyl(en C₁₋₆)-ou bien R¹⁷ et R¹⁸ forment ensemble avec l'atome d'azote auquel ils sont liés, un noyau contenant de l'azote à 5, 6 ou 7 chaînons ;
et R¹⁷ et R¹⁸ sont chacun éventuellement substitués par un ou deux groupes choisis indépendamment parmi un atome d'halogène, un groupe alkyle en C₁₋₆, cycloalkyle en C₃₋₇ et haloalkyle en C₁₋₆;
R¹² est choisi parmi un atome d'hydrogène, un groupe hydroxy, alkyle en C₁₋₆, alcoxy en C₁₋₆, halo, aryle, arylalkyl(en C₁₋₆)-, haloalcoxy en C₁₋₆ et haloalkyle en C₁₋₆ ;
R¹³ est choisi parmi un atome d'hydrogène, un groupe hydroxy, alkyle en C₁₋₆, alcoxy en C₁₋₆, halo, aryle, arylalkyl(en C₁₋₆)-, haloalcoxy en C₁₋₆ et haloalkyle en C₁₋₆ ;
R¹ et R² sont choisis indépendamment parmi un atome d'hydrogène et un groupe alkyle en C₁₋₄, à condition que le nombre total d'atomes de carbone dans R¹ et R² ne dépasse par 4 ;
l'un des R^{1a} et R^{2a} est choisi parmi un atome d'hydrogène et un groupe alkyle en C₁₋₄, et l'autre des R^{1a} et R^{2a} représente un atome d'hydrogène ou un groupe alkyle en C₁₋₄;
m est un entier de 1 à 3 ;
n est un entier de 1 à 4 ; et
p est zéro ou un entier de 1 à 3;
et ---- représente une simple liaison ou une double liaison.

2. Composé de formule (I) selon la revendication 1, ou sel ou solvate de ceux-ci, dans laquelle R^{1a} et R^{2a} représentent chacun un atome d'hydrogène ; et dans le groupe Ar¹ :
R⁴ représente un atome d'halogène, un groupe -(CH₂)_{q}OR⁷, -NR⁷C(O)R⁸, -NR⁷SO₂R⁸, -SO₂NR⁷R⁸, -NR⁷R⁸, -OC(O)R⁹ ou OC(O)NR⁷R⁸ et
R³ représente un atome d'hydrogène ou un groupe alkyle en C₁₋₄.

3. Composé de formule (I) selon la revendication 1, ou sel ou solvate de ceux-ci, dans laquelle R^{1a} et R^{2a} représentent chacun un atome d'hydrogène ; et dans le groupe Ar¹ :
R⁴ représente un groupe -NHR¹⁰ et
R³ et -NHR¹⁰ forment ensemble un noyau hétérocyclique à 5 ou 6 chaînons.

4. Composé de formule (I) selon l'une quelconque des revendications 1 à 3, dans laquelle le groupe Ar¹ est choisi parmi les groupes (a) et (b) tels que définis dans la revendication 1.

5. Composé de formule (I) selon l'une quelconque des revendications 1 à 4, dans laquelle, dans le groupe Ar², R¹¹ est choisi parmi un atome d'hydrogène, un groupe alkyle en C₁₋₄, hydroxy, halo, -NR¹⁴C(O)NR¹⁷R¹⁶, -NR¹⁴SO₂R¹⁵ et XSO₂NR¹⁷R¹⁸, où les R¹⁴ à R¹⁸ sont tels que définis dans la revendication 1.

6. Composé de formule (I) selon l'une quelconque des revendications 1 à 4, dans laquelle, dans le groupe Ar², R¹¹ est choisi parmi un groupe cyano, -CONR¹⁵R¹⁶, SR¹⁴, SOR¹⁴ et SO₂R¹⁴, où les R¹⁴, R¹⁵ et R¹⁶ sont tels que définis dans la revendication 1.

7. Composé de formule (I) selon l'une quelconque des revendications 1 à 6, dans laquelle R¹² et R¹³ représentent chacun un atome d'hydrogène.

8. Composé de formule (I) selon l'une quelconque des revendications 1 à 6, dans laquelle R¹¹ représente un atome d'hydrogène et R¹² et R¹³ représentent chacun un atome d'halogène ou un groupe alkyle en C₁₋₆.

9. Composé de formule (I) selon l'une quelconque des revendications 1 à 8, dans laquelle R¹ et R² sont tous deux un atome d'hydrogène.

10. Composé de formule (I) selon l'une quelconque des revendications 1 à 9, dans laquelle chacun des m et n est indépendamment 1 ou 2, et p est zéro ou 1.

11. Composé de formule (I) choisi parmi les suivants :
4-((1R)-2-{[2-((3R)-3-{[(2,6-dichlorobenzyl)oxy]méthyl}-2,3-dihydro-1,4-benzodioxin-6-yl)éthyl]amino}-1-hydroxyéthyl)-2-(hydroxyméthyl)phénol ;
4-{(1R)-2-[(2-{(3R)-3-[(benzyloxy)méthyl]-2,3,dihydro-1,4, benzodioxin-6-yl}éthyl)amino]-1-hydroxyéthyl}-2-(hydroxyméthyl)phénol ;
4-{(1R)-2-[(2-{(3S)-3-[(benzyloxy)méthyl]-2,3-dihydro-1,4-benzodioxin-6-yl}éthyl)amino]-1-hydroxyéthyl}-2-(hydroxyméthyl)phénol ;
2-(hydroxyméthyl)-4-{(1R)-1-hydroxy-2-[(2-{(3R)-3-[(pyridin-3-ylméthoxy)méthyl]-2,3-dihydro-1,4-benzodioxin-6-yl}éthyl)amino]éthyl}phénol ;
4-((1R)-2-{[2-((3R)-3-{[(6-chloropyridin-3-yl)méthoxy]méthyl}-2,3-dihydro-1,4-benzodioxin-6-yl)éthyl]amino}-1-hydroxyéthyl)-2-(hydroxyméthyl)phénol ;
4-((1R)-2-{[2-((3R)-3-{[(2,6-dichloropyridin-3-yl)méthoxy]méthyl}-2,3-dihydro-1,4-benzodioxin-6-yl)éthyl]amino}-1-hydroxyéthyl)-2-(hydroxyméthyl)phénol ;
4-{(1R)-2-[(2-{2-[(benzyloxy)méthyl]-2,3-dihydro-1,4-benzodioxin-6-yl}éthyl)amino]-1-hydroxyéthyl}-2-(hydroxyméthyl)phénol ;
4-((1R)-2-{[2-((3R)-3-{[(5-bromopyridin-3-yl)méthoxy]méthyl}-2,3-dihydro-1,4-benzodioxin-6-yl)éthyl] amino}-1-hydroxyéthyl)-2-(hydroxyméthyl)phénol ;
3-[({(2R)-7-[2-({(2R)-2-hydroxy-2-[4-hydroxy-3-(hydroxyméthyl)phényl]éthyl}amino)éthyl]-2,3-dihydro-1,4-benzodioxin-2-yl}méthoxy)méthyl]benzonitrile;
3-[({(2R)-7-[2-({(2R)-2-hydroxy-2-[4-hydroxy-3-(hydroxyméthyl)phényl]éthyl}amino)éthyl]-2,3-dihydro-1,4-benzodioxin-2-yl}méthoxy)méthyl]benzamide;
4-((1R)-2-({2-[(3R)-3-({[3-(cyclopentylthio)benzyl]oxy}méthyl)-2,3-dihydro-1,4-benzodioxin-6-yl]éthyl}amino)-1-hydroxyéthyl]-2-(hydroxyméthyl)phénol ;
4-[(1R)-2-({2-[(3R)-3-({[3-(cyclopentylsulfonyl)benzyl]-oxy}méthyl)-2,3-dihydro-1,4-benzodioxin-6-yl]ethyl}amino)-1-hydroxyéthyl]-2-(hydroxyméthyl)phénol ;
2-(hydroxyméthyl)-4-{(1R)-1-hydroxy-2-[(2-{(3R)-3-[({5-[4-(méthylsulfinyl)phényl]pyridin-3-yl}méthoxy)méthyl]-2,3-dihydro-1,4-benzodioxin-6-yl}éthyl)amino]éthyl}phénol ;
N-{3-[({(2R)-7-[2-({(2R)-2-hydroxy-2-[4-hydroxy-3-(hydroxyméthyl)phényl]éthyl}amino)éthyl]-2,3-dihydro-1,4-benzodioxin-2-yl}méthoxy)méthyl]phényl}urée ;
4-((1R)-2-{[2-((3R)-3-{[(4-chlorobenzyl)oxy]méthyl}-2,3-dihydro-1,4-benzodioxin-6-yl)éthyl]amino}-1-hydroxyéthyl)-2-(hydroxyméthyl)phénol ;
4-((1R)-2-{[2-((3R)-3-{[(4-fluorobenzyl)oxy]méthyl}-2,3-dihydro-1,4-benzodioxin-6-yl)éthyl] amino}-1-hydroxyéthyl)-2-(hydroxyméthyl)phénol ;
4-((1R)-2-{[2-((3R)-3-{[(3,5-diméthylbenzyl)oxy]méthyl}-2,3-dihydro-1,4-benzodioxin-6-yl)éthyl]amino}-1-hydroxyéthyl)-2-(hydroxyméthyl)phénol ;
2-(hydroxyméthyl)-4-{(1R)-1-hydroxy-2-[(2-{(3R)-3-[(1-phényléthoxy)méthyl]-2,3-dihydro-1,4-benzodioxin-6-yl}éthyl)amino]éthyl}phénol ;
2-(hydroxyméthyl)-4-{(1R)-1-hydroxy-2-({2-[(3R)-3-({[3-(méthylsulfonyl)benzyl]oxy}méthyl)-2,3-dihydro-1,4-benzodioxin-6-yl]éthyl}amino)éthyl]phénol ;
4-((1R)-2-{[2-((3R)-3-{[3-(2,6-dichlorophényl)propoxy]méthyl}-2,3-dihydro-1,4-benzodioxin-6-yl)éthyl]amino}-1-hydroxyéthyl)-2-(hydroxyméthyl)phénol ;
3-[({(2R)-7-[2-({(2R)-2-hydroxy-2-(4-hydroxy-3-(hydroxyméthyl)phényl]éthyl}amino)éthyl]-2,3-dihydro-1,4-benzodioxin-2-yl}méthoxy)méthyl]benzènesulfonamide ;
6-{2-[(2-{(3R)-3-[(benzyloxy)méthyl]-2,3-dihydro-1,4-benzodioxin-6-yl}éthyl)amino]-1-hydroxyéthyl}-2-(hydroxyméthyl)pyridin-3-ol ; N-(5-{(1R)-2-[(2-{(3R)-3-[(benzyloxy)méthyl]-2,3-dihydro-1,4-benzodioxin-6-yl}éthyl)amino]-1-hydroxyéthyl}-2-hydroxyphényl)méthanesulfonamide ;
4-{(1R)-2-[(2-{(3R)-3-[(benzyloxy)méthyl]-2,3-dihydro-1,4-benzodioxin-6-yl}éthyl)amino]-1-hydroxyéthyl}-2-fluorophénol; 4-{(1R)-2-[(2-{(3R)-3-[(benzyloxy)méthyl]-2,3-dihydro-1,4-benzodioxin-6-yl}éthyl)amino]-1-hydroxyéthyl}-3-méthylphénol ; (1R)-1-(4-amino-3,5-dichlorophényl)-2-[(2-{(3R)-3-[(benzyloxy)méthyl]-2,3-dihydro-1,4-benzodioxin-6-yl}éthyl)amino]éthanol ;
5-{(1R)-2-[(2-{(3R)-3-[(benzyloxy)méthyl]-2,3-dihydro-1,4-benzodioxin-6-yl}éthyl)amino]-1-hydroxyéthyl}-2-hydroxyphénylformamide ;
ou un sel ou un solvate de ceux-ci.

12. Composé de formule (I) selon l'une quelconque des revendications 1 à 10, ou sel ou solvate acceptable du point de vue pharmaceutique de ceux-ci, utile en thérapie médicale.

13. Formulation pharmaceutique comprenant un composé de formule (I) selon l'une quelconque des revendications 1 à 10, ou un sel, solvate ou dérivé fonctionnel du point de vue physiologique de ceux-ci acceptable du point de vue pharmaceutique, et un support ou excipient acceptable du point de vue pharmaceutique, et éventuellement un ou plusieurs autres composants thérapeutiques.

14. Utilisation d'un composé de formule (I) selon l'une quelconque des revendications 1 à 10, ou d'un sel, solvate ou dérivé fonctionnel du point de vue physiologique de ceux-ci acceptable du point de vue pharmaceutique, dans la fabrication d'un médicament destiné à la prophylaxie ou au traitement d'un état clinique pour lequel un agoniste sélectif de β₂-adrénorécepteur est indiqué.

15. Procédé de préparation d'un composé de formule (I) selon l'une quelconque des revendications 1 à 10, ou d'un sel, solvate ou dérivé fonctionnel du point de vue physiologique de ceux-ci, qui comprend :
(a) la déprotection d'un intermédiaire protégé, par exemple de formule (II) : 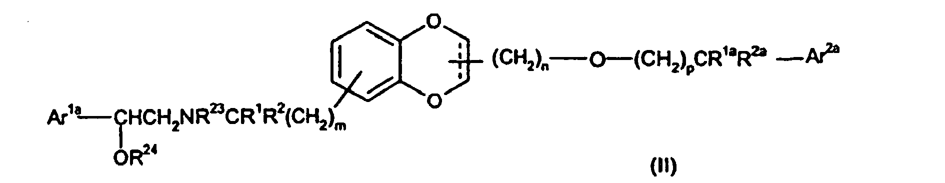 ou d'un sel ou solvate de ceux-ci, dans laquelle R¹, R², R^{1a}, R^{2a}, m, n, p et ---- sont tels que définis pour le composé de formule (I), Ar^{1a} représente une forme éventuellement protégée de Ar¹; Ar^{2a} représente une forme éventuellement protégée de Ar² et R²³ et R²⁴ sont chacun indépendamment soit un atome d'hydrogène, soit un groupe protecteur, à condition que le composé de formule (II) contienne au moins un groupe protecteur ;
(b) l'alkylation d'une amine de formule (VIII) : 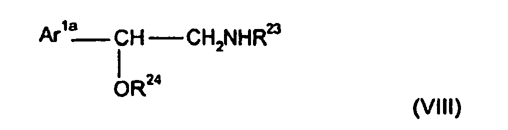
dans laquelle Ar^{1a}, R²³ et R²⁴ sont tels que définis pour la formule (II), avec un composé de formule (XV) : 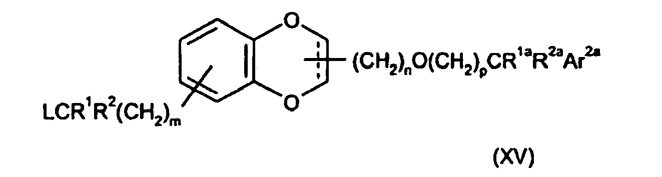 dans laquelle ----, Ar², R¹, R², R^{1a}, R^{2a}, m, n et p sont tels que définis pour le composé de formule (II) et L est un groupe mobile tel que défini pour la formule (IX) ;
suivies par les étapes suivantes dans un ordre quelconque :
(i) élimination éventuelle de quelconques groupes protecteurs ;
(ii) séparation éventuelle d'un énantiomère à partir d'un mélange d'énantiomères ;
(iii) conversion éventuelle du produit en un sel, solvate ou dérivé fonctionnel du point de vue physiologique correspondant de ceux-ci.
